(19) **Europäisches Patentamt / European Patent Office / Office européen des brevets**

(11) **EP 2 361 388 B1**

(12) **EUROPEAN PATENT SPECIFICATION**

(45) Date of publication and mention
of the grant of the patent:
**25.09.2013 Bulletin 2013/39**

(51) Int Cl.:
***G01N 33/543*** *(2006.01)*   ***G01N 33/50*** *(2006.01)*
***G06F 19/00*** *(2011.01)*

(21) Application number: **09796176.7**

(22) Date of filing: **23.11.2009**

(86) International application number:
**PCT/US2009/065483**

(87) International publication number:
**WO 2010/060019 (27.05.2010 Gazette 2010/21)**

(54) **METHODS FOR CHARACTERIZING MOLECULES**

VERFAHREN ZUR CHARAKTERISIERUNG VON MOLEKÜLEN

PROCÉDÉS POUR CARACTÉRISER DES MOLÉCULES

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **24.11.2008 US 117450 P**

(43) Date of publication of application:
**31.08.2011 Bulletin 2011/35**

(73) Proprietor: **Corning Inc.
Corning, NY 14831 (US)**

(72) Inventors:
• **FANG, Ye
Painted Post
New York 14870 (US)**
• **FERRIE, Ann M
Painted Post
New York 14870 (US)**
• **LAHIRI, Joydeep
Painted Post
New York 14870 (US)**
• **TRAN, Elizabeth
Painted Post
New York 14870 (US)**

(74) Representative: **Zijlstra, Robert Wiebo Johan
Elkington and Fife LLP
Prospect House
8 Pembroke Road
Sevenoaks, Kent TN13 1XR (GB)**

(56) References cited:
**WO-A1-2008/130488    US-A1- 2006 160 109
US-A1- 2006 223 051**

• **KUTSYY VADIM ET AL: "COMPOUND CLASSIFICATION USING IMAGE-BASED CELLULAR PHENOTYPES" METHODS IN ENZYMOLOGY; [METHODS IN ENZYMOLOGY], ACADEMIC PRESS INC, SAN DIEGO, CA, US, vol. 414, 1 January 2006 (2006-01-01), pages 440-468, XP008080093 ISSN: 0076-6879**
• **YOUNG DANIEL W ET AL: "Integrating high-content screening and ligand-target prediction to identify mechanism of action" NATURE CHEMICAL BIOLOGY, vol. 4, no. 1, January 2008 (2008-01), pages 59-68, XP002568979**
• **TAYLOR D L ET AL: "Multiplexed high content screening assays create a systems cell biology approach to drug discovery" DRUG DISCOVERY TODAY: TECHNOLOGIES, ELSEVIER, vol. 2, no. 2, 1 July 2005 (2005-07-01), pages 149-154, XP004985713 ISSN: 1740-6749**
• **LEE PAUL H ET AL: "Evaluation of dynamic mass redistribution technology for pharmacological studies of recombinant and endogenously expressed G protein-coupled receptors" ASSAY AND DRUG DEVELOPMENT TECHNOLOGIES, vol. 6, no. 1, February 2008 (2008-02), pages 83-94, XP002568980 ISSN: 1540-658X cited in the application**
• **FANG YE: "Label-free cell-based assays with optical biosensors in drug discovery" ASSAY AND DRUG DEVELOPMENT TECHNOLOGIES, US, vol. 4, no. 5, 1 October 2006 (2006-10-01), pages 583-595, XP002488211 ISSN: 1540-658X**

**(Cont. next page)**

- GIULIANO KENNETH A ET AL: "Systems cell biology based on high-content screening" METHODS IN ENZYMOLOGY; [METHODS IN ENZYMOLOGY], ACADEMIC PRESS INC, SAN DIEGO, CA, US, 1 January 2006 (2006-01-01), pages 601-619, XP009097131 ISSN: 0076-6879
- WILKINSON JENNIFER M ET AL: "Compound profiling using a panel of steroid hormone receptor cell-based assays" JOURNAL OF BIOMOLECULAR SCREENING, vol. 13, no. 8, September 2008 (2008-09), pages 755-765, XP008122231 ISSN: 1087-0571
- VILLA M L ET AL: "Interference of cephalosporins with immune response: Effects of cefonicid on human T-helper cells" INTERNATIONAL JOURNAL OF IMMUNOPHARMACOLOGY, ELMSFORD,NY, US LNKD- DOI: 10.1016/0192-0561(91)90161-Y, vol. 13, no. 8, 1 January 1991 (1991-01-01), pages 1099-1107, XP023812733 ISSN: 0192-0561 [retrieved on 1991-01-01]
- MARRERO PONCE YOVANI ET AL: "Atom-based 2D quadratic indices in drug discovery of novel tyrosinase inhibitors: Results of in silico studies supported by experimental results" QSAR & COMBINATORIAL SCIENCE, vol. 26, no. 4, April 2007 (2007-04), pages 469-487, XP002582517 ISSN: 1611-020X
- MORO S ET AL: "The application of a 3D-QSAR (autoMEP/PLS) approach as an efficient pharmacodynamic-driven filtering method for small-sized virtual library: Application to a lead optimization of a human A3 adenosine receptor antagonist" BIOORGANIC & MEDICINAL CHEMISTRY, PERGAMON, GB LNKD- DOI: 10.1016/J.BMC.2006.03.010, vol. 14, no. 14, 15 July 2006 (2006-07-15), pages 4923-4932, XP025133391 ISSN: 0968-0896 [retrieved on 2006-07-15]
- BEROZA PAUL ET AL: "Target-related affinity profiling: Telik's lead discovery technology" CURRENT TOPICS IN MEDICINAL CHEMISTRY, vol. 5, no. 4, 2005, pages 371-381, XP008122425 ISSN: 1568-0266
- JOHANN GASTEIGER: "Chemoinformatics: a new field with a long tradition" ANALYTICAL AND BIOANALYTICAL CHEMISTRY, SPRINGER, BERLIN, DE LNKD- DOI: 10.1007/S00216-005-0065-Y, vol. 384, no. 1, 1 January 2006 (2006-01-01), pages 57-64, XP019327765 ISSN: 1618-2650

## Description

### Claiming Benefit Of Prior Filed U.S. Application

1. This application claims the benefit of U.S. Provisional Application Serial No. 61/117,450, filed on November 24, 2008.

## I. Background

2. The disclosure relates to biosensors, such as resonant waveguide grating (RWG) biosensors or surface plasmon resonance (SPR) biosensors or electric biosensors, and more specifically to the use of such biosensors to characterize molecules. The disclosure also relates to a method of producing an index for a molecule and a method of drug discovery.

US 2006/0160109 discloses panels of cell-based assays to characterise drug molecules.

US 2006/0223051 discloses optical LID biosensors and dynamic mass redistribution to characterise drug molecules.

## II. Summary

3. The invention in its broadest sense is a method for characterizing a molecule via a cell-based biosensor assay according to claim 1, comprising:

  a. generating a label-free biosensor response of a molecule in each cell in a cell panel comprising at least two types of cells, wherein the biosensor detects a dynamic mass redistribution (DMR), thereby producing a first biosensor data of the molecule in each cell;

  b. generating a label-free biosensor response of each ligand in a ligand panel in each cell in the cell panel in the presence of the molecule, thereby producing second biosensor data of the molecule for each ligand/cell pair, wherein a ligand is a molecule able to bind to and form a complex with a biomolecule to serve a biological purpose;

  c. extracting a specific set of biosensor parameters from the first and second biosensor data, wherein the biosensor parameters in the set are chosen independently;

  d. normalizing each biosensor parameter against the biosensor parameter from the first biosensor data to generate a modulation comparison;

  e. plotting the modulation comparison of at least one biosensor parameter as a function of the ligand or the cell to generate a molecule

  f. modulation index that expresses the ability of the molecule to modulate the biosensor response of the ligand panel acting on the cell panel;

  g. optionally combining the first biosensor data and the molecule modulation index to generate a molecule biosensor index; and

  h. comparing the molecule modulation index or the molecule biosensor index to a library of modulator biosensor indexes.

In an embodiment, the method further comprises identifying one or more ligand modulation profiles in the modulator biosensor index similar to one or more of the molecule modulation profiles in the molecule biosensor index.

In an embodiment, the molecule shares a similar mode of action with the modulator when the molecule biosensor index is similar to the modulator biosensor index.

In an embodiment, the library of modulator biosensor indexes comprises a molecule modulation index.

In an embodiment, different biosensor parameters are chosen for different ligands or cells.

In an embodiment, the cells in the cell panel are primary cells.

## III. Brief Description of the Drawings

4. Figure 1 shows a diagram of a cell profile pharmacology approach for drug discovery, in embodiments of the disclosure.

5. Figure 2 An example of a cell profile pharmacology approach for drug discovery, in embodiments of the disclosure.

6. Figure 3A and 3B show primary profiles of 16 different ligands, each at $10\mu$M, acting on A549 cells, in embodiments of the disclosure.

7. Figure 4 shows a primary profile of epidermal growth factor (EGF) at 32nM acting on quiescent A431 cells, in comparison with and a primary profile of the "vehicle" solution acting on A431 cells (the negative control), in embodiments of the disclosure.

8. Figure 5 shows a primary profile of 32nM EGF acting on quiescent A431 cells (control) and secondary profiles of 4 different ligands, each at 10 $\mu$M. Each secondary profile was obtained by measuring the optical response of A431 cells to 32nM EGF after the about 1hr pretreatment of cells with each ligand (BML-265, AG1478, U0126, and Rottlerin), in embodiments of the disclosure.

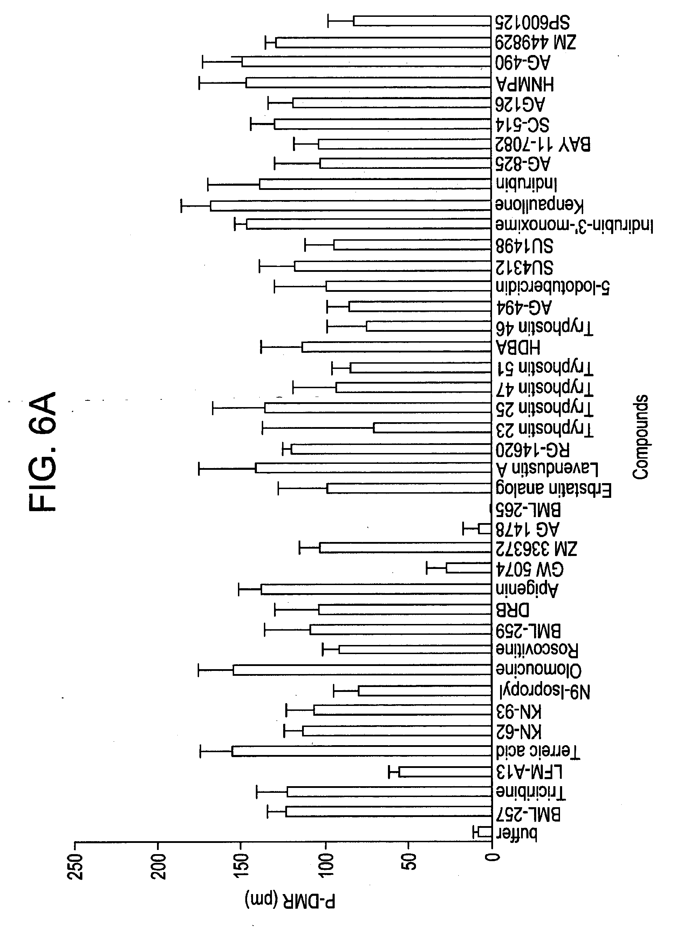

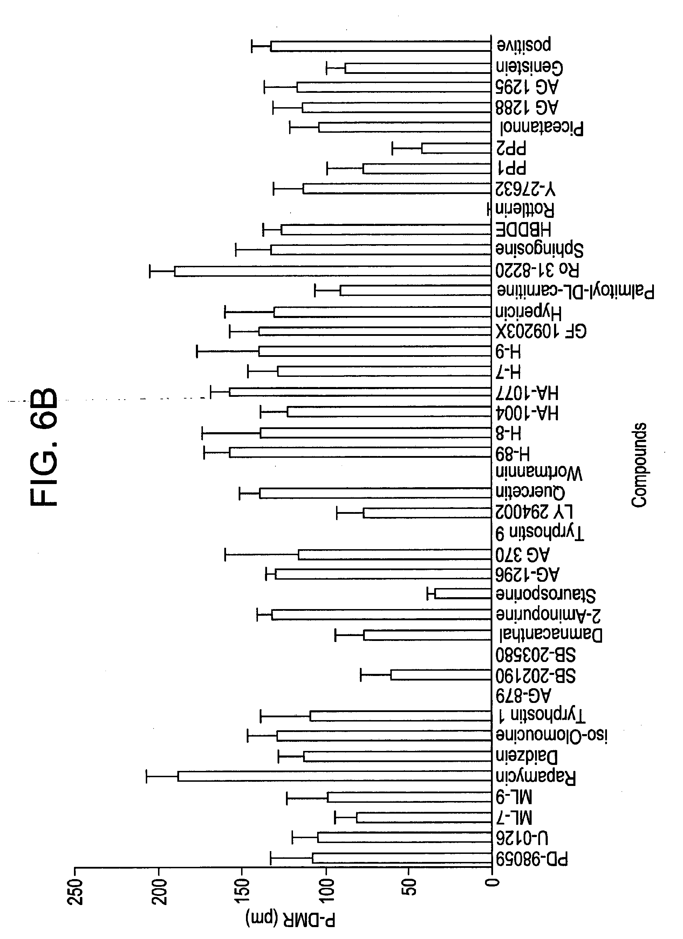

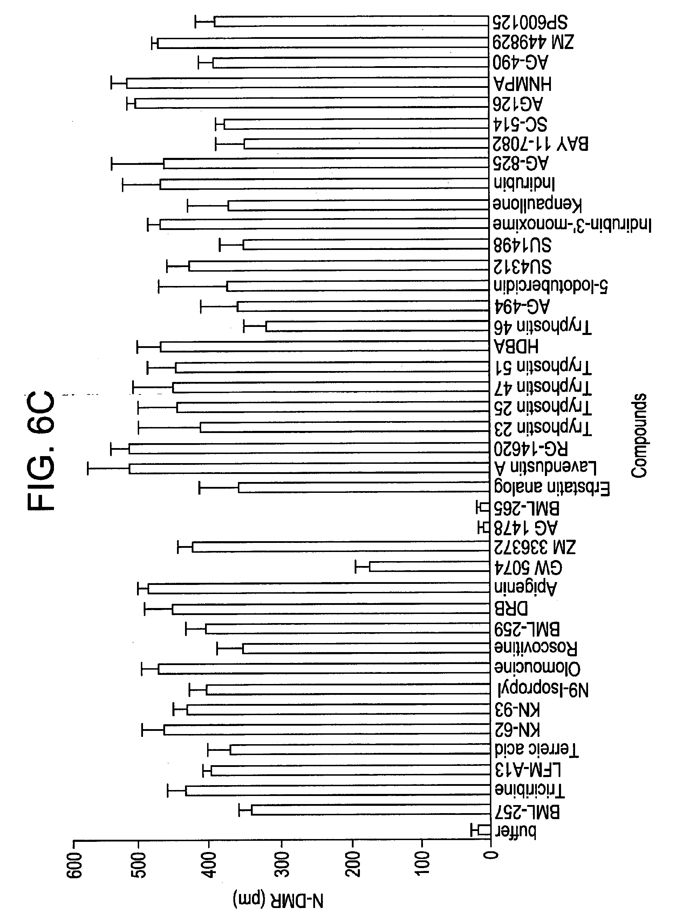

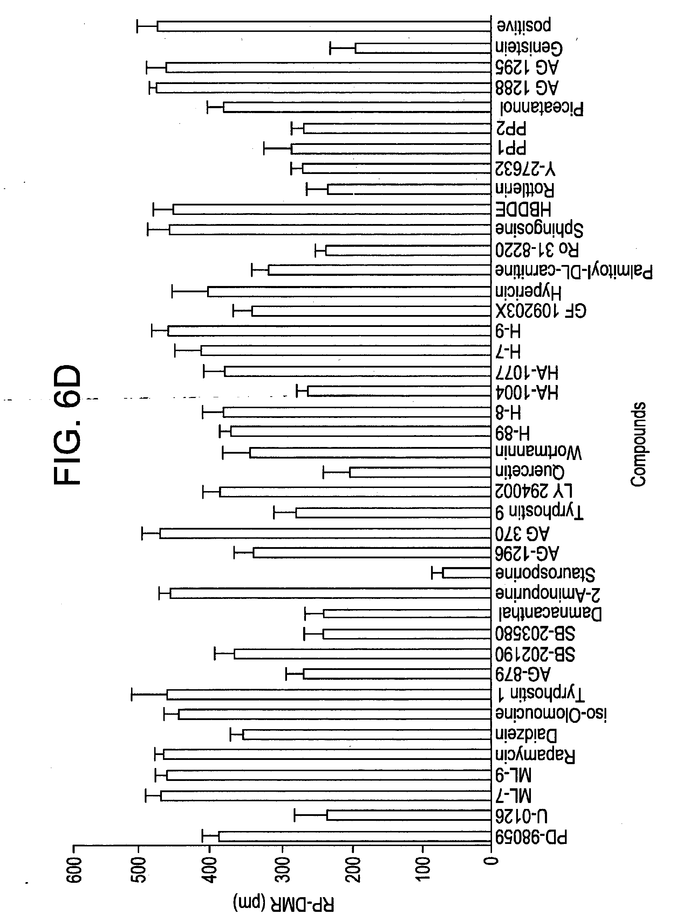

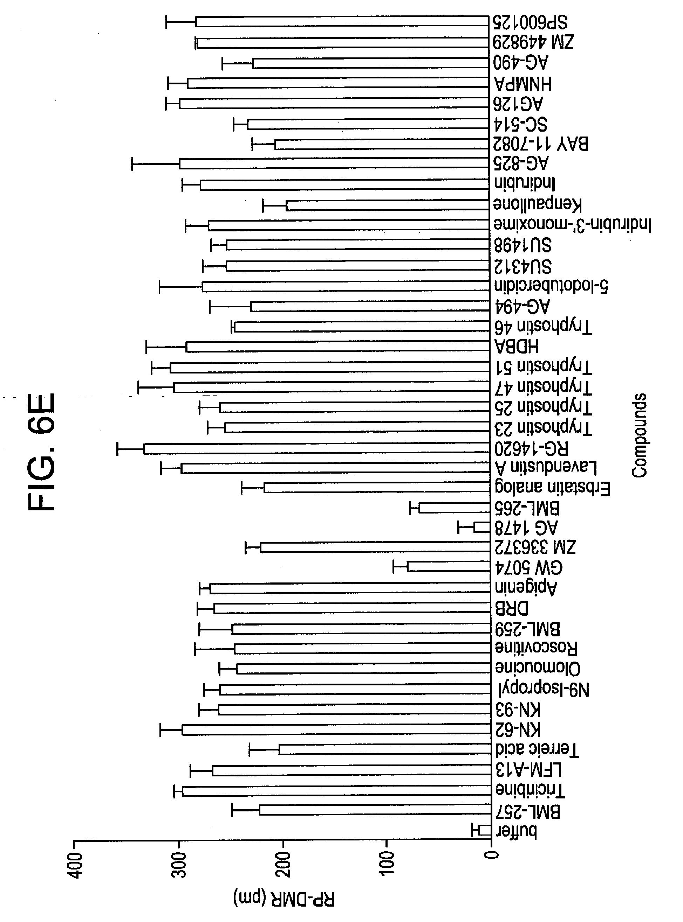

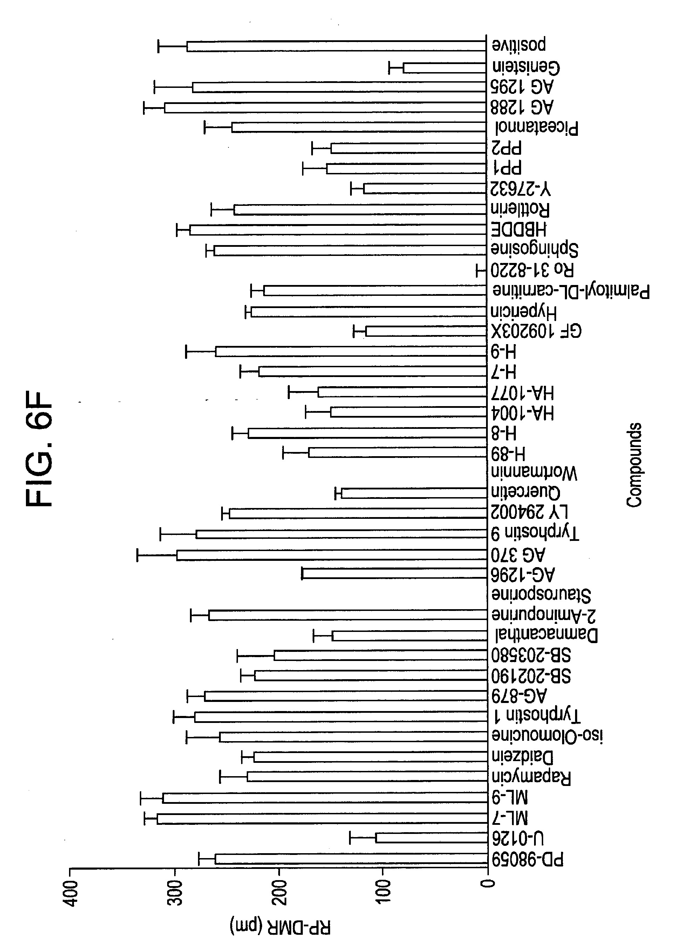

9. Figure 6A, 6B, and 6C show examples of the modulation profiles of the 32nM EGF- induced optical signal of quiescent A431 cells by a panel of ligands. Three optical response parameters of the EGF DMR signals of A431 cells in the absence and presence of a ligand were analyzed and plotted as a function of the ligand. The three parameters are the amplitude of the (A) P- DMR (positive DMR), (B) N- DMR (negative DMR), and (C) RP- DMR (recovery positive DMR) of the EGF DMR signals of A431 cells. The DMR signal of cells without pretreatment with any molecule was used as a positive control (positive), while the DMR signal of cells treated with the vehicle only (i.e., the assay buffer solution) was used a negative control (buffer).

10. Figure 7 shows primary profiles of (A) 32 nM EGF, (B) 5 μM histamine, (C) 1 μM nicotinic acid, and (D) 2 nM epinephrine, acting on quiescent A431 cells.

11. Figure 8 shows primary profiles of (A) 40μM pinacidil, (B) 8μM SLIGKV-amide, (C) 250μg/ml poly (I: C), (D) 8μM phorbol 12-myristate 13-acetate (PMA), (E) 10 μM histamine, and (F) 16μM forskolin, acting on A549 cells.

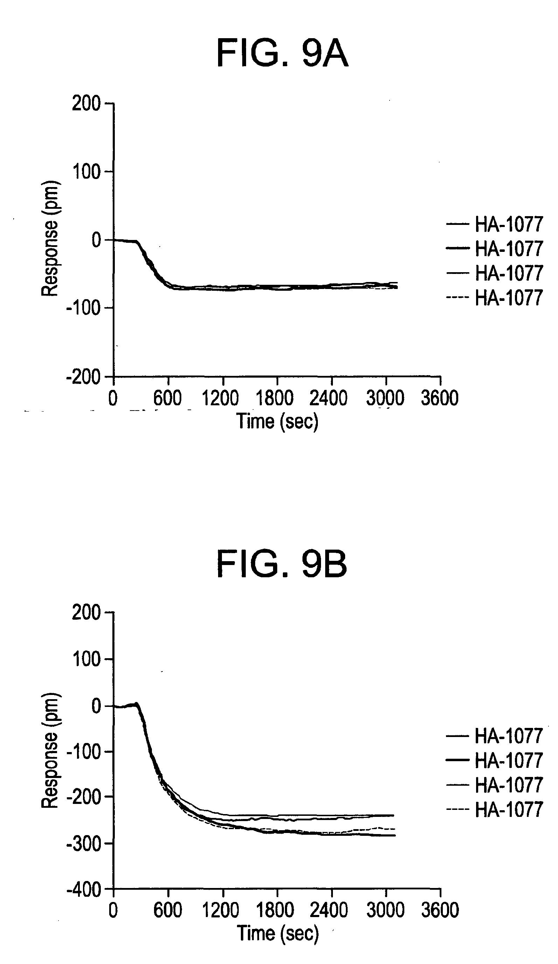

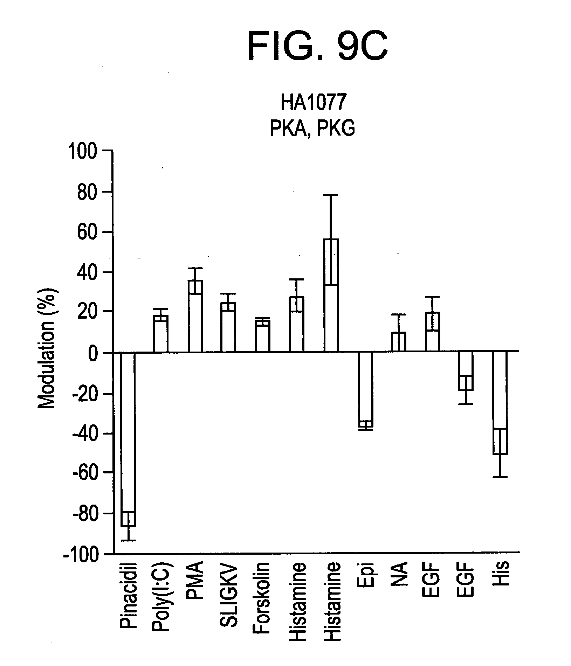

12. Fig. 9 shows (A) the primary profile of the molecule HA-1077 acting on quiescent A431 cells, (B) the primary profile of HA-1077 acting on A549 cells, and (C) the modulation index of HA1077, in embodiments of the disclosure. The modulation index was generated by comparing specific parameter(s) of a marker-induced optical response in a cell in the absence and presence of HA1077. A positive value indicates that HA-1077 potentiates the specific parameter of the marker optical signal in the cell, while a negative value indicates that HA-1077 inhibits or attenuates the specific parameter of the marker-induced signal in the cell.

13. Fig. 10 shows (A) the primary profile of the molecule H-8 acting on quiescent A431 cells, (B) the primary profile of H-8 acting on A549 cells, and (C) the modulation index of H-8, in embodiments of the disclosure.

14. Fig. 11 shows (A) the primary profile of the molecule LY294002 acting on quiescent A431 cells, (B) the primary profile of LY294002 acting on A549 cells, and (C) the modulation index of LY294002, in embodiments of the disclosure.

15. Fig. 12 shows (A) the primary profile of the molecule quercetin acting on quiescent A431 cells, (B) the primary profile of quercetin acting on A549 cells, and (C) the modulation index of quercetin, in embodiments of the disclosure.

**IV. Detailed Description**

16. The term embodiment means embodiment of the disclosure. Only embodiments falling under the scope of the claims constitute the invention.

**A. Definitions**

**1. Material**

17. Material is the tangible part of something (chemical, biochemical, biological, or mixed) that goes into the makeup of a physical object.

**2. Substance**

18. A substance or like terms is any physical object. A material is a substance. Molecules, ligands, markers, cells, proteins, and DNA can be considered substances. A machine or an article would be considered to be made of substances, rather than considered a substance themselves.

**3. Molecule**

19. As used herein, the terms "molecule" or like terms refers to a biological or biochemical or chemical entity that exists in the form of a chemical molecule or molecule with a definite molecular weight. A molecule or like terms is a chemical, biochemical or biological molecule, regardless of its size.

20. Many molecules are of the type referred to as organic molecules (molecules containing carbon atoms, among others, connected by covalent bonds), although some molecules do not contain carbon (including simple molecular gases such as molecular oxygen and more complex molecules such as some sulfur-based polymers). The general term "molecule" includes numerous descriptive classes or groups of molecules, such as proteins, nucleic acids, carbohydrates, steroids, organic pharmaceuticals, small molecule, receptors, antibodies, and lipids. When appropriate, one or more of these more descriptive terms (many of which, such as "protein," themselves describe overlapping groups of molecules) will be used herein because of application of the method to a subgroup of molecules, without detracting from the intent to have such molecules be representative of both the general class "molecules" and the named subclass, such as proteins. Unless specifically indicated, the word "molecule" would include the specific molecule and salts thereof, such as pharmaceutically acceptable salts.

**4. Ligand**

21. A ligand or like terms is a substance or a composition or a molecule that is able to bind to and form a complex with a biomolecule to serve a biological purpose. Actual irreversible covalent binding between a ligand and its target molecule is rare in biological systems. Ligand binding to receptors alters the chemical conformation, i.e., the three dimensional shape of the receptor protein. The conformational state of a receptor protein determines the functional state of the receptor. The tendency or strength of binding is called affinity. Ligands include substrates, blockers, inhibitors, activators, and neurotransmitters. Radioligands are radioisotope labeled ligands, while fluorescent ligands are fluorescently tagged ligands; both can be considered as ligands are often used as tracers for receptor biology and biochemistry studies. Ligand and modulator are used interchangeably.

**5. Marker**

22. A marker or like terms is a ligand which produces a signal in a biosensor cellular assay. The signal is, must also be, characteristic of at least one specific cell signaling pathway(s) and/or at least one specific cellular process(es) mediated through at least one specific target(s). The signal can be positive, or negative, or any combinations (e.g., oscillation).

## 6. Molecule mixture

23. A molecule mixture or like terms is a mixture containing at least two molecules. The two molecules can be, but not limited to, structurally different (i.e., enantiomers), or compositionally different (e.g., protein isoforms, glycoform, or an antibody with different poly(ethylene glycol) (PEG) modifications), or structurally and compositionally different (e.g., unpurified natural extracts, or unpurified synthetic compounds).

## 7. Unknown molecule

24. An unknown molecule or like terms is a molecule with unknown biological/pharmacological/physiological/pathophysiological activity.

## 8. Known molecule

25. A known molecule or like terms is a molecule with known pharmacological/biological/physiological/pathophysiological activity whose precise mode of action(s) may be known or unknown.

## 9. Test molecule

26. A test molecule or like terms is a molecule which is used in a method to gain some information about the test molecule. A test molecule can be an unknown or a known molecule.

## 10. Drug candidate molecule

27. A drug candidate molecule or like terms is a test molecule which is being tested for its ability to function as a drug or a pharmacophore. This molecule may be considered as a lead molecule.

## 11. Modulate

28. To modulate, or forms thereof, means either increasing, decreasing, or maintaining a cellular activity mediated through a cellular target. It is understood that wherever one of these words is used it is also disclosed that it could be, for example, 1%, 5%, 10%, 20%, 50%, 100%, 500%, or 1000% increased from a control, or it could be, for example, 1%, 5%, 10%, 20%, 50%, or 100% decreased from a control.

## 12. Modulator

29. A modulator or like terms is a ligand that controls the activity of a cellular target. It is a signal modulating molecule binding to a cellular target, such as a target protein.

## 13. Known modulator

30. A known modulator or like terms is a modulator where at least one of the targets is known with a known affinity. For example, a known modulator could be a PI3K inhibitor, a PKA inhibitor, a GPCR antagonist, a GPCR agonist, a RTK inhibitor, an epidermal growth factor receptor neutralizing antibody, or a phosphodiesterase inhibition, a PKC inhibitor or activator, etc.

## 14. Cell

31. Cell or like term refers to a small usually microscopic mass of protoplasm bounded externally by a semipermeable membrane, optionally including one or more nuclei and various other organelles, capable alone or interacting with other like masses of performing all the fundamental functions of life, and forming the smallest structural unit of living matter capable of functioning independently including synthetic cell constructs, cell model systems, and like artificial cellular systems.

32. A cell can include different cell types, such as a cell associated with a specific disease, a type of cell from a specific origin, a type of cell associated with a specific target, or a type of cell associated with a specific physiological function. A cell can also be a native cell, an engineered cell, a transformed cell, an immortalized cell, a primary cell, an embryonic stem cell, an adult stem cell, a cancer stem cell, or a stem cell derived cell.

33. Human consists of about 210 known distinct cell types. The numbers of types of cells can almost unlimited, considering how the cells are prepared (e.g., engineered, transformed, immortalized, or freshly isolated from a human body) and where the cells are obtained (e.g., human bodies of different ages or different disease stages, etc).

## 15. Matrix

34. A matrix or like terms is something that constitutes the point from which something else originates, takes form, or develops, according to certain rules. The matrix can have a considerable effect on the way the analysis is conducted and the quality of the results obtained. For example, in embodiments of the disclosure, the matrice for selecting a panel of cells for characterizing molecules includes, but is not limited to, for example, a specific disease (e.g., panels of cells responsible for allergic reactions, or for inflammatory diseases, or for pathogenic infection, or for a breast cancer, or for a skin cancer, or for a colon cancer, or for a liver disease, or for a pancreatic cancer, or for a heart disease, etc), or for a specific origin (e.g., panels of neuronal cells, or lung cells, or skin cells, or muscle cells, or liver cells, etc), or for a specific cellular targets (e.g., a receptor, or an enzyme, or a kinase, or an oncogene, or a structural protein, or a DNA, or a RNA), or for a broad spectrum of types of cells representative to human physiology and pathophysiology (e.g., panels of cells consisting of a Keratinizing epithelial cell, a Wet stratified barrier epithelial cell, an exocrine secretory epithelial cell, a hormone secreting cell, a metabolism and storage cell, a barrier function cell (lung, gut, exocrine glands and urogenital tract), an epithelial cell lining closed internal body cavities, a ciliated cell with propulsive function, an extracellular matrix secretion cell, a contractile cell, a blood and immune system cell, a sensory transducer cell, an autonomic neuron cell, a sense organ and peripheral neuron supporting cell, a central nervous system neurons and glial cell, a lens cell, a pigment cell, a germ cell, a nurse cell, and an interstitial cell). In again another embodiment, a mixed population of at least two types of cells can be used as a cell system.

## 16. Cell culture

35. "Cell culture" or "cell culturing" refers to the process by which either prokaryotic or eukaryotic cells are grown under controlled conditions. "Cell culture" not only refers to the culturing of cells derived from multicellular eukaryotes, especially animal cells, but also the culturing of complex tissues and organs.

## 17. Biosensor

36. Biosensor or like terms refer to a device for the detection of an analyte that combines a biological component with a physicochemical detector component. The biosensor typically consists of three parts: a biological component or element (such as tissue, microorganism, pathogen, cells, or combinations thereof), a detector element (works in a physicochemical way such as optical, piezoelectric, electrochemical, thermometric, or magnetic), and a transducer associated with both components. The biological component or element can be, for example, a living cell, a pathogen, or combinations thereof. In embodiments, an optical biosensor can comprise an optical transducer for converting a molecular recognition or molecular stimulation event in a living cell, a pathogen, or combinations thereof into a quantifiable signal.

## 18. Assaying

37. Assaying, assay, or like terms refers to an analysis to determine a characteristic of a substance, such as a molecule or a cell, such as for example, the presence, absence, quantity, extent, kinetics, dynamics, or type of an a cell's optical or bioimpedance response upon stimulation with one or more exogenous stimuli, such as a ligand or marker. Producing a biosensor signal of a cell's response to a stimulus can be an assay.

## 19. Long term assay

38. "Long term assay" or like terms is used for studying the long-term impact of a given molecule on a living cell. A particular type of long term assay is a "long-term biosensor cellular assay." In one embodiment, each type of cell is exposed to the molecule only for a long period of time (e.g., 8hrs, 16 hrs, 24 hrs, 32hrs, 48hrs, 72hrs). This long-term assay is used to determine the impact of the molecule on the cell healthy state (e.g., viability, apoptosis, cell cycle regulation, cell adhesion regulation, proliferation). Also this long-term assay contains early cell signaling response (e.g., 30min, 60min, 120min, 180min after molecule stimulation), which can be used directly to study the molecule-induced cell signaling events or pathways.

39. In another embodiment, a long-term biosensor cellular assay in the presence of a marker is used to study the cross regulation of the long-term impacts on cell biology and physiology between the molecule and the marker. The marker(s) can be added before, at, and after the molecule. For example, when a marker (e.g., $H_2O_2$) triggers the apoptosis of at least one type of cells in the cell panel, one can use such long-term assays to determine whether the molecule is protective or not. The reverse is also true that such long-terms assays can be used to determine the protective or synergistic role of a marker against a molecule-induced cellular event (e.g., apoptosis, or necrosis).

## 20. Short term assay

40. A "short term assay" or like terms is used for studying the short-term impact of a given molecule on a living cell. A particular type of short term assay is a "short-term biosensor cellular assay." In one embodiment, each type of cell is exposed to the molecule only for a short period of time (e.g., 5min, 10min, 30min, 45min, 60min, 90min, 180min, 240min). This short-term assay is often used for detecting early cell signaling response, which can be used directly to study the molecule-induced cell signaling events or pathways or to study the ability of the molecule to modulate a marker-induced cellular response.

## 21. Two step assay

41. A "two-step assay" or like terms is used, while each type of the cells in the cell panel is exposed to a molecule first to study the molecule-induced biosensor signal, followed by a specific marker or a panel of markers to study the ability of the molecule to modulate the marker-induced biosensor signal(s). This assay can be referred to as a two-mode assay: such as the initial agonism mode and the subsequent antagonism mode. mode of actions (e.g., targets, agonism or antagonism, and potency or efficacy) of the molecule.

## 22. Agonist and antagonist mode

42. The agonism mode or like terms is the assay wherein the cells are exposed to a molecule to determine the ability of the molecule to trigger biosensor signals such as DMR signals, while the antagonism mode is the assay wherein the cells are exposed to a maker in the presence of a molecule to determine the ability of the molecule to modulate the biosensor signal of cells responding to the marker.

### 23. A pulse stimulation assay

43. A "pulse stimulation assay" or like terms can used, wherein the cell is only exposed to a molecule for a very short of time (e.g., seconds, or several minutes). This pulse stimulation assay can be used to study the kinetics of the molecule acting on the cells/targets, as well as its impact on the marker-induced biosensor signals. The pulse stimulation assay can be carried out by simply replacing the molecule solution with the cell assay buffer solution by liquid handling device at a given time right after the molecule addition.

### 24. Treating

44. Treating or treatment or like terms can be used in at least two ways. First, treating or treatment or like terms can refer to administration or action taken towards a subject. Second, treating or treatment or like terms can refer to mixing any two things together, such as any two or more substances together, such as a molecule and a cell. This mixing will bring the at least two substances together such that a contact between them can take place.

45. When treating or treatment or like terms is used in the context of a subject with a disease, it does not imply a cure or even a reduction of a symptom for example. When the term therapeutic or like terms is used in conjunction with treating or treatment or like terms, it means that the symptoms of the underlying disease are reduced, and/or that one or more of the underlying cellular, physiological, or biochemical causes or mechanisms causing the symptoms are reduced. It is understood that reduced, as used in this context, means relative to the state of the disease, including the molecular state of the disease, not just the physiological state of the disease.

### 25. Contacting

46. Contacting or like terms means bringing into proximity such that a molecular interaction can take place, if a molecular interaction is possible between at least two things, such as molecules, cells, markers, at least a compound or composition, or at least two compositions, or any of these with an article(s) or with a machine. For example, contacting refers to bringing at least two compositions, molecules, articles, or things into contact, i.e. such that they are in proximity to mix or touch. For example, having a solution of composition A and cultured cell B and pouring solution of composition A over cultured cell B would be bringing solution of composition A in contact with cell culture B. Contacting a cell with a ligand would be bringing a ligand to the cell to ensure the cell have access to the ligand.

47. It is understood that anything disclosed herein can be brought into contact with anything else. For example, a cell can be brought into contact with a marker or a molecule, a biosensor, and so forth.

### 26. Trigger

48. A trigger or like terms refers to the act of setting off or initiating an event, such as a response.

### 27. Detect

49. Detect or like terms refer to an ability of the apparatus and methods of the disclosure to discover or sense a molecule- or a marker-induced cellular response and to distinguish the sensed responses for distinct molecules.

### 28. Response

50. A response or like terms is any reaction to any stimulation.

### 29. Cellular Response

51. A "cellular response" or like terms is any reaction by the cell to a stimulation.

### 30. Biosensor Response

52. A "biosensor response", "biosensor output signal", "biosensor signal" or like terms is any reaction of a sensor system having a cell to a cellular response. A biosensor converts a cellular response to a quantifiable sensor response. A biosensor response is an optical response upon stimulation as measured by an optical biosensor such as RWG or SPR or it is a bioimpedence response of the cells upon stimulation as measured by an electric biosensor. Since a biosensor response is directly associated with the cellular response upon stimulation, the biosensor response and the cellular response can be used interchangeably, in embodiments of disclosure.

### 31. DMR response

53. A "DMR response" or like terms is a biosensor response using an optical biosensor. The DMR refers to dynamic mass redistribution or dynamic cellular matter redistribution. A P-DMR is a positive DMR response indicative of increased mass located within the sensing zone, a N-DMR is a negative DMR response indicative of decreasing mass located within the sensing zone, and a RP-DMR is a recovery P-DMR response indicative of mass distribution returning to a basal level after a stimulatory event.

## 32. Assaying the response

54. "Assaying the response" or like terms means using a means to characterize the response. For example, if a molecule is brought into contact with a cell, a biosensor can be used to assay the response of the cell upon exposure to the molecule.

## 33. A profile

55. A profile or like terms refers to the data which is collected for a composition, such as a cell. A profile can be collected from a label free biosensor as described herein.

## 34. Primary profile

56. A "primary profile" or like terms refers to a biosensor response or biosensor output signal or profile which is produced when a molecule contacts a cell. Typically, the primary profile is obtained after normalization of initial cellular response to the net-zero biosensor signal (i.e., baseline)

## 35. Secondary profile

57. A "secondary profile" or like terms is a biosensor response or biosensor output signal of cells in response to a marker in the presence of a molecule. A secondary profile can be used as an indicator of the ability of the molecule to modulate the marker-induced cellular response or biosensor response.

## 36. Modulation profile

58. A "modulation profile" or like terms is the comparison between a secondary profile of the marker in the presence of a molecule and the primary profile of the marker in the absence of any molecule. The comparison can be by, for example, subtracting the primary profile from secondary profile or subtracting the secondary profile from the primary profile or normalizing the secondary profile against the primary profile.

## 37. Library

59. A library or like terms is a collection. The library can be a collection of anything disclosed herein. For example, it can be a collection, of indexes, an index library; it can be a collection of profiles, a profile library; or it can be a collection of DMR indexes, a DMR index library; Also, it can be a collection of molecule, a molecule library; it can be a collection of cells, a cell library; it can be a collection of markers, a marker library; A library can be for example, random or non-random, determined or undetermined. For example, disclosed are libraries of DMR indexes or biosensor indexes of known modulators.

## 38. Panel

60. A panel or like terms is a predetermined set of specimens (e.g., markers, or cells, or pathways). A panel can be produced from picking specimens from a library.

## 39. Marker panel

61. A "marker panel" or like terms is a panel which comprises at least two markers. The markers can be for different pathways, the same pathway, different targets, or even the same targets.

## 40. Cell panel

62. A "cell panel" or like terms is a panel which comprises at least two types of cells. The cells can be of any type or combination disclosed herein.

## 41. In the presence of the molecule

63. "in the presence of the molecule" or like terms refers to the contact or exposure of the cultured cell with the molecule. The contact or exposure can be taken place before, or at the time, the stimulus is brought to contact with the cell.

## 42. Biosensor Signal

64. A "biosensor signal" or like terms refers to the signal of cells measured with a biosensor that is produced by the response of a cell upon stimulation.

## 43. DMR signal

65. A "DMR signal" or like terms refers to the signal of cells measured with an optical biosensor that is produced by the response of a cell upon stimulation.

## 44. Normalizing

66. Normalizing or like terms means, adjusting data, or a profile, or a response, for example, to remove at least one common variable. For example, if two responses are generated, one for a marker acting a cell and one for a marker and molecule acting on the cell, normalizing would refer to the action of comparing the marker-induced response in the absence of the molecule and the response in the presence of the molecule, and removing the response due to the marker only, such that the normalized response would represent the response due to the modulation of the molecule against the marker. A modulation comparison is produced by normalizing a primary profile of the marker and a secondary profile of the marker in the presence of a molecule (modulation profile).

## 45. Control

67. The terms control or "control levels" or "control cells" or like terms are defined as the standard by which a change is measured, for example, the controls are not subjected to the experiment, but are instead subjected to a defined set of parameters, or the controls are based on pre- or post-treatment levels. They can either be run in parallel with or before or after a test run, or they can be a pre-determined standard. For example, a control can refer to the results from an experiment in which the subjects or objects or reagents etc are treated as in a parallel experiment except for omission of the procedure or agent or variable etc under test and which is used as a standard of comparison in judging experimental effects. Thus, the control can be used to determine the effects related to the procedure or agent or variable etc. For example, if the effect of a test molecule on a cell was in question, one could a) simply record the characteristics of the cell in the presence of the molecule, b) perform a and then also record the effects of adding a control molecule with a known activity or lack of activity, or a control composition (e.g., the assay buffer solution (the vehicle)) and then compare effects of the test molecule to the control. In certain circumstances once a control is performed the control can be used as a standard, in which the control experiment does not have to be performed again and in other circumstances the control experiment should be run in parallel each time a comparison will be made.

## 46. Positive control

68. A "positive control" or like terms is a control that shows that the conditions for data collection can lead to data collection.

## 47. Modulation comparison

69. A "modulation comparison" or like terms is a result of normalizing a primary profile and a secondary profile.

## 48. Index

70. An index or like terms is a collection of data. For example, an index can be a list, table, file, or catalog that contains one or more modulation profiles. It is understood that an index can be produced from any combination of data. For example, a DMR profile can have a P-DMR, a N-DMR, and a RP-DMR. An index can be produced using the completed date of the profile, the P-DMR data, the N-DMR data, the RP-DMR data, or any point within these, or in combination of these or other data. The index is the collection of any such information. Typically, when comparing indexes, the indexes are of like data, i.e. P-DMR to P-DMR data.

## 49. Biosensor Index

71. A "biosensor index" or like terms is an index made up of a collection of biosensor data. A biosensor index can be a collection of biosensor profiles, such as primary profiles, or secondary profiles. The index can be comprised of any type of data. For example, an index of profiles could be comprised of just an N-DMR data point, it could be a P-DMR data point, or both or it could be an impedence data point. It could be all of the data points associated with the profile curve.

## 50. DMR index

72. A "DMR index" or like terms is a biosensor index made up of a collection of DMR data.

## 51. Molecule biosensor index

73. A "molecule biosensor index" or like terms is a biosensor index produced by data collected for a molecule. For example, a molecule biosensor index can be made up of a profile of the molecule acting on the panel of cells, and the modulation profile of the molecule against the panels of markers, each panel of markers for a cell in the panel of cells.

## 52. Molecule DMR index

74. A "molecule DMR index" or like terms is a DMR index produced by data collected for a molecule. For example, a molecule biosensor index can be made up of a profile of the molecule acting on the panel of cells, and the modulation profile of the molecule against the panels of markers, each panel of markers for a cell in the panel of cells.

## 53. Molecule index

75. A "molecule index" or like terms is an index related to the molecule.

## 54. Modulator biosensor index

76. A "modulator biosensor index" or like terms is a biosensor index produced by data collected for a modulator. For example, a modulator biosensor index can be made up of a profile of the modulator acting on the panel of cells, and the modulation profile of the modulator against the panels of markers, each panel of markers for a cell in the panel of cells.

## 55. Modulator DMR index

77. A "modulator DMR index" or like terms is a DMR index produced by data collected for a modulator.

For example, a modulator DMR index can be made up of a profile of the modulator acting on the panel of cells, and the modulation profile of the modulator against the panels of markers, each panel of markers for a cell in the panel of cells.

## 56. Molecule modulation index

78. A "molecule modulation index" or like terms is an index to display the ability of the molecule to modulate the biosensor output signals of the panels of markers acting on the panel of cells. The modulation index is generated by normalizing a specific biosensor output signal parameter of a response of a cell upon stimulation with a marker in the presence of a molecule against that in the absence of any molecule.

## 57. Known modulator biosensor index

79. A "known modulator biosensor index" or like terms is a modulator biosensor index produced by data collected for a known modulator. For example, a known modulator biosensor index can be made up of a profile of the known modulator acting on the panel of cells, and the modulation profile of the known modulator against the panels of markers, each panel of markers for a cell in the panel of cells.

## 58. Known modulator DMR index

80. A "known modulator DMR index" or like terms is a modulator DMR index produced by data collected for a known modulator. For example, a known modulator DMR index can be made up of a profile of the known modulator acting on the panel of cells, and the modulation profile of the known modulator against the panels of markers, each panel of markers for a cell in the panel of cells.

## 59. Marker biosensor index

81. A "marker biosensor index" or like terms is a biosensor index produced by data collected for a marker. For example, a marker biosensor index can be made up of a profile of the marker acting on the panel of cells, and the modulation profile of the marker against the panels of markers, each panel of markers for a cell in the panel of cells.

## 60. Marker DMR index

82. A "marker biosensor index" or like terms is a biosensor DMR index produced by data collected for a marker. For example, a marker DMR index can be made up of a profile of the marker acting on the panel of cells, and the modulation profile of the marker against the panels of markers, each panel of markers for a cell in the panel of cells.

## 61. Similarity of indexes

83. "Similarity of indexes" or like terms is a term to express the similarity between two indexes, or among at least three indices, one for a molecule, based on the patterns of indices, and/or a matrix of scores. The matrix of scores are strongly related to their counterparts, such as the signatures of the primary profiles of different molecules in corresponding cells, and the nature and percentages of the modulation profiles of different molecules against each marker. For example, higher scores are given to more-similar characters, and lower or negative scores for dissimilar characters. Because there are only three types of modulation, positive, negative and neutral, found in the molecule modulation index, the similarity matrices are relatively simple. For example, a simple matrix will assign identical modulation (e.g., a positive modulation) a score of +1 and non-identical modulation a score of -1.

84. Alternatively, different scores can be given for a type of modulation but with different scales. For example, a positive modulation of 10%, 20%, 30%, 40%, 50%, 60%, 100%, 200%, etc, can be given a score of +1, +2, +3, +4, +5, +6, +10, +20, correspondingly. Conversely, for negative modulation, similar but in opposite score can be given. Following this approach, the modulation index of HA- 1077 against panels of markers, as shown in Figure 9C, illustrates that the PKA/PKG inhibitor HA1077 modulates differently the biosensor response induced by different markers: pinacidil (- 86%), poly (I: C) (+18%), PMA (+36%), SLIGKV- amide (+22%), forskolin (+18%), histamine (early P- DMR, +26%), histamine (late P- DMR, +55%), all in A549 cell; and epinephrine (- 38%), nicotinic acid (+8%), EGF (P- DMR, +19%), EGF (N- DMR, -20%), and histamine (- 50%), all in quiescent A431 cells. Thus, the score of HA1077 modulation index in coordination can be assigned as (- 8.6, 1.8, 3.6, 2.2, 1.8, 2.6, 5.5, -3.8, 0.8, 1.9, -2, -5) . On the other hand, for H8, its score in coordination is (- 7.2, 1.8, 2.2, 3.2, 2, 3.8, 6.5, -1.6, 1, 0.3, -2, 1.6) .

85. The indexes of HA1077 and H8 can be compared based on (1) the simple score matrix wherein their primary indexes in both A549 and A431 are similar (with a score, +1, +1), and the modulation index are similar (with a score, +1, +1, +1, +1, +1, +1, +1, +1, +1, +1, +1, -1); (2) the score matrix based on scale, wherein both score coordination are similar, but no identical, possibly due to their potency difference acting on a cellular target. It is worthy of noting that both indexes are generated using a same dose (10 $\mu$M); and (3) sorting using a significant modulation profile (e.g., the pinacidil response), wherein both molecules led to similar modulation. Taking together, one can conclude that both HA1077 and H8 exhibit similar primary mode(s) of action in the two cell lines examined. Similarly, both LY294002 and quercetin also exhibit similar primary mode(s) of action in the two cell lines examined. However, HA1077/H8 and LY294002/quercetin exhibit different mode(s) of action.

### 62. Characterizing

86. Characterizing or like terms refers to gathering information about any property of a substance, such as a ligand, molecule, marker, or cell, such as obtaining a profile for the ligand, molecule, marker, or cell.

### 63. Potentiate

87. Potentiate, potentiated or like terms refers to an increase of a specific parameter of a biosensor response of a marker in a cell caused by a molecule. By comparing the primary profile of a marker with the secondary profile of the same marker in the same cell in the presence of a molecule, one can calculate the modulation of the marker-induced biosensor response of the cells by the molecule. A positive modulation means the molecule to cause increase in the biosensor signal induced by the marker.

### 64. Higher and inhibit and like words

88. The terms higher, increases, elevates, or elevation or like terms or variants of these terms, refer to increases above basal levels, e.g., as compared a control. The terms low, lower, reduces, decreases or reduction or like terms or variation of these terms, refer to decreases below basal levels, e.g., as compared to a control. For example, basal levels are normal in vivo levels prior to, or in the absence of, or addition of a molecule such as an agonist or antagonist to a cell. Inhibit or forms of inhibit or like terms refers to to reducing or suppressing.

### 65. Molecule pharmacology

89. Molecule pharmacology or the like terms refers to the systems cell biology or systems cell pharmacology or mode(s) of action of a molecule acting on a cell. The molecule pharmacology is often characterized by, but not limited, toxicity, ability to influence specific cellular process(es) (e.g., proliferation, differentiation, reactive oxygen species signaling), or ability to modulate a specific cellular target (e.g, PI3K, PKA, PKC, PKG, JAK2, MAPK, MEK2, or actin).

### 66. Receptor

90. A receptor or like terms is a protein molecule embedded in either the plasma membrane or cytoplasm of a cell, to which a mobile signaling (or "signal") molecule may attach. A molecule which binds to a receptor is called a "ligand," and may be a peptide (such as a neurotransmitter), a hormone, a pharmaceutical drug, or a toxin, and when such binding occurs, the receptor goes into a conformational change which ordinarily initiates a cellular response. However, some ligands merely block receptors without inducing any response (e.g. antagonists). Ligand-induced changes in receptors result in physiological changes which constitute the biological activity of the ligands.

### 67. Cellular target

91. A "cellular target" or like terms is a biopolymer such as a protein or nucleic acid whose activity can be modified by an external stimulus. Celluar targets are most commonly proteins such as enzymes, kinases, ion channels, and receptors.

### 68. Signaling pathway(s)

92. A "defined pathway" or like terms is a path of a cell from receiving a signal (e.g., an exogenous ligand) to a cellular response (e.g., increased expression of a cellular target). In some cases, receptor activation caused by ligand binding to a receptor is directly coupled to the cell's response to the ligand. For example, the neurotransmitter GABA can activate a cell surface receptor that is part of an ion channel. GABA binding to a GABA A receptor on a neuron opens a chloride-selective ion channel that is part of the receptor. GABA A receptor activation allows negatively charged chloride ions to move into the neuron which inhibits the ability of the neuron to produce action potentials. However, for many cell surface receptors, ligand-receptor interactions are not directly linked to the cell's response. The activated receptor must first interact with other proteins inside the cell before the ultimate physiological effect of the ligand on the cell's behavior is produced. Often, the behavior of a chain of several interacting cell proteins is altered following receptor activation. The entire set of cell changes induced by receptor activation is called a signal transduction mechanism or pathway. The signaling pathway can be either relatively simple or quite complicated.

### 69. Molecule-treated cell

93. A molecule-treated cell or like terms is a cell that has been exposed to a molecule.

### 70. Cellular process

94. A cellular process or like terms is a process that takes place in or by a cell. Examples of cellular process include, but not limited to, proliferation, apoptosis, necrosis, differentiation, cell signal transduction, polarity change, migration, or transformation.

### 71. "Period of time"

95. A "period of time" refers to any period representing a passage of time. For example, 1 second, 1 minute, 1 hour, 1 day, and 1 year are all periods of time.

## 72. "Short period of time"

96. A "short period of time" or like terms is a time period between 1 min to 300min.

## 73. "another period of time"

97. An "another period of time" or like terms is a period of time sequentially occurring after a period of time.

## 74. "across the panel of cells and against the panels of markers"

98. The phrase "across the panel of cells and against the panels of markers" refers to a systematic process to examine the primary profiles of a molecule acting on each cell in the panel of cells, as well as the modulation profiles of the molecule to modulate the panels of markers. For a marker/cell pair, the process starts with first examining the primary profile of a molecule independently acting on each type of cells, followed by examining the secondary profile of a maker in the presence of the molecule in the same cell. The term "against" is specifically used to manifest the ability of the molecule to modulate the marker-induced biosensor response.

## 75. indicator for the mode of action of the molecule

99. An "indicator" or like terms is a thing that indicates. Specifically, "an indicator for the mode of action of the molecule" means a thing, such as the similarity of biosensor index of a molecule in comparison with a biosensor index of a well-known modulator, that can be interpreted that the molecule and the well-known modulator share similar mode of action.

## 76. "representative of a particular human physiology and pathophysiolgy"

100. "representative" or like terms is to being an example or type of a certain class or kind of thing. For example, the cellular characteristics of human lung cancer cell line A549 is considered to be representative to physiology of human lung cancer; thus, A549 is used as a model cell line for studying cell biology and physiology of human lung cancers.

## 77. "long-term biosensor signal" ###

101. A "long term biosensor signal" is a biosensor signal produced from a long term assay.

## 78. "long-term DMR signal"

102. A long term DMR signal or like terms is an optical biosensor signal produced from a long term optical biosensor cellular assay.

## 79. Panning

103. Panning or like terms refers to screening a cell or cells for the presence of one or more receptors or cellular targets.

## 80. "Robust biosensor signal"

104. A "robust biosensor signal" is a biosensor signal whose amplitude(s) is significantly (such as 3x, 10x, 20x, 100x, or 1000x) above either the noise level, or the negative control response. The negative control response is often the biosensor response of cells after addition of the assay buffer solution (i.e., the vehicle). The noise level is the biosensor signal of cells without further addition of any solution. It is worthy of noting that the cells are always covered with a solution before addition of any solution.

## 81. "Robust DMR signal"

105. A "robust DMR signal" or like terms is a DMR form of a "robust biosensor signal."

## 82. Potency

106. Potency or like terms is a measure of molecule activity expressed in terms of the amount required to produce an effect of given intensity. For example, a highly potent drug evokes a larger response at low concentrations. The potency is proportional to affinity and efficacy. Affinity is the ability of the drug molecule to bind to a receptor.

## 83. Efficacy

107. Efficacy or like terms is the capacity to produce a desired size of an effect under ideal or optimal conditions. It is these conditions that distinguish efficacy from the related concept of effectiveness, which relates to change under real-life conditions. Efficacy is the relationship between receptor occupancy and the ability to initiate a response at the molecular, cellular, tissue or system level.

## 84. Defined pathway(s)

108. A "defined pathway" or like terms is a specific pathway, such as Gq pathway, Gs pathway, Gi pathway, EGFR pathway, or PKC pathway.

## 85. Network interaction

109. A "network interaction" or like terms is an interaction between at least two specific signaling cascades or pathways. For example, the activation of bradykinin B2 receptor in A431 cells leads to at least dual signaling pathways: Gq and Gs pathways, wherein the two pathways can cross-regulated each other. Such cross-regulation is a type of network interaction. Another example is EGFR signaling in A431 cells, which involves complex multi-component signal transduction pathways. These pathways provide opportunities for feedback, signal amplification, and interactions inside one cell between multiple signals and signaling pathways, primarily through network interactions.

## 86. Chemical biology approach

110. "chemical biology approach" or like terms is a scientific approach that involves the application of chemical techniques and tools, often compounds produced through synthetic chemistry, to the study and manipulation of biological systems. Some forms of chemical biology attempt to answer biological questions by directly probing living systems at the chemical level. In contrast to research using biochemistry, genetics, or molecular biology, where mutagenesis can provide a new version of the organism or cell of interest, chemical biology studies sometime probe systems in vitro and in vivo with small molecules that have been designed for a specific purpose or identified on the basis of biochemical or cell-based screening.

## 87. Cell biology approaches

111. A "cell biology approach" or like terms is a scientific approach that involves studies cells - their physiological properties, their structure, the organelles they contain, interactions with their environment, their life cycle, division and death. This is done both on a microscopic and molecular level. Knowing the components of cells and how cells work is fundamental to all biological sciences.

## 88. Biosensor cellular assay-centered cell profile pharmacology

112. A "biosensor cellular assay-centered cell profile pharmacology" or like terms is a method to determine the pharmacology of molecules using label-free biosensor cellular assays.

## 89. Systems biology

113. "Systems biology" or like terms is the 'systematic' interrogation of the biological processes within the complex, physiological milieu in which they function.

## 90. Systems pharmacology

114. "Systems pharmacology" or like terms is using systems biology in the pursuit of a pharmacology goal.

## 91. Modulate the biosensor signal of a marker

115. "Modulate the biosensor signal or like terms is to cause changes of the biosensor signal or profile of a cell in response to stimulation with a marker.

## 92. Modulate the DMR signal

116. "Modulate the DMR signal or like terms is to cause changes of the DMR signal or profile of a cell in response to stimulation with a marker.

## 93. Direct action (of a drug candidate molecule)

117. A "direct action" or like terms is a result (of a drug candidate molecule") acting independently on a cell.

## 94. Cell profile pharmacology

118. The "cell profile pharmacology" or like terms uses a label-free biosensor, particularly an optical biosensor, to generate primary profiles of a cell in response to stimulation individually or collectively with a molecule, as well as secondary profiles of a cell in response to stimulation individually or collectively with panels of marker molecules in the absence of the molecule. The collection of both primary profile and the secondary profile, and their resulting modulation profiles is used, independently or collectively, to determine the pharmacology of the molecule.

## 95. Kinetic response of the cells/markers in the absence and presence of a molecule

119. "kinetic response of the cells/markers in the absence and presence of a molecule" or like phrases refers to the entire assay or partial assay time series of cellular responses induced by a marker in the absence and presence of a molecule which can be directly used for examining the pharmacology or mode of action of the molecule, using, for example, pattern recognition analysis.

## 96. Attach

120. "Attach," "attachment," "adhere," "adhered," "adherent," "immobilized", or like terms generally refer to immobilizing or fixing, for example, a surface modifier substance, a compatibilizer, a cell, a ligand candidate molecule, and like entities of the disclosure, to a surface, such as by physical absorption, chemical bonding, and like processes, or combinations thereof. Particularly, "cell attachment," "cell adhesion," or like terms refer to the interacting or binding of cells to a surface, such as by culturing, or interacting with cell anchoring materials, compatibilizer (e.g., fibronectin, collagen, lamin, gelatin, polylysine, etc.), or both. "Adherent cells," "immobilized cells", or like terms refer to a cell or a cell line or a cell system, such as a prokaryotic or eukaryotic cell, that remains associated with, immobilized on, or in certain contact with the outer surface of a substrate. Such types of cells after culturing can withstand or survive washing and medium exchanging processes staying adhered, a process that is prerequisite to many cell-based assays.

## 97. Weakly adherent cells

121. "Weakly adherent cells" refers to a cell or a cell line or a cell system, such as a prokaryotic or eukaryotic cell, which weakly interacts, or associates or contacts with the surface of a substrate during cell culture. However, these types of cells, for example, human embryonic kidney (HEK) cells, dissociate from the surface of a substrate by the physically disturbing approach of washing or medium exchange.

## 98. Suspension cells

122. "Suspension cells" refers to a cell or a cell line that is preferably cultured in a medium wherein the cells do not attach or adhere to the surface of a substrate during the culture. However, suspension cells can, in general, be brought to contact with the biosensor surface, by either chemical (e.g., covalent attachment, or antibody-cell surface receptor interactions), or physical means (e.g., settlement down, due to the gravity force, the bottom of a well wherein a biosensor is embedded). Thus, suspension cells can also be used for biosensor cellular assays.

## 99. Subject

123. As used throughout, by a subject or like terms is meant an individual. Thus, the "subject" can include, for example, domesticated animals, such as cats, dogs, etc., livestock (e.g., cattle, horses, pigs, sheep, goats, etc.), laboratory animals (e.g., mouse, rabbit, rat, guinea pig, etc.) and mammals, non-human mammals, primates, non-human primates, rodents, birds, reptiles, amphibians, fish, and any other animal. In one aspect, the subject is a mammal such as a primate or a human. The subject can be a non-human.

## 100. Optional

124. "Optional" or "optionally" or like terms means that the subsequently described event or circumstance can or cannot occur, and that the description includes instances where the event or circumstance occurs and instances where it does not. For example, the phrase "optionally the composition can comprise a combination" means that the composition may comprise a combination of different molecules or may not include a combination such that the description includes both the combination and the absence of the combination (i.e., individual members of the combination).

## 101. A

125. As used in the specification and the appended claims, the singular forms "a," "an" and "the" or like terms include plural referents unless the context clearly dictates otherwise. Thus, for example, reference to "a pharmaceutical carrier" includes mixtures of two or more such carriers, and the like.

## 102. Abbreviations

126. Abbreviations, which are well known to one of ordinary skill in the art, may be used (e.g., "h" or "hr" for hour or hours, "g" or "gm" for gram(s), "mL" for milliliters, and "rt" for room temperature, "nm" for nanometers, "M" for molar, and like abbreviations).

## 103. Ranges

127. Ranges can be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that when a value is disclosed that "less than or equal to" the value, "greater than or equal to the value" and possible ranges between values are also disclosed, as appropriately understood by the skilled artisan. For example, if the value "10" is disclosed the "less than or equal to 10"as well as "greater than or equal to 10" is also disclosed. It is also understood that the throughout the application, data is provided in a number of different formats, and that this data, represents endpoints and starting points, and ranges for any combination of the data points. For example, if a particular data point "10" and a particular data point 15 are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

## 104. Comprise

128. Throughout the description and claims of this specification, the word "comprise" and variations of the word, such as "comprising" and "comprises," means "including but not limited to," and is not intended to exclude, for example, other additives, components, integers or steps.

## 105. Consisting essentially of

129. "Consisting essentially of" in embodiments refers, for example, to a surface composition, a method of making or using a surface composition, formulation, or composition on the surface of the biosensor, and articles, devices, or apparatus of the disclosure, and can include the components or steps listed in the claim, plus other components or steps that do not materially affect the basic and novel properties of the compositions, articles, apparatus, and methods of making and use of the disclosure, such as particular reactants, particular additives or ingredients, a particular agents, a particular cell or cell line, a particular surface modifier or condition, a particular ligand candidate, or like structure, material, or process variable selected. Items that may materially affect the basic properties of the components or steps of the disclosure or may impart undesirable characteristics to the present disclosure include, for example, decreased affinity of the cell for the biosensor surface, aberrant affinity of a stimulus for a cell surface receptor or for an intracellular receptor, anomalous or contrary cell activity in response to a ligand candidate or like stimulus, and like characteristics.

## 106. Stable

130. When used with respect to pharmaceutical compositions, the term "stable" or like terms is generally understood in the art as meaning less than a certain amount, usually 10%, loss of the active ingredient under specified storage conditions for a stated period of time. The time required for a composition to be considered stable is relative to the use of each product and is dictated by the commercial practicalities of producing the product, holding it for quality control and inspection, shipping it to a wholesaler or direct to a customer where it is held again in storage before its eventual use. Including a safety factor of a few months time, the minimum product life for pharmaceuticals is usually one year, and preferably more than 18 months. As used herein, the term "stable" references these market realities and the ability to store and transport the product at readily attainable environmental conditions such as refrigerated conditions, 2°C to 8°C.

## 107. Components

131. Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these molecules may not be explicitly disclosed, each is specifically contemplated and described herein. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

## 108. Mimic

132. As used herein, "mimic" or like terms refers to performing one or more of the functions of a reference object. For example, a molecule mimic performs one or more of the functions of a molecule.

## 109. Or

133. The word "or" or like terms as used herein means any one member of a particular list and also includes any combination of members of that list.

## 110. Publications

134. Throughout this application, various publications are referenced. The disclosures of these publications in their entireties are hereby incorporated by reference into this application in order to more fully describe the state of the art to which this pertains. The references disclosed are also individually and specifically incorporated by reference herein for the material contained in them that is discussed in the sentence in which the reference is relied upon.

## 111. Sample

135. By sample or like terms is meant an animal, a plant, a fungus, etc.; a natural product, a natural product extract, etc.; a tissue or organ from an animal; a cell (either within a subject, taken directly from a subject, or a cell maintained in culture or from a cultured cell line); a cell lysate (or lysate fraction) or cell extract; or a solution containing one or more molecules derived from a cell or cellular material (e.g. a polypeptide or nucleic acid), which is assayed as described herein. A sample may also be any body fluid or excretion (for example, but not limited to, blood, urine, stool, saliva, tears, bile) that contains cells or cell components.

## 112. About

136. About modifying, for example, the quantity of an ingredient in a composition, concentrations, volumes, process temperature, process time, yields, flow rates, pressures, and like values, and ranges thereof, employed in describing the embodiments of the disclosure, refers to variation in the numerical quantity that can occur, for example, through typical measuring and handling procedures used for making compounds, compositions, concentrates or use formulations; through inadvertent error in these procedures; through differences in the manufacture, source, or purity of starting materials or ingredients used to carry out the methods; and like considerations. The term "about" also encompasses amounts that differ due to aging of a composition or formulation with a particular initial concentration or mixture, and amounts that differ due to mixing or processing a composition or formulation with a particular initial concentration or mixture. Whether modified by the term "about" the claims appended hereto include equivalents to these quantities.

## 113. Values

137. Specific and preferred values disclosed for components, ingredients, additives, cell types, markers, and like aspects, and ranges thereof, are for illustration only; they do not exclude other defined values or other values within defined ranges. The compositions, apparatus, and methods of the disclosure include those having any value or any combination of the values, specific values, more specific values, and preferred values described herein.
13 8. Thus, the disclosed methods, compositions, articles, and machines, can be combined in a manner to comprise, consist of, or consist essentially of, the various components, steps, molecules, and composition, and the like, discussed herein. They can be used, for example, in methods for characterizing a molecule including a ligand as defined herein; a method of producing an index as defined herein; or a method of drug discovery as defined herein.

## 114. Compounds and compositions

139. Compounds and compositions have their standard meaning in the art. It is understood that wherever, a particular designation, such as a molecule, substance, marker, cell, or reagent compositions comprising, consisting of, and consisting essentially of these designations are disclosed. Thus, where the particular designation marker is used, it is understood that also disclosed would be compositions comprising that marker, consisting of that marker, or consisting essentially of that marker. Where appropriate wherever a particular designation is made, it is understood that the compound of that designation is also disclosed. For example, if particular biological material, such as EGF, is disclosed EGF in its compound form is also disclosed.

## B. Compositions, methods, articles, and machines

140. The pharmaceutical and biotech industries are challenged by seemingly opposing goals: (1) achieving lower attrition rates for new drugs and (2) reducing the introduction time of new drugs into the market. Drug discovery requires selecting an elusive molecule with desired pharmacological and physiological qualities out of a nearly unlimited number of chemical entities. Unfortunately, the selection of a drug can be an extremely costly and an intrinsically low efficiency process. Despite substantial investment in advanced technologies, the number of new drug approvals has remained low in the recent years. The current R&D productivity gap - the increasing amount of pharmaceutical R&D spending relative to the number of new drug candidates introduced per year - has generated widespread concern, and several divergent opinions about the problem and its potential solutions.
141. To exacerbate the situation, recent advances in genomics and proteomics have significantly increased the number of potential targets for new drugs. Target-oriented drug discovery techniques, despite previous successes against known targets, have often failed to deliver drugs against new targets (i.e. molecules not the targets of previous drugs). Significantly, over the past decade, the entire industry has averaged only two to three small-molecule drugs against such "innovative" targets per year. As a result, many companies are reexamining the tools, techniques, and practices used in drug discovery and development. This introspection has highlighted the need for systems biology and systems pharmacology-based assessment and validation of drug actions, and for more physiologically relevant technologies, particularly in drug discovery.

## 1. Method for Characterizing a Molecule

142. Disclosed are methods of using label- free biosensor cellular assays for drug discovery. Specifically, disclosed are methods relating to label- free biosensor cellular assay- centered cell profile pharmacology for drug discovery. The present methods involve the use of label- free biosensors to determine the systems cell pharmacology of a drug candidate molecule by directly monitoring its actions on panels of different types of cells representative to human physiology and human pathophysiology, as well as determining the ability of the drug candidate molecule to modulate the biosensor signals of each cell in response of stimulation, independently or collectively, with a panel of marker molecules. The direct action of a molecule on a cell leads to its primary profile, while the modulation of the molecule against a marker-induced biosensor signal results in a secondary profile. Both types of profiles are generally recorded as real time kinetic cellular responses. Combining the primary profiles in the absence of a molecule with the secondary profiles in the presence of the molecule across multiple cells on which panels of markers act leads to panels of modulation profiles of the molecule against these markers. The entire or partial panels of modulation profiles, for example, can be combined to produce a modulation index, such as a biosensor index, such as a DMR index. Comparing the molecule index with established indexes of panels of pharmacologically known modulators allows one to determine the cellular receptor (s) or target (s) or pathway (s) with which the molecule intervene (s) . This cell profile pharmacology approach not only provides information regarding the mode of actions (e.g., target (s), pathway (s), agonism or antagonism, or toxicity) of any molecule, but also enables the determination of their potency, selectivity, and systems cell pharmacology.

143. The present drug discovery approaches and methods can use any of the physical components disclosed herein including but not limited to materials, substances, molecules, ligands, markers, molecules, compositions, unknown molecules, known molecules, test molecules, drug candidate molecules, molecule mixtures, modulators, known modulators, cells, agonists, antagonists, libraries, panels, marker panels, cell panels, receptors, cellular targets, defined pathways, molecule-treated cells, signal pathways, attached cells, weakly adherent cells, suspended cells, biosensors, and even subjects.

144. The present drug discovery approaches and methods can use any of the physical/metaphysical or information related components disclosed herein including but not limited to responses, cellular responses, biosensor responses, DMR responses, profiles, primary profiles, secondary profiles, modulation profiles, biosensor signals, DMR signals, controls, positive controls, negative controls, modulator comparisons, indexes, biosensor indexes, DMR indexes, molecule biosensor indexes, molecule DMR indexes, molecule indexes, modulator biosensor indexes, modulator DMR indexes, molecule modulator indexes, known modulator biosensor indexes, known modulator DMR indexes, marker biosensor indexes, marker DMR indexes, marker biosensor indexes, marker indexes, similarity of indexes, matrices, molecule pharmacology, cellular processes, periods of time, short periods of time, another periods of time, indicators, human physiology, human pathophysiology, long term biosensor signals, long term DMR signals, robust biosensor signals, robust DMR signals, potency, efficacy, network interactions, chemical biology, cellular assay-centered cell profile pharmacology, systems cell pharmacology, and cell profile pharmacology.

145. The present drug discovery approaches and methods can use any of the method components disclosed herein including but not limited to modulating, cell culturing, assaying, long term assaying, short term assaying, two step assaying, pulse stimulation assaying, treating, contacting, triggering, detecting, assaying the response, normalizing, characterizing, potentiating, panning, modulating the biosensor signal, modulating the DMR signal, and attaching.

146. It is understood that any of the components can be used in any combination herein, and each permutation is specifically recited herein, at least for the components recited in the above component lists.

147. Disclose are matrices to determine panels of different types of cells for cell profile pharmacology-based drug discovery.

148. In certain embodiments human cells are used to determine the pharmacology of a molecule, such as a drug candidate molecule. It is almost impossible in practice to use all types of human cells for determining the pharmacology of a drug candidate. To ensemble the potential and druggability of a drug candidate molecule, different panels of cells can be chosen and used for specific purposes. For example, panels of broad spectrum types of cells can be used for assessing the potential side effect of the molecule, while the panels of specific disease-related cells can be used for determining the potency and efficacy of the molecule to cure the specific disease. On the other hand, panels of cells from a specific organ can be used to study the specificity of the molecule, while panels of cells for a specific target can be used to determine the broad implications of the molecule acting through the same target. It is known that due to the specific cellular background of each cell, the modulation of the activity of a specific cellular target can lead to distinct cellular responses (often referred to cellular context-dependent "phenotypic" responses). A drug candidate molecule can display functional selectivity to modulate the activity of the same cellular target, thus leading to different cellular responses across different types of cells expressing the same target

**a) Signaling Pathways and Cellular Targets**

149. Disclosed are uses of panels of markers/cellular targets/pathways for each type of cell. The selection of markers/cellular targets/pathways starts with the predetermination of panels of ligands for their ability to trigger a biosensor signal such a DMR signal in each type of cells, followed by chemical biology modulation profiling for determining the system cell biology (e.g., pathway(s) and network interaction involved downstream from the ligand-induced modulation of the target) and systems cell pharmacology (e.g., target specificity and potency and efficacy) of each ligand. A ligand can qualify as a marker, when (1) the ligand produces a robust biosensor signal in a cell using the biosensor cellular assay, (2) the ligand biosensor signal is characteristic of at least one specific cell signaling pathway(s) and/or at least one specific cellular process(es) mediated through at least one specific target(s).

150. The marker(s) can be different for each type of cell in the cell panel. For example, for cell 1, markers A and B and C can be used; while for cell 2, markers D and E and F can be used. In another embodiment, at least one marker is common in at least two types of cells in the panel. For example, histamine can be used as a marker in both A549 and A431 cells in a panel. In other embodiments, the numbers of markers for each cell in the panel can be different (e.g., 1 marker for cell 1, 2 markers for cell 2, 2 markers for cell 3, and 4 markers for cell 4). In other embodiments, the numbers of markers for each cell in the panel can be identical (e.g., 1 marker for each cell, or 2 markers for each cell, or 3 markers for each cell).

151. In embodiments, panels of markers can be chosen to comprehensively cover the majority of cell signaling pathways (such as one maker for Gq pathway, one for $G_i$ pathway, one for $G_s$ pathway, one for EGFR pathway, one for Toll-like receptor pathway, so on so far). In certain embodiments, panels of markers chosen cover a specific set of signaling pathways (e.g., PI3K pathway, Akt pathway, PKA pathway, MAPK pathway, JNK pathway, toll-like receptor pathway, $G_q$ pathway, $G_s$ pathway, $G_i$ pathway, $G_{12/13}$ pathway, EGFR pathway, mTOR pathway, or IKK pathway, etc.), depending on the specific disease of interest (e.g., inflammatory diseases, infectious diseases, cancers, diabetes, obesity, neuronal diseases, autoimmune diseases, airway inflammation in asthma, airway pathology in COPD, Alzheimer's disease, autoimmune causing Vitiligo, colorectal cancer, pancreatic adenocarcinoma, or virus-induced airway inflammation, etc.).

152. The major cell signaling pathways include, but are not limited to, 14-3-3 induced intracellular signaling (e.g., cell cycle regulation, apoptosis), actin-based motility by Rho family GTPases, cAMP-PKA pathway, Gs pathway mediated through GPCRs, Gq pathway mediated through GPCRs, Gi pathway mediated through GPCRs, G12/13 pathway mediated through GPCRs, AIF pathway, Akt signaling, aldosterone signaling in epithelial cells, AMPK enzyme complex pathway, antigen processing and presentation by MHCs, apoptosis through death receptors or by HIV1, bitter taste signaling, cAMP Pathway, CD28 signaling in T-helper cell, cellular apoptosis pathway, ceramide pathway, chemokine signaling, CREB pathway, cyclins and cell cycle regulation, eNOS signaling, ERK signaling, estrogen pathway, FAK signaling, FGF pathway, G-$\beta\gamma$ signaling, GPCR pathway, growth hormone signaling, GSK3 signaling, interleukin (e.g., IL-1, IL-2, IL-22, IL-3, IL-23, IL-9) pathway, iNOS signaling, insulin receptor pathway, integrin pathway, interferon pathway, intracellular calcium signaling, IP3 pathway, JAK-STAT pathway, JNK pathway, MAPK signaling, mitochondrial apoptosis, mTOR pathway, NF-$\kappa$B

pathway, NGF pathway, Notch signaling, p38 signaling, p53 mediated apoptosis, PDGF pathway, phospholipase-C pathway, PI3K signaling, PKA signaling, PKC pathway, PTEN pathway, Rac1 pathway, RANK pathway, Rap1 pathway, Ras pathway, RhoA pathway, RNAi pathway, siRNA pathway, SMAD signaling, STAT3 pathway, SUMO pathway, T-Cell receptor signaling, TGF-Beta pathway, thrombin signaling, TNF signaling, Toll-like receptors pathway, VEGF pathway, and WNT signaling. Since many of these pathways and signaling events share many converging point(s) (termed integrator point or joints) and interact with each other, the biosensor cellular assays disclosed herein can provide information regarding the signaling and network interactions. It is not necessary to have all these pathways included in the panels of markers/targets/pathways for cell profile pharmacology drug discovery as provided herein.

153. Disclosed are uses of multiple types of assays for biosensor cell profile pharmacology-based discovery. In one embodiment, a long-term biosensor cellular assay is used for studying the long-term impact of a molecule on panels of cells. Here each type of cell is exposed to the molecule only for a long period of time (e.g., 8hrs, 16 hrs, 24 hrs, 32hrs, 48hrs, 72hrs). This long-term assay is used to determine the impact of the molecule on a healthy cell state (e.g., viability, apoptosis, cell cycle regulation, cell adhesion degree, proliferation). Also this long-term assay contains early cell signaling responses (e.g., 10min, 30min, 60min, 120min, 180min after molecule stimulation), which can be used directly to study the molecule-induced cell signaling events or pathways.

154. In another embodiment, a long-term biosensor cellular assay in the presence of a marker is used to study the cross regulation of the long-term impacts on cell biology and physiology between the molecule and the marker. The marker(s) can be added before, at, and after the molecule. For example, when a marker (e.g., $H_2O_2$) triggers the apoptosis of at least one type of cell in a cell panel, one can use such long-term assays to determine whether the molecule is protective or not.

155. In another embodiment, at least a two-step assay is used, while each type of cell in the cell panel is exposed to a molecule first to study the molecule-induced biosensor signal such as DMR signal, followed by treating a specific marker or a panel of markers independently. This assay is often referred to as a two-mode assay: the initial agonism mode and the subsequent antagonism mode. The agonism mode is designed to determine the ability of the molecule to trigger a biosensor signal such as DMR signal, while the antagonism mode is designed to determine the ability of the molecule to modulate the biosensor signals such as DMR signals of panels of markers, each panel of markers for a cell type in the cell panel.

156. In another embodiment, a pulse stimulation assay can be used, wherein the cell is only exposed to the molecule for a very short of time (e.g., seconds, or minutes). This pulse stimulation assay can be used to study the kinetics of the molecule acting on the cells/ targets, as well as its impact on the marker-induced DMR signals. The pulse stimulation assay can be carried out by simply replacing the molecule solution with the cell assay buffer solution by a liquid handling device at a given time right after the molecule addition.

## 2. Method for Producing an Index

157. Disclosed are methods to study systems cell pharmacology of a molecule such as a drug candidate molecule. The methods involve the creation of a biosensor signal index such as a DMR index for direct analysis of the systems cell pharmacology of the molecule, or indirect analysis based on pattern recognition between the molecule- and a well-known modulator (e.g., a PI3K inhibitor, a PKA inhibitor, a GPCR antagonist, a GPCR agonist, a RTK inhibitor, an EGFR neutralizing antibody, or a phosphodiesterase inhibition, a PKC inhibitor or activator, etc) induced biosensor signal index such as DMR index.

158. In one embodiment the method comprises: (1) collecting a biosensor response such as a DMR response of a molecule in an agonist mode, (2) collecting biosensor responses such as DMR responses of panels of markers in a cell panel in the absence and presence of the molecule; (3) extracting specific sets of biosensor response parameters such as DMR parameters from each biosensor signal such as DMR signal; (4) normalizing each parameter against a positive control (i.e., the cells treated with the marker in the absence of any molecule) to generate the percentage of modulation (which can either indicate potentiation, or inhibition, or no modulation), (5) plotting the percentage of modulation of each parameter as a function of markers/cells to generate a biosensor signal index of the molecule; and (6) comparing the molecule-induced biosensor signal index with a library of known modulator biosensor signal indexes, or a library of any molecule-induced biosensor signal index, against the panels of cells/markers.

159. In one embodiment the method comprises: (1) collecting a DMR response of a molecule in an agonist mode, (2) collecting DMR responses of panels of markers in a cell panel in the absence and presence of the molecule; (3) extracting specific sets of DMR parameters from each DMR signal; (4) normalizing each DMR parameter against a positive control (i.e., the cells treated with the marker in the absence of any molecule) to generate the percentage of modulation (which can either potentiation, or inhibition, or no modulation), (5) plotting the percentage of modulation of each DMR parameter as a function of markers/cells to generate a DMR index of the molecule; and (6) comparing the molecule-induced DMR index with a library of known modulator DMR indexes, or a library of any molecule-induced DMR index, against the panels of cells/markers.

160. The similarity between the molecule biosensor signal index such as DMR index and a modulator biosensor signal index such as DMR index can be used as an indicator of the modes of action (e.g., targets, agonism or antagonism, and potency or efficacy) of the molecule. The closer the molecule biosensor signal index such as DMR index is to the modulator biosensor signal index such as DMR index, the higher possibility the molecule modulates the same target(s) that the modulator acts on. The molecule biosensor signal such as DMR signal in the agonist mode can be also included in the biosensor signal index such as DMR index.

161. In certain embodiments molecules are identified that have at least 50%, 55%, 60%, 65%, 70%, 75%, 80%, 85%, 90%, 95%, 98%, similarity between one index and another index, such as a molecule index to a known marker index. The similarity can be determined at however many points the index is produced from, such as P-DMR data from a profile. In this example, the similarity can be determined in one of two ways:1) at least for example 80% across all P-DMR data points for each index, or 2) at least 80% similarity at each data point for each index.

162. In certain embodiments, the modes of action of a molecule are identified using conventional pattern recognition analysis of the molecule index, in comparison with a library of marker indexes, modulator indexes, or any molecule indexes.

163. In certain embodiments, the modes of action of a molecule are identified using a matrix of scores. The matrix of scores are strongly related to their counterparts, such as the signatures of the primary profiles of different molecules in corresponding cells, and the nature and percentages of the modulation profiles of different molecules against each marker. For example, higher scores are given to more-similar characters, and lower or negative scores for dissimilar characters. Because there are only three types of modulation, positive, negative and neutral, found in the molecule modulation index, the similarity matrices are relatively simple. For example, a simple matrix will  assign identical modulation (e.g., a positive modulation) a score of +1 and non-identical modulation a score of -1. In alternative embodiments, different scores can be given for a type of modulation but with different scales. For example, a positive modulation of 10%, 20%, 30%, 40%, 50%, 60%, 100%, 200%, etc, can be given a score of +1, +2, +3, +4, +5, +6, +10, +20, correspondingly. Conversely, for negative modulation, similar but in opposite score can be given. Following this approach, the modulation index of HA-1077 against panels of markers, as shown in Figure 9C, illustrates that the PKA/PKG inhibitor HA1077 modulates differently the biosensor response induced by different markers: pinacidil (-95%), poly(I:C) (+18%), PMA (+36%), SLIGKV-amide (+22%), forskolin (+18%), histamine (+26%), all in A549 cell; and epinephrine (-38%), nicotinic acid (+8%), EGF (P-DMR, +19%), EGF (N-DMR, -20%), and histamine (-50%), all in quiescent A431 cells. Thus, the score of HA1077 modulation index in coordination can be assigned as (-9.5, 1.8, 3.6., 2.2, 1.8, 2.6, -3.8, 0.8, 1.9, -2, -5). Similarly, for H8, its score in coordination is (-7.2, 1.8, 2.2, 3.2, 2, 3.8, 6.5, -1.6, 1, 0.3, -2, 1.6). By comparing the scores between HA1077 and H8, one can conclude that both molecules exhibits similar mode(s) of action in the two cell lines examined.

## 3. Method of Drug Discovery

164. Disclosed are methods for systems pharmacology or systems cell pharmacology assessment of any molecule including drug candidate molecules. The method relies on the choice of cells/markers/targets/pathways to create a biosensor index, such as a DMR index, of the molecule. The assembly of the ability of the molecule to trigger biosensor signals, such as DMR signals, as well as to modulate the markers-induced biosensor signals, such as DMR signals, in conjunction with different types of assays, leads to systematically assess the integrative pharmacology of the molecule.

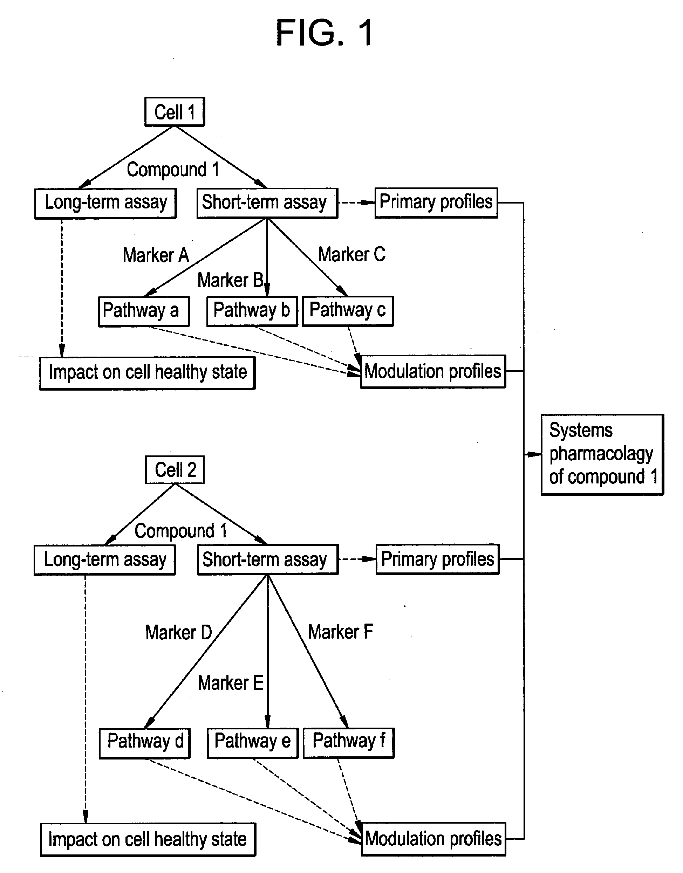

165. Referring to Fig. 1, it is a schematic drawing showing the principle of the disclosed cell profile pharmacology approach for drug discovery. In this example, two types of cells, cell 1 and cell 2, are used. The two types of cells can be related, or not. For each type of cells, two types of assays: a long- term assay with the molecule only, and a short- term such as a two step (or two mode) assay. The long- term assay is used to determine the impact of the molecule on the healthy cell state (e.g., proliferation rate, cell cycle regulation, toxicity, or alterations in cell adhesion, etc) . The two modes assays include the initial agonist mode to determine the molecule- induced biosensor signals, such as DMR signals, in both types of cells, and the subsequent antagonist mode to determine the modulation of the molecule on the biosensor signal, such as DMR signals, induced by a panel of markers for each cell. Here each cell has its own panel of markers, such as marker **A, B and C** for **cell 1,** or makers **D, E** and **F** for the cell **2.** Each maker could lead to signaling through different pathway (s), such as pathway **a, b,** and **c,** correspondingly in cell **1,** or pathway **d, e** and **f,** correspondingly in cell **2.** Alternatively, these pathway (s) could interact with each other, or at least two of them can be similar or identical. The assembly of the modulation profiles can be used to create a modulation index. Together with the primary profiles of the molecule, a biosensor index, such as a DMR index for the molecule against the two types of cells and 6 markers, can be created. The biosensor index, such as a DMR index, is further used to determine the molecule pharmacology, through pattern recognitions against a library of biosensor indexes, such as DMR indexes for a library of known modulators.

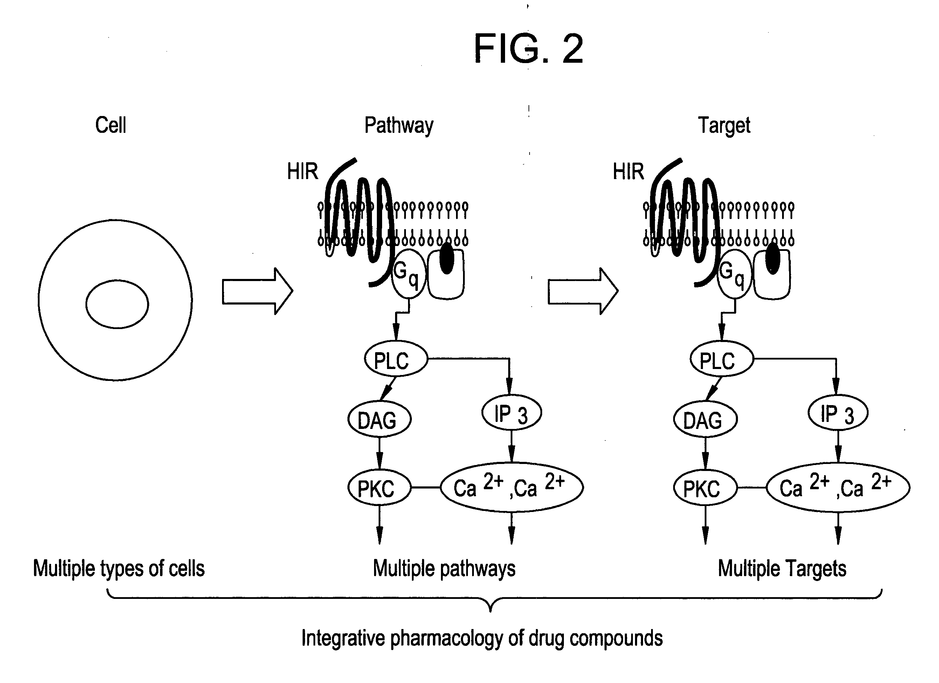

166. Referring to Fig. 2, it is a schematic drawing how the biosensor cell profile pharmacology-based approach is used for molecule pharmacology determination. This approach starts with a cell, and tests the molecule against a panel of markers which trigger cell signaling through specific pathway(s) (e.g., Gq-coupled histamine 1 receptor mediated signaling), and ends up with pattern recognition (or specific data point(s)) through comparison with a library of known modulators-induced biosensor signal indexes, such as DMR indexes, to determine specific target(s) that the molecule acts through. As a result, the integrative pharmacology of the molecule can be determined.

167. The disclosed cell profile pharmacology uses a label-free biosensor, particularly an optical biosensor, to generate primary profiles of a cell in response to stimulation individually or collectively with panels of marker molecules. Each marker molecule activates a receptor or cellular target, leading to a biosensor signal in the cell. Disclosed are methods to determine and screen molecule pharmacology using biosensor-centered measurements, comprising:

168. 1). Selecting panels of cells, wherein the cells are chosen based on a matrix;

169. 2) . Determining panels of cellular targets and markers in each type of cells, wherein the modulation of a cellular target by a marker molecule not only leads to cell signaling through defined pathway (s), but also results in a biosensor signal detected by a label- free biosensor;

170. 3). Treating each cell with a molecule;

171. 3a) treating each cell with panels of markers to generate primary profiles of each marker from the panel.

172. 4). Monitoring the cellular response for a short period of time to generate the primary profile of the molecule;

173. 5). Adding panels of markers individually to the molecule-treated cells, wherein each marker is at a specific dose (eg., $EC_{50}$, $EC_{80}$, $EC_{90}$, $EC_{95}$ or $EC_{100}$), to generate the secondary profile of the molecule impacting the signal of each marker;

174. 6). Monitoring the cellular response for another period of time;

175. 6a) Comparing the secondary profiles of each marker in the presence of the molecule with the respective primary profiles of each maker from the panel of markers

176. 7). Generating a biosensor index of the molecule across the panel of cells and against the panels of markers.

177. 8). Comparing the biosensor index of the molecule with the biosensor index of modulators with known pharmacology, wherein the similarity between the molecule biosensor index and the modulator biosensor index is an indicator for the mode(s) of action of the molecule.

178. 9) Optionally, generating long-term biosensor signal of the molecule in the absence and presence of a marker in a cell.

179. The disclosed cell profile pharmacology uses a label-free optical biosensor to generate primary DMR profiles of a cell in response to stimulation individually or collectively with panels of marker molecules. Each marker molecule activates a receptor or cellular target, leading to a DMR signal in the cell. Disclosed are methods to determine and screen molecule pharmacology using biosensor-centered measurements, comprising:

180. 1). Selecting panels of cells, wherein the cells are chosen based on a matrix;

181. 2) . Determining panels of cellular targets and markers in each type of cells, wherein the modulation of a cellular target by a marker molecule not only leads to cell signaling through defined pathway (s), but also results in a DMR signal detected by a label- free biosensor;

182. 3). Treating each cell with a molecule;

183. 4). Monitoring the cellular DMR response for a short period of time to generate the primary DMR profile of the molecule;

184. 5). Adding panels of markers individually to the molecule-treated cells, wherein each marker is at a specific dose (eg., $EC_{50}$, $EC_{80}$, $EC_{90}$, $EC_{95}$ or $EC_{100}$), to generate the secondary DMR profile of the molecule impacting the signal of each marker;

185. 6). Monitoring the cellular DMR response for another period of time;

186. 7). Generating a DMR index of the molecule across the panel of cells and against the panels of markers.

187. 8). Comparing the DMR index of the molecule with the DMR index of modulators with known pharmacology, wherein the similarity between the molecule DMR index and the modulator DMR index is an indicator for the mode(s) of action of the molecule.

188. 9) Optionally, generating long-term DMR signal of the molecule in the absence and presence of a marker in a cell.

189. Unlike conventional systems biology and systems pharmacology-based drug discovery which highly relies on the use of cell systems and the point-of-contact measures (e.g., cell growth rate, or release of a specific cytokines), the disclosed methods are uses of a panel of different types of native cells or engineered cells, in conjunction with label-free biosensor-based kinetic measurements, for studying molecule pharmacology and pathopharmacology. Conventional systems biology approaches often use large scale analytical tools for characterizing, such as protein microarrays, DNA microarrays, or mass spectroscopy.

190. Also disclosed are methods using native or engineered cell systems for cell profile pharmacology. The disease relevant complexity can be engineered into cell 'systems' by combining different cell types together, and by activating multiple pathways simultaneously to elicit network regulation and emergent properties. For example, a cell systems approach to inflammatory diseases can include combinations of endothelial cells and blood mononuclear cells, and complex cytokine or immune-stimulating environments.

191. Systems biology, and especially the elucidation and dynamic analysis of cellular signaling pathways, provides a new framework for drug discovery. Insight into the combined behavior of these many, diverse, interacting components is achieved through the integration of experimental, mathematical and computational sciences in an iterative approach. Through this contextual understanding of the molecular mechanisms of disease, a systems approach has the potential to further facilitate the identification and validation of the therapeutic modulation of regulatory and metabolic networks and hence help identify targets and biomarkers, as well as 'off-target' and side effects of drug candidates. The disclosed methods allow for using a systems biology approach for studying molecules or substances, for example.

192. The disclosed methods also have the benefits of conventional systems biology-based approaches. For example, the combination of cells, stimuli and readouts in each system, and the combination of systems assayed together can be used to detect and discriminate as many known disease-modulating agents and drugs as possible with a minimal set of measurements. The disclosed drug discovery approach again places biology first, and applies biological insights throughout the discovery process: it identifies hits and optimizes leads on the basis of their elicited biology in complex cell systems or panels of cells that model diverse active cells, pathways and networks in disease states of interest. In this approach, the drug candidate itself, not a target gene or protein, is used for validation.

## 4. Biosensors and Biosensor Assays

193. Label-free cell-based assays generally employ a biosensor to monitor molecule-induced responses in living cells. The molecule can be naturally occurring or synthetic, and can be a purified or unpurified mixture. A biosensor typically utilizes a transducer such as an optical, electrical, calorimetric, acoustic, magnetic, or like transducer, to convert a molecular recognition event or a molecule-induced change in cells contacted with the biosensor into a quantifiable signal. These label-free biosensors can be used for molecular interaction analysis, which involves characterizing how molecular complexes form and disassociate over time, or for cellular response, which involves characterizing how cells respond to stimulation. The biosensors that are applicable to the present methods can include, for example, optical biosensor systems such as surface plasmon resonance (SPR) and resonant waveguide grating (RWG) biosensors, resonant mirrors, ellipsometers, and electric biosensor systems such as bioimpedance systems.

## a) SPR Biosensors and Systems

194. SPR relies on a prism to direct a wedge of polarized light, covering a range of incident angles, into a planar glass substrate bearing an electrically conducting metallic film (e.g., gold) to excite surface plasmons. The resultant evanescent wave interacts with, and is absorbed by, free electron clouds in the gold layer, generating electron charge density waves (i.e., surface plasmons) and causing a reduction in the intensity of the reflected light. The resonance angle at which this intensity minimum occurs is a function of the refractive index of the solution close to the gold layer on the opposing face of the sensor surface

### b) RWG Biosensors and Systems

195. An RWG biosensor can include, for example, a substrate (e.g., glass), a waveguide thin film with an embedded grating or periodic structure, and a cell layer. The RWG biosensor utilizes the resonant coupling of light into a waveguide by means of a diffraction grating, leading to total internal reflection at the solution-surface interface, which in turn creates an electromagnetic field at the interface. This electromagnetic field is evanescent in nature, meaning that it decays exponentially from the sensor surface; the distance at which it decays to $1/e$ of its initial value is known as  the penetration depth and is a function of the design of a particular RWG biosensor, but is typically on the order of about 200 nm. This type of biosensor exploits such evanescent wave to characterize ligand-induced alterations of a cell layer at or near the sensor surface.

196. RWG instruments can be subdivided into systems based on angle-shift or wavelength-shift measurements. In a wavelength-shift measurement, polarized light covering a range of incident wavelengths with a constant angle is used to illuminate the waveguide; light at specific wavelengths is coupled into and propagates along the waveguide. Alternatively, in angle-shift instruments, the sensor is illuminated with monochromatic light and the angle at which the light is resonantly coupled is measured.

197. The resonance conditions are influenced by the cell layer (e.g., cell confluency, adhesion and status), which is in direct contact with the surface of the biosensor. When a ligand or an analyte interacts with a cellular target (e.g., a GPCR, a kinase) in living cells, any change in local refractive index within the cell layer can be detected as a shift in resonant angle (or wavelength).

198. The Corning® Epic® system uses RWG biosensors for label-free biochemical or cell-based assays (Coming Inc., Coming, NY). The Epic® System consists of an RWG plate reader and SBS (Society for Biomolecular Screening) standard microtiter plates. The detector system in the plate reader exploits integrated fiber optics to measure the shift in wavelength of the incident light, as a result of ligand-induced changes in the cells. A series of illumination-detection heads are arranged in a linear fashion, so that reflection spectra are collected simultaneously from each well within a column of a 384-well microplate. The whole plate is scanned so that each sensor can be addressed multiple times, and each column is addressed in sequence. The wavelengths of the incident light are collected and used for analysis. A temperature-controlling unit can be included in the instrument to minimize spurious shifts in the incident wavelength due to the temperature fluctuations. The measured response represents an averaged response of a population of cells. Varying features of the systems can be automated, such as sample loading, and can be multiplexed, such as with a 96 or 386 well microtiter plate. Liquid handling is carried out by either on-board liquid handler, or an external liquid handling accessory. Specifically, molecule solutions are directly added or pipetted into the wells of a cell assay plate having cells cultured in the bottom of each well. The cell assay plate contains certain volume of assay buffer solution covering the cells. A simple mixing step by pipetting up and down certain times can also be incorporated into the molecule addition step.

### c) Electrical Biosensors and Systems

199. Electrical biosensors consist of a substate (e.g., plastic), an electrode, and a cell layer. In this electrical detection method, cells are cultured on small gold electrodes arrayed onto a substrate, and the system's electrical impedance is followed with time. The impedance is a measure of changes in the electrical conductivity of the cell layer. Typically, a small constant voltage at a fixed frequency or varied frequencies is applied to the electrode or electrode array, and the electrical current through the circuit is monitored over time. The ligand-induced change in electrical current provides a measure of cell response. Impedance measurement for whole cell sensing was first realized in 1984. Since then, impedance-based measurements have been applied to study a wide range of cellular events, including cell adhesion and spreading, cell micromotion, cell morphological changes, and cell death. Classical impedance systems suffer from high assay variability due to use of a small detection electrode and a large reference electrode. To overcome this variability, the latest generation of systems, such as the CellKey system (MDS Sciex, South San Francisco, CA) and RT-CES (ACEA Biosciences Inc., San Diego, CA), utilize an integrated circuit having a microelectrode array.

### d) High Spatial Resolution Biosensor Imaging Systems

200. Optical biosensor imaging systems, including SPR imaging systems, ellipsometry imaging systems, and RWG imaging systems, offer high spatial resolution, and can be used in embodiments of the disclosure. For example, SPR imager®II (GWC Technologies Inc) uses prism-coupled SPR, and takes SPR measurements at a fixed angle of incidence, and collects the reflected light with a CCD camera. Changes on the surface are recorded as reflectivity changes. Thus, SPR imaging collects measurements for all elements of an array simultaneously.

201. A swept wavelength optical interrogation system based on RWG biosensor for imaging-based application may be employed. In this system, a fast tunable laser source is used to illuminate a sensor or an array of RWG biosensors in a microplate format. The sensor spectrum can be constructed by detecting the optical power reflected from the sensor as a function of time as the laser wavelength scans, and analysis of the measured data with computerized resonant wavelength interrogation modeling results in the construction of spatially resolved images of biosensors having immobilized receptors or a cell layer. The use of an image sensor naturally leads to an imaging based interrogation scheme. 2 dimensional label-free images can be obtained without moving parts.

202. Alternatively, angular interrogation system with transverse magnetic or $p$-polarized $TM_0$ mode can also be used. This system consists of a launch system for generating an array of light beams such that each illuminates a RWG sensor with a dimension of approximately 200 $\mu$m x 3000 $\mu$m or 200 $\mu$m x 2000 $\mu$m, and a CCD camera-based receive system for recording changes in the angles of the light beams reflected from these sensors. The arrayed light beams are obtained by means of a beam splitter in combination with diffractive optical lenses. This system allows up to 49 sensors (in a 7x7 well sensor array) to be simultaneously sampled at every 3 seconds, or up to the whole 384well microplate to be simultaneously sampled at every 10 seconds.

203. Alternatively, a scanning wavelength interrogation system can also be used. In this system, a polarized light covering a range of incident wavelengths with a constant angle is used to illuminate and scan across a waveguide grating biosensor, and the reflected light at each location can be recorded simultaneously. Through scanning, a high resolution image across a biosensor can also be achieved

### 5. Dynamic Mass Redistribution (DMR) Signals in Living Cells

204. The cellular response to stimulation through a cellular target can be encoded by the spatial and temporal dynamics of downstream signaling networks. For this reason, monitoring the integration of cell signaling in real time can provide physiologically relevant information that is useful in understanding cell biology and physiology.

205. Optical biosensors including resonant waveguide grating (RWG) biosensors, can detect an integrated cellular response related to dynamic redistribution of cellular matters, thus providing a non-invasive means for studying cell signaling. All optical biosensors are common in that they can measure changes in local refractive index at or very near the sensor surface. In principle, almost all optical biosensors are applicable for cell sensing, as they can employ an evanescent wave to characterize ligand-induced change in cells. The evanescent-wave is an electromagnetic field, created by the total internal reflection of light at a solution-surface interface, which typically extends a short distance (~hundreds of nanometers) into the solution at a characteristic depth known as the penetration depth or sensing volume.

206. Recently, theoretical and mathematical models have been developed that describe the parameters and nature of optical signals measured in living cells in response to stimulation with ligands. These models, based on a 3-layer waveguide system in combination with known cellular biophysics, link the ligand-induced optical signals to specific cellular processes mediated through a receptor.

207. Because biosensors measure the average response of the cells located at the area illuminated by the incident light, a highly confluent layer of cells can be used to achieve optimal assay results. Due to the large dimension of the cells as compared to the short penetration depth of a biosensor, the sensor configuration is considered as a non-conventional three-layer system: a substrate, a waveguide film with a grating structure, and a cell layer. Thus, a ligand-induced change in effective refractive index (i.e., the detected signal) can be, to first order, directly proportional to the change in refractive index of the bottom portion of the cell layer:

$$\Delta N = S(C)\Delta n_c$$

208. where $S(C)$ is the sensitivity to the cell layer, and $\Delta n_c$ the ligand-induced change in local refractive index of the cell layer sensed by the biosensor. Because the refractive index of a given volume within a cell is largely determined by the concentrations of bio-molecules such as proteins, $\Delta n_c$ can be assumed to be directly proportional to ligand-induced change in local concentrations of cellular targets or molecular assemblies within the sensing volume. Considering the exponentially decaying nature of the evanescent wave extending away from the sensor surface, the ligand-induced optical signal is governed by:

$$\Delta N = S(C)\alpha d \sum_i \Delta C_i \left[ e^{\frac{-z_i}{\Delta Z_c}} - e^{\frac{-z_{i+1}}{\Delta Z_c}} \right]$$

209. where $\Delta Z_c$ is the penetration depth into the cell layer, $\alpha$ the specific refraction increment (about 0.18/mL/g for proteins), $z_i$ the distance where the mass redistribution occurs, and $d$ an imaginary thickness of a slice within the cell layer. Here the cell layer is divided into an equal-spaced slice in the vertical direction. The equation above indicates that the ligand-induced optical signal is a sum of mass redistribution occurring at distinct distances away from the sensor surface, each with an unequal contribution to the overall response. Furthermore, the detected signal, in terms of wavelength or angular shifts, is primarily sensitive to mass redistribution occurring perpendicular to the sensor surface. Because of its dynamic nature, it also is referred to as dynamic mass redistribution (DMR) signal.

### 6. Cells and biosensors

210. Cells rely on multiple cellular pathways or machineries to process, encode and integrate the information they receive. Unlike the affinity analysis with optical biosensors that specifically measures the binding of analytes to a protein target, living cells are much more complex and dynamic.

211. To study cell signaling, cells can be brought into contact with the surface of a biosensor, which can be achieved through cell culture. These cultured cells can be attached onto the biosensor surface through three types of contacts: focal contacts, close contacts and extracellular matrix contacts, each with its own characteristic separation distance from the surface. As a result, the basal cell membranes are generally located away from the surface by ~10-100mn. For suspension cells, the cells can be brought to contact with the biosensor surface through either covalent coupling of cell surface receptors, or specific binding of cell surface receptors, or simply settlement by gravity force. For this reason, biosensors are able to sense the bottom portion of cells.

212. Cells, in many cases, exhibit surface-dependent adhesion and proliferation. In order to achieve robust cell assays, the biosensor surface can require a coating to enhance cell adhesion and proliferation. However, the surface properties can have a direct impact on cell biology. For example, surface-bound ligands can influence the response of cells, as can the mechanical compliance of a substrate material, which dictates how it will deform under forces applied by the cell. Due to differing culture conditions (time, serum concentration, confluency, *etc.),* the cellular status obtained can be distinct from one surface to another, and from one condition to another. Thus, special efforts to control cellular status can be necessary in order to develop biosensor-based cell assays.

213. Cells are dynamic objects with relatively large dimensions - typically in the range of tens of microns. Even without stimulation, cells constantly undergo micromotion - a dynamic movement and remodeling of cellular structure, as observed in tissue culture by time lapse microscopy at the sub-cellular resolution, as well as by bio-impedance measurements at the nanometer level.

214. Under un-stimulated conditions cells generally produce an almost net-zero DMR response as examined with a RWG biosensor. This is partly because of the low spatial resolution of optical biosensors, as determined by the large size of the laser spot and the long propagation length of the coupled light. The size of the laser spot determines the size of the area studied - and usually only one analysis point can be tracked at a time. Thus, the biosensor typically measures an averaged response of a large population of cells located at the light incident area. Although cells undergo micromotion at the single cell level, the large populations of cells give rise to an average net-zero DMR response. Furthermore, intracellular macromolecules are highly organized and spatially restricted to appropriate sites in mammalian cells. The tightly controlled localization of proteins on and within cells determines specific cell functions and responses because the localization allows cells to regulate the specificity and efficiency of proteins interacting with their proper partners and to spatially separate protein activation and deactivation mechanisms. Because of this control, under un-stimulated conditions, the local mass density of cells within the sensing volume can reach an equilibrium state, thus leading to a net-zero optical response. In order to achieve a consistent optical response, the cells examined can be cultured under conventional culture conditions for a period of time such that most of the cells have just completed a single cycle of division.

215. Living cells have exquisite abilities to sense and respond to exogenous signals. Cell signaling was previously thought to function via linear routes where an environmental cue would trigger a linear chain of reactions resulting in a single well-defined response. However, research has shown that cellular responses to external stimuli are much more complicated. It has become apparent that the information that cells receive can be processed and encoded into complex temporal and spatial patterns of phosphorylation and topological relocation of signaling proteins. The spatial and temporal targeting of proteins to appropriate sites can be crucial to regulating the specificity and efficiency of protein-protein interactions, thus dictating the timing and intensity of cell signaling and responses. Pivotal cellular decisions, such as cytoskeletal reorganization, cell cycle checkpoints and apoptosis, depend on the precise temporal control and relative spatial distribution of activated signal-transducers. Thus, cell signaling mediated through a cellular target such as G protein-coupled receptor (GPCR) typically proceeds in an orderly and regulated manner, and consists of a series of spatial and temporal events, many of which lead to changes in local mass density or redistribution in local cellular matters of cells. These changes or redistribution, when occurring within the sensing volume, can be followed directly in real time using optical biosensors

### 7. DMR Signals as a Physiological Response of Living Cells

216. Through comparison with conventional pharmacological approaches for studying receptor biology, it has been shown that when a ligand is specific to a receptor expressed in a cell system, the ligand-induced DMR signal is receptor-specific, dose-dependent and saturate-able. For a great number of G protein-coupled receptor (GPCR) ligands, the efficacies (measured by $EC_{50}$ values) are found to be almost identical to those measured using conventional methods. In addition, the DMR signals exhibit expected desensitization patterns, as desensitization and re-sensitization is common to all GPCRs. Furthermore, the DMR signal also maintains the fidelity of GPCR ligands, similar to those obtained using conventional technologies. In addition, the biosensor can distinguish full agonists, partial agonists, inverse agonists, antagonists, and allosteric modulators. Taken together, these findings indicate that the DMR is capable of monitoring physiological responses of living cells.

## 8. DMR Signals Contain Systems Cell Biology Information of Ligand-Receptor Pairs in Living Cells

217. The stimulation of cells with a ligand leads to a series of spatial and temporal events, non-limiting examples of which include ligand binding, receptor activation, protein recruitment, receptor internalization and recycling, second messenger alternation, cytoskeletal remodeling, gene expression, and cell adhesion changes. Because each cellular event has its own characteristics (e.g., kinetics, duration, amplitude, mass movement), and the biosensor is primarily sensitive to cellular events that involve mass redistribution within the sensing volume, these cellular events can contribute differently to the overall DMR signal. Chemical biology, cell biology and biophysical approaches can be used to elucidate the cellular mechanisms for a ligand-induced DMR signal. Recently, chemical biology, which directly uses chemicals for intervention in a specific cell signaling component, has been used to address biological questions. This is possible due to the identification of a great number of modulators that specifically control the activities of many different types of cellular targets. This approach has been adopted to map the signaling and its network interactions mediated through a receptor, including epidermal growth factor (EGF) receptor, and Gq- and $G_s$-coupled receptors.

218. EGFR belongs to the family of receptor tyrosine kinases. EGF binds to and stimulates the intrinsic protein-tyrosine kinase activity of EGFR, initiating a signal transduction cascade, principally involving the MAPK, Akt and JNK pathways. Upon EGF stimulation, there are many events leading to mass redistribution in A431 cells - a cell line endogenously over-expressing EGFRs. It is known that EGFR signaling depends on cellular status. As a result, the EGF-induced DMR signals are also dependent on the cellular status. In quiescent cells obtained through 20hr culturing in 0.1% fetal bovine serum, EGF stimulation leads to a DMR signal with three distinct and sequential phases: (i) a positive phase with increased signal (P-DMR), (ii) a transition phase, and (iii) a decay phase (N-DMR). Chemical biology and cell biology studies show that the EGF-induced DMR signal is primarily linked to the Ras/MAPK pathway, which proceeds through MEK and leads to cell detachment. Two lines of evidence suggest that the P-DMR is mainly due to the recruitment of intracellular targets to the activated receptors at the cell surface. First, blockage of either dynamin or clathrin activity has little effect on the amplitude of the P-DMR event. Dynamin and clathrin, two downstream components of EGFR activation, play crucial roles in executing EGFR internalization and signaling. Second, the blockage of MEK activity partially attenuates the P-DMR event. MEK is an important component in the MAPK pathway, which first translocates from the cytoplasm to the cell membrane, followed by internalization with the receptors, after EGF stimulation.

219. On the other hand, the N-DMR event is due to cell detachment and receptor internalization. Fluorescent images show that EGF stimulation leads to a significant number of receptors internalized and cell detachment. It is known that blockage of either receptor internalization or MEK activity prevents cell detachment, and receptor internalization requires both dynamin and clathrin. This suggests that blockage of either dynamin or clathrin activity should inhibit both receptor internalization and cell detachment, while blockage of MEK activity should only inhibit cell detachment, but not receptor internalization. As expected, either dynamin or clathrin inhibitors completely inhibit the EGF-induced N-DMR (~100%), while MEK inhibitors only partially attenuate the N-DMR (~80%). Fluorescent images also confirm that blocking the activity of dynamin, but not MEK, impairs the receptor internalization

## 9. DMR Signals Contain Systems Cell Pharmacology Information of a Ligand Acting on Living Cells.

220. Since the DMR signal is an integrated cellular response consisting of contributions of many cellular events involving dynamic redistribution of cellular matters within the bottom portion of cells, a ligand-induced biosensor signal, such as a DMR signal contains systems cell pharmacology information. It is known that GPCRs often display rich behaviors in cells, and that many ligands can induce operative bias to favor specific portions of the cell machinery and exhibit pathway-biased efficacies. Thus, it is highly possibly that a ligand can have multiple efficacies, depending on how cellular events downstream of the receptor are measured and used as readout(s) for the ligand pharmacology. It is difficult in practice for conventional cell assays, which are mostly pathway-biased and assay only a single signaling event, to systematically represent the signaling potentials of GPCR ligands. However, because label-free biosensors cellular assays do not require prior knowledge of cell signaling, and are pathway-unbiased and pathway-sensitive, these biosensor cellular assays are amenable to studying ligand-selective signaling as well as systems cell pharmacology of any ligands.

## 10. Biosensor parameters

221. A label-free biosensor such as RWG biosensor or bioimpedance biosensor is able to follow in real time ligand-induced cellular response. The non-invasive and manipulation-free biosensor cellular assays do not require prior knowledge of cell signaling. The resultant biosensor signal contains high information relating to receptor signaling and ligand pharmacology. Multi-parameters can be extracted from the kinetic biosensor response of cells upon stimulation. These parameters include, but not limited to, the overall dynamics, phases, signal amplitudes, as well as kinetic parameters including the transition time from one phase to another, and the kinetics of each phase (see Fang, Y., and Ferrie, A.M. (2008) "label-free optical biosensor for ligand-directed functional selectivity acting on $\beta2$ adrenoceptor in living cells". FEBS Lett. 582, 558-564; Fang, Y., et al., (2005) "Characteristics of dynamic mass redistribution of EGF receptor signaling in living cells measured with label free optical biosensors". Anal. Chem., 77, 5720-5725; Fang, Y., et al., (2006) "Resonant waveguide grating biosensor for living cell sensing". Biophys. J., 91, 1925-1940).

## 11. DMR parameters

## a) Biosensor Output Parameters

222. A number of different biosensor output parameters are discussed herein. For example, six parameters defining the kinetics of the stimulation-induced directional mass redistribution within the cells can be overall dynamics (i.e., shape), phases of the response (in the specific example of the EGF-induced DMR signal in quiescent A431 cells, there are three main phases relating to the cell response: Positive-Dynamic Mass Redistribution (P-DMR), Negative-Dynamic Mass Redistribution (N-DMR), and Recovery Positive-Dynamic Mass Redistribution (RP-DMR)), kinetics, total duration time of each phase, total amplitudes of each DMR event, and transition time from the P- to N-DMR phase, or from N-DMR to RP-DMR. Dynamic mass redistribution is often termed as dynamic cellular matter redistribution or directional mass redistribution. Other biosensor output parameters can be obtained from a resonant peak. For example, peak position, intensity, peak shape and peak width at half maximum (PWHM) can be used. Biosensor output parameters can also be obtained from the resonant band image of a biosensor. Five additional features: band shape, position, intensity, distribution and width. All of these parameters can be used independently or together for any given application of any cell assays using biosensors as disclosed herein. The use of the parameters in any subset or combination can produce a signature for a given assay or given variation on a particular assay, such as a signature for a cell receptor assay, and then a specific signature for an EGF receptor based assay.

## (1) Parameters related to the kinetics of stimulation-induced directional mass redistribution

223. There are a number of biosensor output parameters that are related to the kinetics of the stimulation-induced DMR. These parameters look at rates of change that occur to biosensor data output as a stimulatory event to the cell occurs. A stimulatory event is any event that may change the state of the cell, such as the addition of a molecule to the culture medium, the removal of a molecule from the culture medium, a change in temperature or a change in pH, or the introduction of radiation to the cell, for example. A stimulatory event can produce a stimulatory effect which is any effect, such as a directional mass redistribution, on a cell that is produced by a stimulatory event. The stimulatory event could be a molecule, a chemical, a biochemical, a biological, a polymer. The biochemical or biological could a peptide, a synthic peptide or naturally occuring peptide. For example, many different peptides act as signaling molecules, including the proinflammatory peptide bradykinin, the protease enzyme thrombin, and the blood pressure regulating peptide angiotensin. While these three proteins are distinct in their sequence and physiology, and act through different cell surface receptors, they share in a common class of cell surface receptors called G- protein coupled receptors (GPCRs). Other polypeptide ligands of GPCRs include vasopressin, oxytocin, somatostatin, neuropeptide Y, GnRH, leutinizing hormone, follicle stimulating hormone, parathyroid hormone, orexins, urotensin II, endorphins, enkephalins, and many others. GPCRs are a broad and diverse gene family that respond not only to peptide ligands but also small molecule neurotransmitters (acetylcholine, dopamine, serotonin and adrenaline), light, odorants, taste, lipids, nucleotides, and ions. The main signaling mechanism used by GPCRs is to interact with G-protein GTPase proteins coupled to downstream second messenger systems including intracellular calcium release and cAMP production. The intracellular signaling systems used by peptide GPCRs are similar to those used by all GPCRs, and are typically classified according to the G-protein they interact with and the second messenger system that is activated. For Gs-coupled GPCRs, activation of the G-protein Gs by receptor stimulates the downstream activation of adenylate cyclase and the production of cyclic AMP, while Gi-coupled receptors inhibit cAMP production. One of the key results of cAMP production is activation of protein kinase A. Gq-coupled receptors stimulate phospholipase C, releasing IP3 and diacylglycerol. IP3 binds to a receptor in the ER to cause the release of intracellular calcium, and the subsequent activation of protein kinase C, calmodulin-dependent pathways. In addition to these second messenger signaling systems for GPCRs, GPCR pathways exhibit crosstalk with other signaling pathways including tyrosine kinase growth factor receptors and map kinase pathways. Transactivation of either receptor tyrosine kinases like the EGF receptor or focal adhesion complexes can stimulate ras activation through the adaptor proteins She, Grb2 and Sos, and downstream Map kinases activating Erkl and Erk2. Src kinases may also play an essential intermediary role in the activation of ras and map kinase pathways by GPCRs."

224. It is possible that some stimulatory events can occur but there is no change in the data output. This situation is still a stimulatory event because the conditions of the cell have changed in some way that could have caused a directional mass redistribution or a change in the cell or cell culture.

225. It is understood that a particular signature can be determined for any assay or any cell condition as disclosed herein. There are numerous "signatures" disclosed herein for many different assays, but for any assay performed herein, the "signature" of that assay can be determined. It is also possible that there can be more than one "signatures" for any given assay and each can be determined as described herein. After collecting the biosensor output data and looking at one or more parameters, or the signature for the given assay can be obtained. It may be necessary to perform multiple experiments to identify the optimal signature and it may be necessary to perform the experiments under different conditions to find the optimal signature, but this can be done. It is understood that any of the method disclosed herein can have the step of "identifying" or "determining" or "providing", for example, a signature added onto them.

## (a) Overall dynamics

226. One of the parameters that can be looked at is the overall dynamics of the data output. This overall dynamic parameter observes the complete kinetic picture of the data collection. One aspect of the overall dynamics that can be observed is a change in the shape of the curve produced by the data output over time. Thus the shape of the curve produced by the data output can either be changed or stay steady upon the occurrence of the stimulatory event. The direction of the changes indicates the overall mass distribution; for example, a positive-DMR (P-DMR) phase indicates the increased mass within the evanescent tail of the sensor; a net-zero DMR suggests that there is almost no net-change of mass within the evanescent tail of the sensor, whereas a negative-DMR indicates a net-deceased mass within the evanescent tail of the sensor.

227. The overall dynamics of a stimulation- induced cell response obtained using the optical biosensors can consist of a single phase (either P- DMR or N-DMR or net- zero- DMR), or two phases (e.g., the two phases could be any combinations of these three phases), or three phases, or multiple phases (e.g., more one P- DMR can be occurred during the time course) .

## (b) Phases of the response

228. Another parameter that can observed as a function of time are the phase changes that occur in the data output. A label free biosensor produces a data output that can be graphed which will produce a curve. This curve will have transition points, for example, where the data turns from an increasing state to a decreasing state or vice versa. These changes can be called phase transitions and the time at which they occur and the shape that they take can be used, for example, as a biosensor output parameter. For example, there can be a P-DMR, a net-zero DMR, a N-DMR, or a RP-DMR. The amplitude of the P-DMR, N-DMR, and the RP-DMR can be measured as separate biosensor output parameters (See Figure 6A, B and C for examples).

## (c) Kinetics

229. Another biosensor output parameter can be the kinetics of any of the aspects of data output. For example, the rate at the completion of the phase transitions. For example, how fast the phase transition is completed or how long it does take to complete data output. Another example of the kinetics that can be measured would be the length of time for which an overall phase of the data output takes. Another example is the total duration of time of one or both of the P- and N-DMR phases. Another example is the rate or time in which it takes to acquire the total amplitudes of one or both of the P- and N-DMR phases. Another example can be the transition time $\tau$ from the P- to N-DMR phase. The kinetics of both P-DMR and N-DMR events or phases can also be measured.

## (2) Parameters related to the resonant peak

230. Resonant peaks of a given guided mode are a type of data output that occurs by looking at, for example, the intensity of the light vs the angle of coupling of the light into the biosensor or the intensity of the light versus the wavelength of coupled light into the biosensor. The optical waveguide lightmode spectrum is a type of data output that occurs by looking at the intensity of the light vs the angle of coupling of the light into the biosensor in a way that uses a broad range of angles of light to illuminate the biosensor and monitors the intensity of incoupled intensity as a function of the angle. In this spectrum, multiple resonant peaks of multiple guided modes are co-occurred. Since the principal behind the resonant peaks and OWLS spectra is the same, one can use the resonant peak of a given guided mode or OWLS spectra of multiple guided modes interchangeably, hi a biosensor, when either a particular wavelength of light occurs or when the light is produced such that it hits the biosensor at a particular angle, the light emitted from the light source becomes coupled into the biosensor and this coupling increases the signal that arises from the biosensor.

This change in intensity as a function of coupling light angle or wavelength is called the resonant peak. Distinct given modes of the sensor can give rise to similar resonant peaks with different characteristics. There are a number of different parameters defining the resonant peak or resonant spectrum of a given mode that can be used related to this peak to assess DMR or cellular effects. A subset of these are discussed below.

*(a) Peak position*

231. When the data output is graphed the peak of the resonance peak occurs, for example, at either a particular wavelength of light or at a particular angle of incidence for the light coupling into the biosensor. The angle or wavelength that this occurs at, the position, can change due to the mass redistribution or cellular event (s) in response to a stimulatory event. For example, in the presence of a potential growth factor for a particular receptor, such as the EGF receptor, the position of the resonant peak for the cultured cells can either increase or decrease the angle of coupling or the wavelength of coupling which will result in a change in the central position of the resonant peak. It is understood that the position of the peak intensity can be measured, and is a good point to measure, the position of any point along the resonant peak can also be measured, such as the position at 75% peak intensity or 50% peak intensity or 25% peak intensity, or 66% peak intensity or 45% peak intensity, for example (all levels from 1-100% of peak intensity are considered disclosed). However, when one uses a point other than the peak intensity, there will always be a position before the peak intensity and a position after the peak intensity that will be at, for example, 45% peak intensity. Thus, for any intensity, other than peak intensity, there will always be two positions within the peak where that intensity will occur. The position of these non-peak intensities can be utilized as biosensor output parameters, but one simply needs to know if the position of the intensity is a pre-peak intensity or a post-peak intensity.

*(b) Intensity*

232. Just as the position of a particular intensity of a resonant peak can used as a biosensor output parameter, so to the amount of intensity itself can also be a biosensor output parameter. One particularly relevant intensity is the maximum intensity of the resonant peak of a given mode. This magnitude of the maximum intensity, just like the position, can change based on the presence of a stimulatory event that has a particular effect on the cell or cell culture and this change can be measured and used a signature. Just as with the resonant peak position, the resonant peak intensity can also be measured at any intensity or position within the peak. For example, one could use as a biosensor output parameter, an intensity that is 50% of maximum intensity or 30% of maximum intensity or 70% of maximum intensity or any percent between 1% and 100% of maximum intensity. Likewise, as with the position of the intensity, if an intensity other than the maximum intensity will be used, such as 45% maximum intensity, there will always be two positions within the resonant peak that have this intensity. Just as with the intensity position parameter, using a non-maximum intensity can be done, one just must account for whether the intensity is a pre-maximum intensity or a post-maximum intensity.

233. For example, the presence of both inhibitors and activators results in the decrease in the peak width at half maximum (PWHM) after culture when the original cell confluency is around 50% (at ~50% confluency, the cells on the sensor surface tend to lead to a maximum PWHM value); however, another biosensor output parameter, such as the total angular shift (i.e., the central position of the resonant peak) can be used to distinguish an inhibitors from an activators from a molecule having no effect at all. The PWHM is length of a line drawn between the points on a peak that are at half of the maximum intensity (height) of the peak, as exampled in Figure 6B. The inhibitors, for example, of cell proliferation, tend to give rise to angular shift smaller than the shift for cells with no treatment at all, whereas the activators tend to give rise to a bigger angular shift, as compared to the sensors having cells without any treatment at all, when the cell densities on all sensors are essentially identical or approximately the same. The potency or ability of the molecules that either inhibit (as inhibitors) or stimulate (as activator) cell proliferation can be determined by their effect on the PWHM value, given that the concentration of all molecules are the same. A predetermined value of the PWHM changes can be used to filter out the inhibitors or activators, in combination with the changes of the central position of the resonant peak. Depending on the interrogation system used to detect the resonant peak of a given mode, the unit or value of the PWHM could be varied. For example, for an angular interrogation system, the unit can be degrees. The change in the PWHM of degrees could be 1 thousandths, 2 thousandths, 3 thousandths, 5 thousandths, 7 thousandths, or 10 thousandths, for example.

*(c) Peak shape*

234. Another biosensor output parameter that can be used is the overall peak shape, or the shape of the peak between or at certain intensities. For example, the shape of the peak at the half maximal peak intensity, or any other intensity (such as 30%, 40%, 70%, or 88%, or any percent between 20 and 100%) can be used as a biosensor output parameter. The shape can be characterized by the area of the peak either below or above a particular intensity. For example, at the half maximal peak intensity there is a point that is pre-peak intensity and a point that is post-peak intensity. A line can be drawn between these two points and the area above this line within the resonant peak or the area below the line within the resonant peak can be determined and become a biosensor output parameter. It is understood that the integrated area of a given peak can also be used to analyze the effect of molecules acting on cells.

235. Another shape related biosensor output parameter can be the width of the resonant peak for a particular peak intensity. For example, at the width of the resonant peak at the half maximal peak intensity (HMPW) can be determined by measuring the size of the line between the pre-peak intensity point on the resonant peak that is 50% of peak intensity and the point on the line that is post-peak which is at 50% peak intensity. This measurement can then be used as a biosensor output parameter. It is understood that the width of the resonant peak can be determined in this way for any intensity between 20 and 100% of peak intensity. (Examples of this can be seen through out the figures, such as figure 6B).

### (3) **Parameters related to the resonant band image of a biosensor**

236. To date, most optical biosensors monitor the binding of target molecules to the probe molecules immobilized on the sensor surface, or cell attachment or cell viability on the sensor surface one at a time. For the binding event or cell attachment or cell viability on multiple biosensors, researchers generally monitor these events in a time-sequential manner. Therefore, direct comparison among different sensors can be a challenge. Furthermore, these detection systems whether it is wavelength or angular interrogation utilize a laser light of a small spot ($\sim$100-500$\mu$m in diameter) to illuminate the sensor. The responses or resonant peaks represent an average of the cell responses from the illuminating area. For a 96 well biosensor microplate (e.g., Coming's Epic microplate), each RWG sensor is approximately 3x3 mm$^2$ and lies at the bottom of each well, whereas the sensor generally has a dimension of 1x1 mm$^2$ for a 384well microplate format. Therefore, the responses obtained using the current sensor technology only represent a small portion of the sensor surface. Ideally, a detection system should not only allow one to simultaneously monitor the responses of live cells adherent on multiple biosensors, but also allow signal interrogation from relatively large area or multiple areas of each sensor.

237. Resonant bands through an imaging optical interrogation system (e.g., a CCD camera) are a type of data output that occurs by looking at, for example, the intensity of the reflected (i.e., outcoupled) light at the defined location across a single sensor versus the physical position. Reflected light is directly related to incoupled light. Alternatively, a resonant band can be collected through a scanning interrogation system in a way that uses a small laser spot to illuminate the sensor, and scan across the whole sensor in one-dimension or two-dimension, and collect the resonant peak of a given guided mode. The resonant peaks or the light intensities as a function of position within the sensors can be finally reconsisted to form a resonant band of the sensor. In a biosensor, when either a particular wavelength of light occurs or when the light is produced such that it hits the biosensor at a particular angle, the outcoupled light varies as a function of the refractive index changes at/near the sensor surface and this changes lead to the shift of the characteristics of the resonant band of each sensor collected by the imaging system. Furthermore, the uneven attachment of the cells across the entire sensor after cultured can be directly visualized using the resonant band (See the circled resonant band in Figure 1, for example). In an ideal multi-well biosensor microplate, the location of each sensor is relative to normalize to other biosensors; i.e., the sensors are aligned through the center of each well across the row or the column in the microplate. Therefore, the resonant band images obtained can be used as an internal reference regarding to the cell attachment or cellular changes in response to the stimulation. Therefore, such resonant band of each sensor of a given mode provides additional parameters that can be used related to this band to assess DMR or cellular effects. A subset of these are discussed below.

### *(a) Band shape*

238. Another biosensor output parameter that can be used is the shape of the resonant band of each biosensor of a given mode. The shape is defined by the intensity distribution across a large area of each sensor. The shape can be used as an indicator of the homogeneity of cells attached or cell changes in response to stimulation across the large area (for example, as shown in Figure 1, each resonant band represents responses across the entire sensor with a dimension of $\sim$200 mm x 3000 mm).

### *(b) Position*

239. Similar to the position of the resonant peak of each sensor of a given mode, the position of each resonant band can be used as a biosensor output parameter. The intensity can be quantified using imaging software to generate the center position with maximum intensity of each band. Such position can be used to examine the cellular changes in response to stimulation or molecule treatment.

### *(c) Intensity*

240. Just as the position of the resonant band, the intensity of the outcoupled light collected using the imaging system can be used as a biosensor output parameter. The average intensity of the entire band or absolute intensity of each pixel in the imaging band can be used to examine the quality of the cell attachment and evaluate the cellular response.

*(d) Distribution*

241. The distribution of the outcoupled light with a defined angle or wavelength collected using the imaging system can be used as a biosensor output parameter. This parameter can be used to evaluate the surface properties of the sensor itself when no cells or probe molecules immobilized, and to examine the quality of cell attachment across the illuminated area of the sensor surface. Again, this parameter can also be used for examining the uniformity of molecule effect on the cells when the cell density across the entire area is identical; or for examining the effect of the cell density on the molecule-induced cellular responses when the cell density is distinct one region from others across the illuminated area.

*(e) Width*

242. Just like the PWHM of a resonant peak of a given mode, the width of the resonant band obtained using the imaging system can be used as a biosensor output parameter. This parameter shares almost identical features, thus the useful information content, to those of the PWHM value of a resonant peak, except that one can obtain multiple band widths at multiple regions of the illuminated area of the sensor, instead of only one PWHM that is available for a resonant peak. Similar to other parameters obtained by the resonant band images, the width can be used for the above mentioned applications.

243. All of these parameters can be used independently or together for any given application of any cell assays using biosensors as disclosed herein. The use of the parameters in any subset or combination can produce a signature for a given assay or given variation on a particular assay, such as a signature for a cell receptor assay, and then a specific signature for an EGF receptor based assay.

**12. Methods**

244. Disclosed are methods for characterizing a molecule, comprising: collecting a biosensor response of a molecule producing a primary profile; collecting a biosensor response of a marker panel in a cell panel in the presence of the molecule producing a secondary profile; extracting a specific set of biosensor parameters from each biosensor signal; normalizing each biosensor parameter against a positive control to generate a modulation comparison; plotting the modulation comparison of at least one biosensor parameter as a function of the marker panel or the cell panel to generate a molecule modulation index; optionally combining the primary profiles of the molecule and the molecule modulation index to generate a molecule biosensor index; and optionally comparing the molecule biosensor index to a library of modulator biosensor indexes.

245. Alsodiclosed are methods for screening molecules using biosensor cellular assays, comprising:

selecting a panel of cells; selecting panels of markers, each panel of maker for one cell in the cell panel; treating each cell in the panel with a molecule producing a panel of molecule-treated cells; monitoring the biosensor response to generate a panel of primary profiles of the molecule, one for each cell; adding each marker of a marker panel individually to each cell of the panel of molecule-treated cells, wherein each marker is at a specific dose; monitoring the biosensor response to generate a panel of secondary profiles of the molecule impacting the biosensor signals of markers, one for each marker; generating a molecule biosensor index across the panel of cells and against the panels of markers; optionally comparing the molecule biosensor index with a known modulator biosensor index; wherein the similarity between the molecule biosensor index and the modulator biosensor index is an indicator for the mode of action of the molecule

246. Disclosed are methods of characterizing a molecule, comprising, panning a panel of cells with a panel of known lignads to determine whether the known ligands can trigger a robust biosensor signal in a given cell type, selecting known ligands which can trigger a robust biosensor signal as markers to generate a library of markers for a given cell type, determining the potency and efficacy of each marker for its ability to trigger a biosensor signal in a specific cell type, determining the signaling pathway(s) and network interactions triggered by each marker in a specific cell type, down-selected panels of markers for each cell type, performing assays to examine the ability of a molecule to trigger biosensor signals in the panel of cells to generate a panel of primary profiles of the molecule, and to examine the ability of the molecule to modulate the markers induced biosensor signals in the panel of cells to generate a panel of secondary profiles or modulation profiles of the molecule against the marker panels, generating a molecule biosensor index, and optionally comparing the molecule biosensor index with the library of known modulators biosensor index.

247. Also disclosed are methods for characterizing a molecule, comprising: collecting a DMR response of a molecule producing a primary profile; collecting a DMR response of a marker panel in a cell panel in the presence of the molecule producing a secondary profile; extracting a specific set of DMR parameters from each biosensor signal; normalizing each DMR parameter against a positive control to generate a modulation comparison; plotting the modulation comparison of at least one biosensor parameter as a function of the marker panel or the cell panel to generate a molecule modulation index; optionally combining the primary profiles of the molecule and the molecule modulation index to generate a molecule DMR index; and optionally comparing the molecule DMR index to a library of modulator DMR indexes.

248. Also disclosed are methods, wherein the biosensor detects a DMR, further comprising identifying one or more marker modulation profiles in the modulator biosensor index similar to one or more of the molecule modulation profiles in the molecule biosensor index, wherein the molecule shares similar mode of action with the modulator when the molecule biosensor index is similar to the modulator biosensor index, wherein step a) is performed in an agonist mode, wherein the positive control in step d) comprises a primary profile of a marker produced when a sample of the cells is treated with the marker only, wherein the modulation comparison comprises a potentiation, wherein the modulation comparison comprises an inhibition, wherein the modulation comparison comprises no modulation, wherein the library of modulator biosensor indexes comprises a molecule modulation index, wherein the library of modulator biosensor indexes comprises a molecule biosensor index, wherein the library of modulator biosensor indexes comprises different molecules but against the same marker panel for the same cell panel, wherein the similarity between the molecule biosensor index and a modulator biosensor index is used as an indicator of the mode of actions, wherein the similarity is based on pattern recognition analysis between the molecule biosensor indexes, wherein the similarity is based on pattern recognition analysis between the molecule modulation indexes, wherein the similarity is based on a matrix of scores for the molecule modulation indexes, wherein the panel of cells are associated with a specific disease, a specific cellular target, a specific origin, or representative of a particular human physiology and pathophysiolgy, wherein at least one cell system is included in the panel of cells, wherein the cell system comprises two types of cells, wherein the panel of markers is selected from a library of ligands, each of which produces a biosensor signal indicative of specific cell signaling pathway(s) in a cell, wherein the specific dose for a marker in step e) comprises at least a concentration being its $EC_{50}$, $EC_{80}$, $EC_{90}$, $EC_{95}$ or $EC_{100}$ to trigger a biosensor signal in the cell, further comprising generating a long-term biosensor signal of the molecule in the absence and presence of a marker in a cell, wherein the marker is added before, at the time, or after the molecule is added to the cell, wherein the cell panel comprises a type of cell associated with a specific disease, a type of cell from a specific origin, a type of cell associated with a specific target, or a type of cell associated with a specific physiological function, wherein the cell panel comprises a native cell, an engineered cell, a transformed cell, an immortalized cell, a primary cell, an embryonic stem cell, an adult stem cell, a cancer stem cell, or a stem cell derived cell, wherein steps (a) comprise contacting the cell with a specific dose of the molecule, wherein steps (b) comprise contacting the cell with a specific dose of a marker in the marker panel, wherein steps (b) comprise contacting the cell with a specific dose of the molecule, wherein the specific dose is a certain concentration of the molecule selecting from $1\mu M$, $5\mu M$, $10\mu M$, or $50\mu M$, wherein the specific dose is a certain concentration of the molecule selecting from 1ng/ml, 10ng/ml, 100ng/ml. $1\mu g/ml$, $10\mu g/ml$, or

$100\mu g/ml$, wherein the specific dose is the $EC_{50}$, $EC_{80}$, $EC_{90}$, $EC_{95}$, or $EC_{100}$ of a marker to trigger its biosensor signal in the cell, wherein step (b) comprises contacting the cell with the ligand for a period of time and then contacting the cell with the marker, wherein step (b) comprises contacting the cell with the marker for a period of time and then contacting the cell with the molecule, wherein step (b) comprises contacting the cell with the marker and with the molecule at the same time, wherein the step (d) comprises comparing (1) the amplitude of the P-DMR of the primary profile of a marker to the amplitude of the P-DMR of the secondary profile of the molecule against the marker, (2) the amplitude of the N-DMR of the primary profile of a marker to the amplitude of the N-DMR of the secondary profile of the molecule against the marker, (3) the amplitude of the RP-DMR of the primary profile of a marker to the amplitude of the RP-DMR of the secondary profile of the molecule against the marker, or combinations thereof, wherein the modulation comparison comprises a modulation difference, a modulation percentage, or combinations thereof.

249. Disclosed are methods of producing an index comprising: collecting modulation profiles of a molecule against more than one marker; and listing the modulation comparison of the molecule in an index.

250. Also disclosed are methods, wherein modulation index were obtained in one cell type and for multiple markers, wherein modulation index were obtained in multiple cell types and for single marker, wherein the modulation index were obtained in multiple cell types and for multiple markers, wherein the modulation index were obtained for multiple markers.

251. Also disclosed are methods of drug discovery comprising: comparing an unknown molecule index with a known modulator index, determining whether the unknown molecule ligand index is similar to the known modulator index.

252. Disclosed are methods of characterizing a molecule comprising: contacting a cell with a marker; assaying the response of the cell to the marker to obtain a primary profile;contacting a cell with a marker and the molecule; and assaying the response of the cell to the marker and the molecule to obtain a modulation profile.

253. Disclosed are methods of characterizing a molecule comprising: contacting a cell with a marker and a molecule, assaying the response of the cell to the marker and the molecule.

254. Also disclosed are methods, further comprising the step of synthesizing the molecule after the preceding steps, further comprising the step of manufacturing a library of derivatives of the molecule, wherein one or more of the steps is performed on a machine, wherein the machine is a general computer, wherein the machine is a biosensor.

255. Disclosed are methods of making a composition comprising, synthesizing a molecule, wherein the molecule is characterized as a molecule, wherein the molecule index has a similarity to a modulator index, and can be identified as such using the method set forth in herein.

256. Disclosed are products produced by the process of the methods disclosed herein.

## V. Examples

### A. Experimental Procedures

#### 1. Materials

257. ADP, ATP, UTP, UDP, adenosine, spermine, histamine, epinephrine, elaidic acid, lipoxin A4, nicotinic acid, pinacidil, poly (I:C), forskolin, and epidermal growth factor (EGF) were purchased from Sigma Chemical Co. (St. Louis, MO). Phorbol 12-myristate 13-acetate (PMA), HA1077, H-8, LY294002, quercetin, U0126, AG1478, BML-265, rottlerin, and BioMol Kinase library consisting of 80 kinase inhibitors were obtained from BioMol International Inc (Plymouth Meeting, PA). Bradykinin, adrenomedulin, SFLLR-amide, angiotensin, neuropeptide Y, and SLIGKV-amide were obtained from BaChem Americas Inc. (Torrance, CA). Epic® 384 biosensor microplates cell culture compatible were obtained from Coming Inc. (Coming, NY).

#### 2. Cell Culture

258. All cell lines were obtained from American Type Cell Culture (Manassas, VA). The cell culture medium was as follows: Dulbecco's modified Eagle's medium (DMEM) supplemented with 10% fetal bovine serum (FBS), 4.5g/liter glucose, 2 mM glutamine, and antibiotics for human epidermoid carcinoma A431 and human lung carcinoma A549.

259. Cells were typically grown using ~1 to 2 x $10^4$ cells per well at passage 3 to 15 suspended in 50 μl of the corresponding culture medium in the biosensor microplate, and were cultured at 37 °C under air/5% $CO_2$ for ~1 day. Except for A431, which was subject to ~20hr starvation through continuous culture in the serum-free DMEM, the other cell types were directly assayed without starvation. The confluency for all cells at the time of assays was ~95% to 100%.

#### 3. Optical Biosensor System and Cell Assays

260. Epic® wavelength interrogation system (Coming Inc., Coming, NY) was used for whole cell sensing. This system consists of a temperature-control unit, an optical detection unit, and an on-board liquid handling unit with robotics. The detection unit is centered on integrated fiber optics, and enables kinetic measures of cellular responses with a time interval of ~ 15 sec.

261. The RWG biosensor is capable of detecting minute changes in local index of refraction near the sensor surface. Since the local index of refraction within a cell is a function of density and its distribution of biomass (e.g., proteins, molecular complexes), the biosensor exploits its evanescent wave to non-invasively detect ligand-induced dynamic mass redistribution in native cells. The evanescent wave extends into the cells and exponentially decays over distance, leading to a characteristic sensing volume of ~150 nm, implying that any optical response mediated through the receptor activation only represents an average over the portion of the cell that the evanescent wave is sampling. The aggregation of many cellular events downstream the receptor activation determines the kinetics and amplitudes of a ligand-induced DMR.

262. For biosensor cellular assays, molecule solutions were made by diluting the stored concentrated solutions with the HBSS (1x Hanks balanced salt solution, plus 20mM Hepes, pH 7.1), and transferred into a 384well polypropylene molecule storage plate to prepare a molecule source plate. Both molecule and marker source plates were made separately when a two-step assay was performed. In parallel, the cells were washed twice with the HBSS and maintained in 30μl of the HBSS to prepare a cell assay plate. Both the cell assay plate and the molecule and marker source plate(s) were then incubated in the hotel of the reader system. After ~1hr of incubation the baseline wavelengths of all biosensors in the cell assay microplate were recorded and normalized to zero. Afterwards, a 2 to 10 minute continuous recording was carried out to establish a baseline, and to ensure that the cells reached a steady state. Cellular responses were then triggered by pipetting 10μl of the marker solutions into the cell assay plate using the on-board liquid handler.

263. To study the influence of molecules on a marker-induced response, a second stimulation with the marker at a fixed dose (typically at $EC_{80}$ or $EC_{100}$) was applied. The resonant wavelengths of all biosensors in the microplate were normalized again to establish a second baseline, right before the second stimulation. The two stimulations were usually separated by ~1hr. All studies were carried out at a controlled temperature (28°C). At least two independent sets of experiments, each with at least three replicates, were performed. The assay coefficient of variation was found to be <10%.

### B. Example 1: Outline of Cell Profile Pharmacology-Based Drug Discovery

264. First, two epithelial cancerous cell lines were selected to illustrate the cell profile pharmacology approach to drug discovery. The epithelial layer is the initial barrier to infection and one of the key players in innate immunity. During infection all pathogens must find a way to pass the epithelium via airways, the gastrointestinal tract, the genitourinary tract, or cuts in the skin (or burns). The two cell lines were human airway epithelial cell A549, and human skin epithelial cell A431.

265. Second, target panning studies were conducted using known ligands (e.g., GPCR agonists, receptor tyrosine kinase agonists, Toll-like receptors (TLRs) agonists, protein kinase C (PKC) activators, adenylyl cyclase activators, phosphodiesterase inhibitors, ion channel openers or activators, actin disruption agents, or cell apoptotic agents, etc.), to determine whether the known ligands could trigger robust DMR signals in the selected cell types:

266. Third, the potency and efficacy of each ligand was determined in terms of the ligand's ability to trigger a DMR signal in each cell type.

267. Fourth, the signaling pathway(s) and network interactions triggered by each ligand in each specific cell type were determined by combining chemical biology and cell biology approaches.

268. Fifth, panels of markers were selected from the collection of these ligands for each cell type, dependent on the signaling pathway(s) that these markers trigger, as well as other factors (such as disease-specific targets or pathways). Generally, a ligand that is able to result in a robust biosensor signal, such as DMR signal, containing specific pathway information then becomes a marker. A library of markers can be identified for a given type of cells. It is worthy of noting that more than one markers can be used for a specific cellular target or receptor. For example, for beta2-adrenergic receptor in A431, epinephrine, norepinephrine, isoproterenol, salmeterol, CGP12177 and pindolol can be chosen as markers.

269. Sixth, a series of assays were carried out to examine the ability of a molecule to trigger DMR signals (i.e., primary profiles of the molecule) in the panel of cells, and the ability of the molecule to alter the marker induced DMR signals in the panel of cells (i.e., secondary profiles of the molecule).

270. Seventh, a DMR index was generated for the molecule against the panel of cells/markers.

271. Eighth, the molecule DMR index was compared with the library of known modulator DMR indexes against the same panel of cells/markers.

## C. Example 2: Target Panning Identified Markers/Targets in A549 Cells

272. Target panning studies were carried out to probe (1) whether A549 cells endogenously express receptors to selected panels of ligands; and (2) determine whether the targeting of these receptors with the panels of ligands would lead to robust DNM signals.

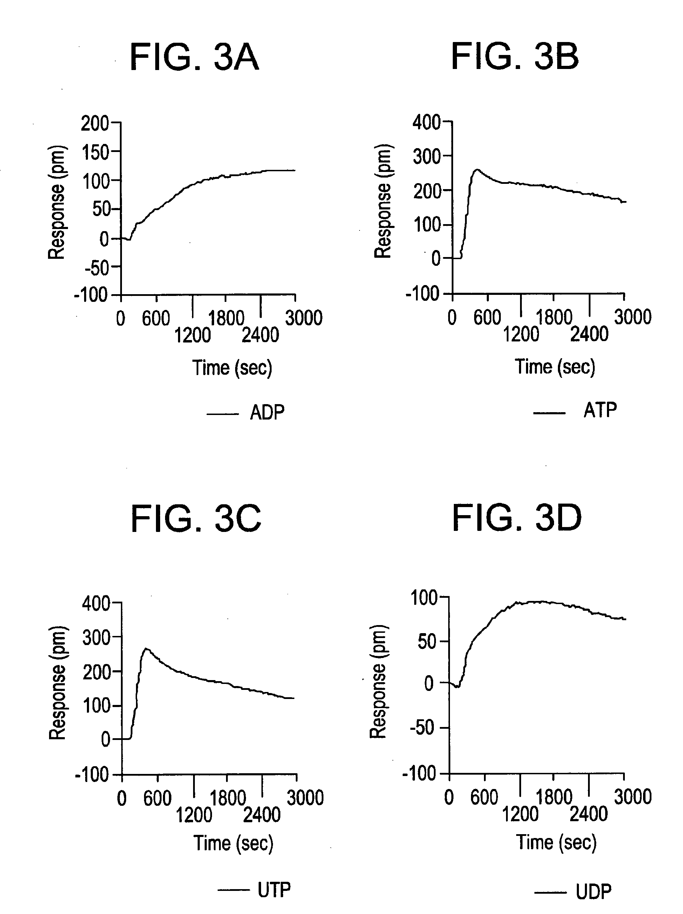

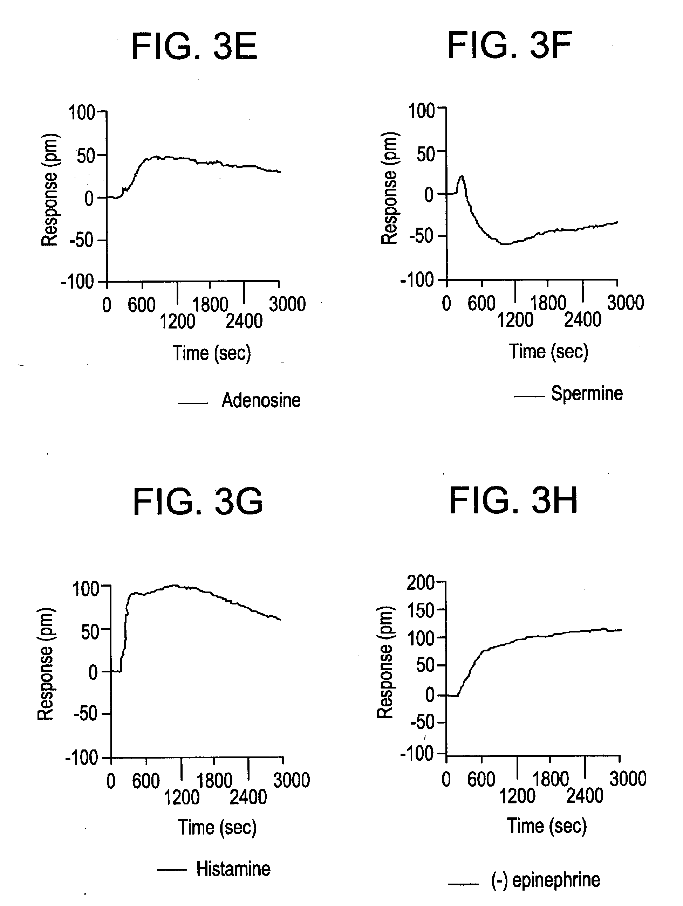

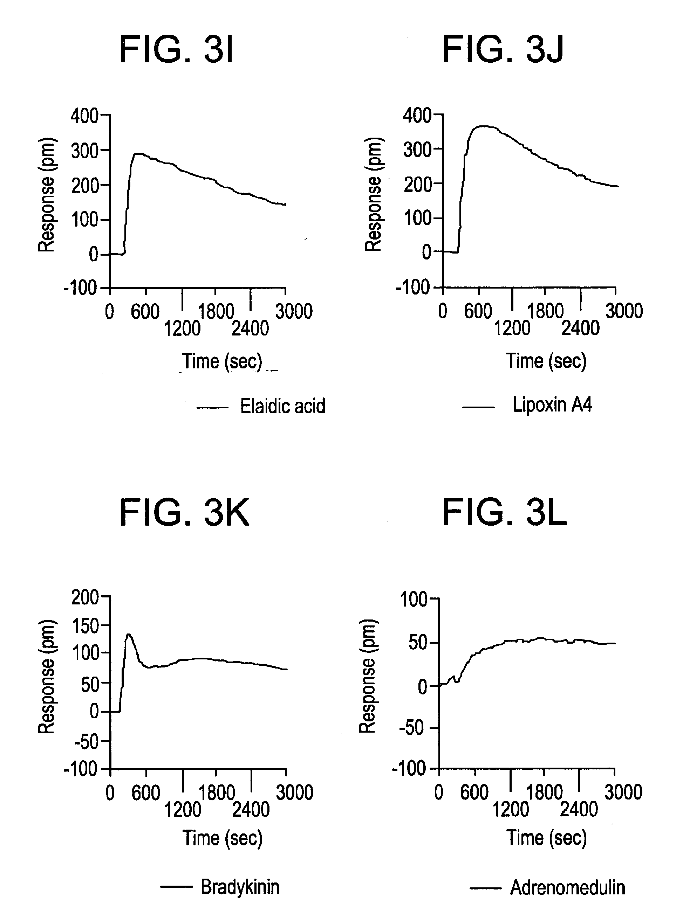

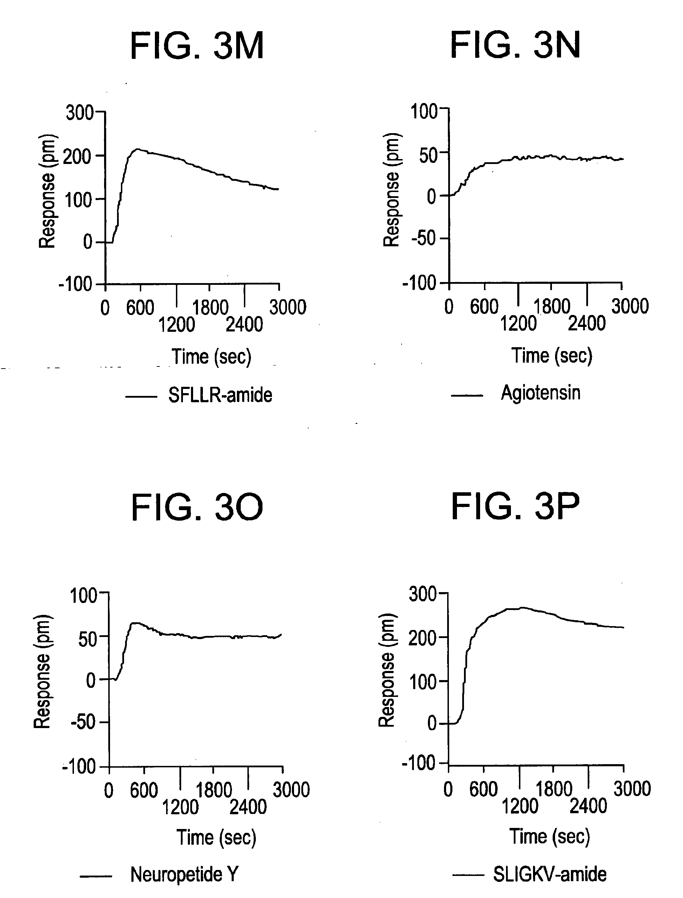

273. First, a panel of ligands targeting GPCRs, each at 10 $\mu$M concentration, was chosen, and examined their actions on the A549 cells using DMR measurements with the Epic® system. The ligand panel included the four P2Y agonists ADP, AT, UTP and UDP, the adenosine receptor agonist adenosine, the calcium sensing receptor agonist spermine, the histamine receptor agonist histamine, the adrenergic receptor agonist epinephrine, the free fatty acid receptor agonist elaidic acid, the lipoxin receptor agonist lipoxin A4, the bradykinin receptor agonist bradykinin, the adrenodullin receptor agonist adrenodullin, the PAR1 agonist SFLLR-amide, the angiotensin receptor agonist angiotensin, the neuropeptide Y receptor agonist neuropeptide Y, and the PAR2 agonist SLIGKV-amide. As shown in Figures 3A and 3B, all these ligands resulted in robust DMR signals in A549 cells

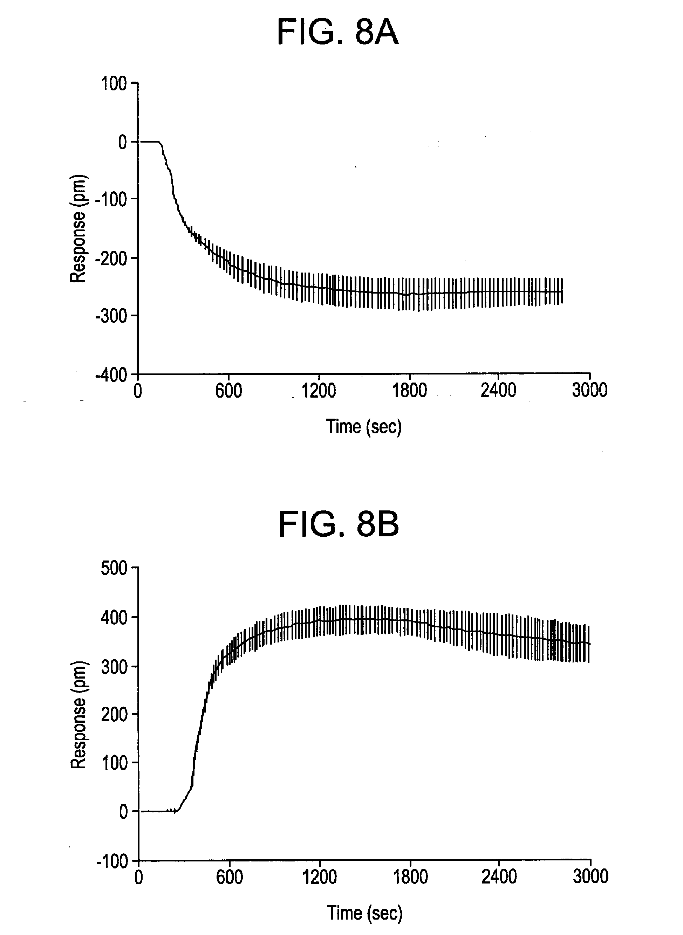

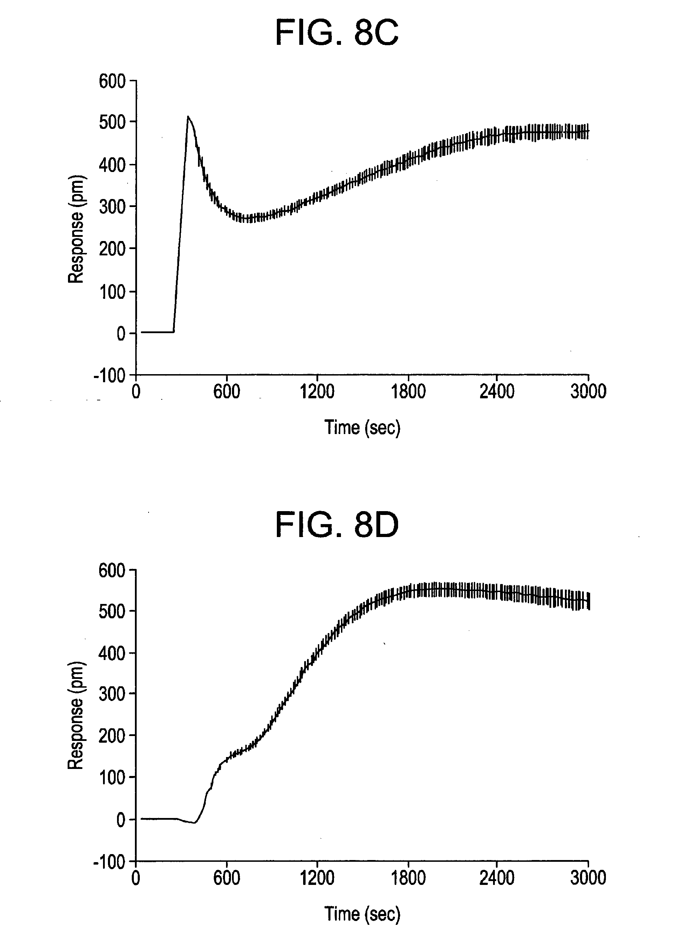

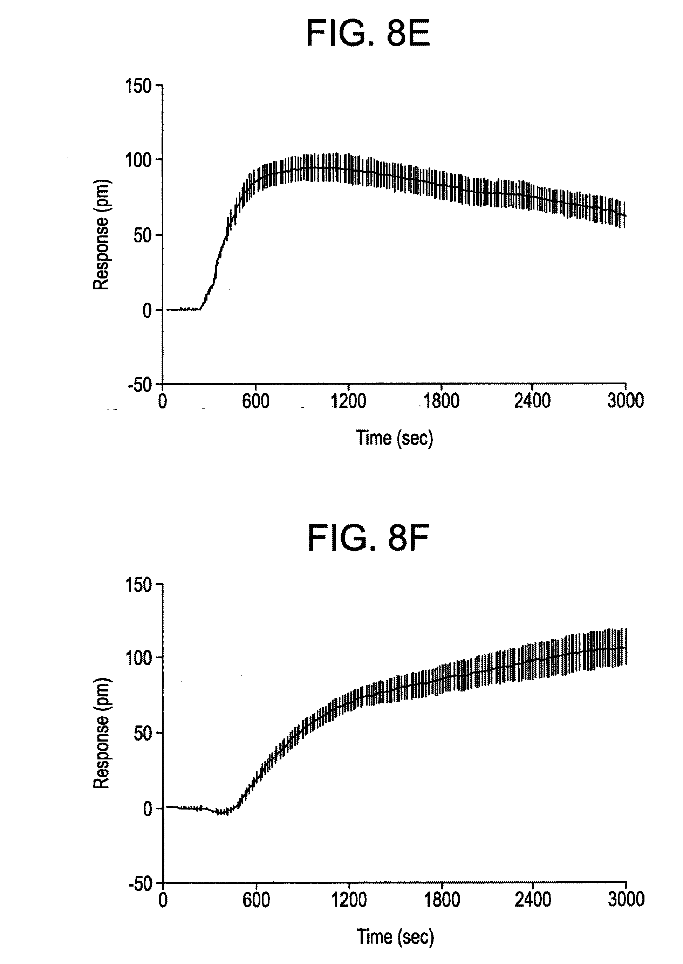

274. Next, a second ligand panel was chosen, and examined their actions on the A549 cells using DMR measures with the Epic® system. The panel included the protein kinase C activator PMA, the endogenous $K_{ATP}$ ion channel activator pinacidil, the adenylyl cyclase activator forskolin, and endogenous Toll-like receptor (TLR) activator poly(I:C). As shown in Fig. 8, this panel of ligands resulted in robust DMR signals in A549 cells.

275. After the determination that these ligands triggered robust DMR signals in A549 cells, the potency and efficacy of each marker was determined by obtaining the dose-dependent responses of A549 cells to the markers using the Epic® system.

276. Next, the cell signaling pathway(s) accounting for the DMR signal of each ligand were further examined using chemical biology, and cell biology approaches. Results showed that all these ligand-induced DMR signals contain specific, but different, signaling pathway information. Thus, all these ligands can be chosen to be makers for A549 cells. The entire or partial collections of these markers can be assembled as a panel of markers for A549 cells.

## D. Example 3: Systems Cell Biology Analysis of Epidermal Growth Factor Receptors in A431 Cells

277. An Epic® cellular assay was conducted on partially quiescent A431 cells obtained through 1 day culture with serum containing medium followed by 20 hour starvation culturing in a medium containing 0.1% fetal bovine serum. The assay demonstrated that, EGF stimulation of A431 cells leads to a DMR signal with two distinct and sequential phases: (i) a positive phase with increased signal (P-DMR), and (ii) a decay phase (N-DMR). Chemical biology and cell biology studies have shown that this EGF-induced DMR signal is primarily linked to the Ras/MAPK pathway, which proceeds through MEK and leads to cell detachment (Fang, Y., et al., (2005) "Characteristics of dynamic mass redistribution of EGF receptor signaling in living cells measured with label free optical biosensors". Anal. Chem., 77, 5720-5725).

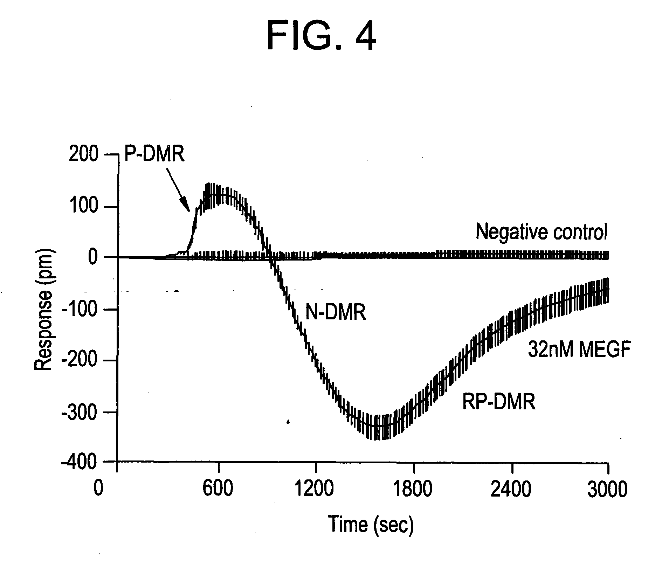

278. Here, the systems cell biology of EGFR in fully quiescent A431 cells was then reexamined using the wavelength interrogation Epic® system. The EGF was suspended in 1xHBSS containing 0.1% protease-free and fatty acid-free bovine serum albumin (BSA). Under optimal conditions, EGF triggered a dose-dependent response in quiescent A431 cells (data not shown). As shown in Figure 4, EGF at $EC_{80}$ (~32nM) led to a distinct and robust DMR signal in the fully quiescent cells. The EGF DMR signal consisted of three phases: an initial P-DMR event, followed by a N-DMR event and a recovery P-DMR (RP-DMR) event. In contrast, as shown in the negative control of Figure 4, the assay buffer alone (i.e., the vehicle) did not trigger any obvious DMR signal. In Figure 4, each DMR signal represents the averaged response of 32 replicates.

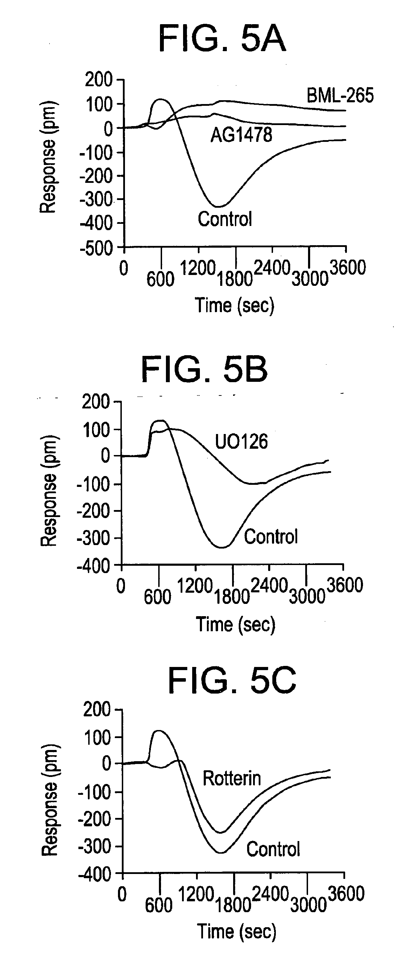

279. Next, the target specificity and signaling pathway of EGF was probed using a second set of assays. As shown in Figure 5, the pretreatment of the quiescent A431 cells with either BML-265 or AG1478 almost completely blocked the DMR signal induced by EGF of 32nM. This suggests that the EGF DMR signal is due to the activation of EGFR, as both BML-265 and AG1478 are EGFR tyrosine kinase inhibitors. As also shown in Figure 5, the pretreatment of the quiescent A431 cells with U0126 significantly and selectively attenuated the N-DMR and the RP-DMR signals. This suggests that the N-DMR event at least partly proceeds through MAPK pathway, possibly due to MEK-FAK-mediated cell detachment, as U0126 is a MEK kinase inhibitor. As also shown in Figure 5, the pretreatment of cells with rottlerin specifically caused the truncation of the early P-DMR event, as well as part of the early N-DMR event. This suggests that the early DMR response in the EGF DMR signal is due to the activation of the PKC pathway downstream of the EGFR, as rottlerin is a PKC inhibitor.

280. Finally, the EGF DMR signal was systematically probed using the BioMol Kinase inhibitor library of 80 markers. Each kinase marker at a 10 micromolar concentration was used to treat the fully quiescent A431 cells for about 1 hour. After the molecule pretreatment, the EGF induced DMR signals were measured. Three DMR parameters, the amplitudes of P-DMR, N-DMR and RP-DMR, were extracted from each response, and plotted as a function of the kinase modulators. As shown in Figures 6A, 6B, and 6C, these various kinase inhibitors modulated distinct EGF DMR parameters differently. This suggests that the EGF DMR signal indeed contains systems cell biology information for EGFR mediated signaling in the quiescent A431 cells

### E. Example 4: DMR Signals Induced by Different Panels of Markers for A431 and A549 Cells

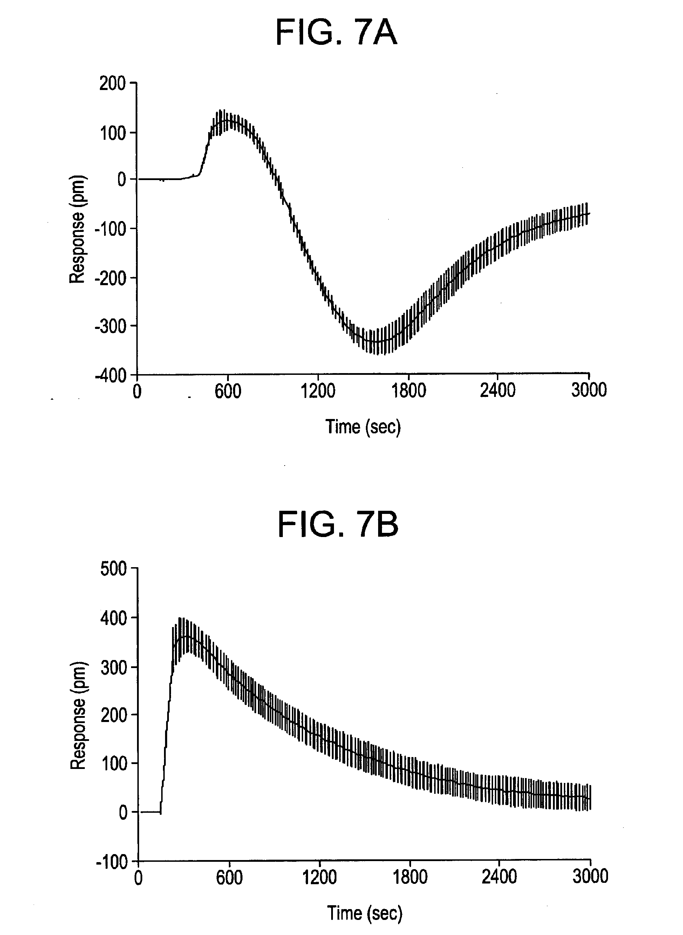

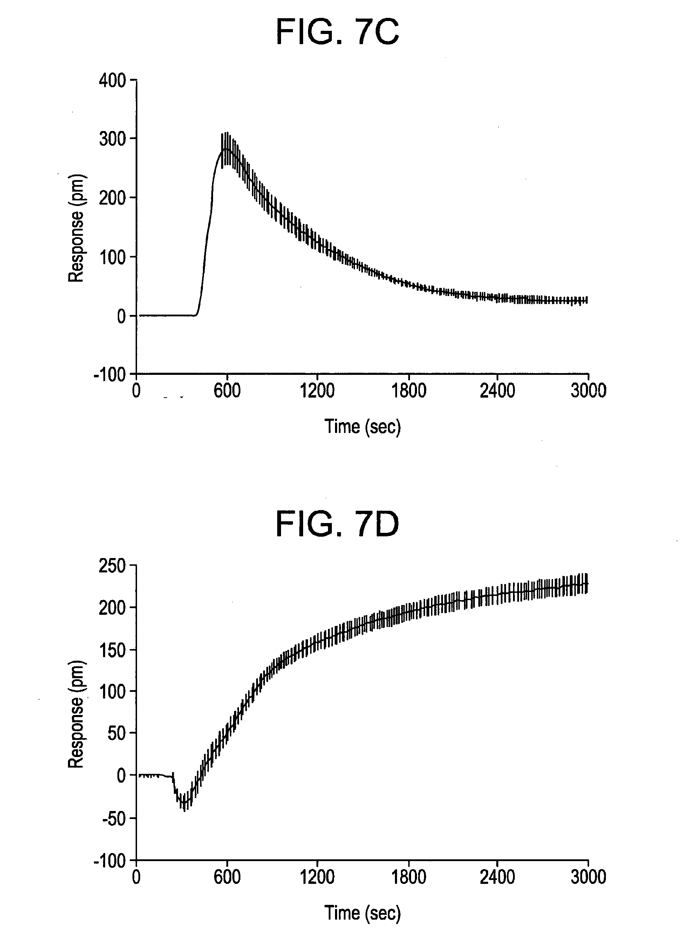

281. A first panel of markers was contacted with quiescent A431 cells at concentrations close to each of the marker's $EC_{80}$ or $EC_{100}$, and DMR measurements were obtained with and EPIC® system. This panel of markers was selected from a library of markers identified in A431 cells using Epic® cellular assays. This panel included epidermal growth factor (EGF), epinephrine, nicotinic acid, and histamine. Each of these markers leads to a wide array of cell signaling. Using Epic® cellular assays in conjunction with chemical biology and cell biology approach similar to the EGFR DMR mentioned above, we have determined the pathway(s) and network interaction(s) that is accounted for each marker-induced DMR signal in A431 cells. The main observations are summarized below. Epidermal growth factor is a natural agonist for endogenous EGF receptor in A431 whose activation leads to a divergent array of cell signaling, including PLC-PI3K pathway, MAPK pathway, STAT pathway, and PKC pathway. Epinephrine is a natural agonist for endogenous beat2-adrenergic receptor in A431 cells, whose activation leads to cAMP-PKA pathway. Nicotinic acid is a natural agonist for endogenous GPR109A receptor in A431, whose activation leads to Gi-mediated signaling and MAPK pathway. Histamine is a natural agonist for endogenous histamine type 1 receptor (H1R) in A431, whose activation leads to Gq-mediated signaling and PKC pathway. Figure 7 shows the DMR signals of fully quiescent A431 cells in response to stimulation with the first panel of markers as determined by the Epic® cellular assays. Each DMR signal represents an averaged response of 4 replicates.

282. A second panel of markers was contacted with A549 cells at concentrations close to each of the marker's $EC_{80}$ or $EC_{100}$, and DMR measurements were obtained with and EPIC® system. This panel included poly(I:C), SLIGKV-amide, pinacidil, PMA, histamine, and forskolin. Each of these markers also leads to a wide array of cell signaling. Using Epic® cellular assays in conjunction with chemical biology and cell biology approach similar to the EGFR DMR mentioned above, we have determined the pathway(s) and network interaction(s) that is accounted for each marker-induced DMR signal in A549 cells. The main observations are summarized below. Poly(I:C) is an agonist for endogenous Toll-like receptor(s) in A549, whose activation leads to IKK pathway and AKT pathway. SLIGKV-amide is an agonist for endogenous protease activated receptor subtype 2 (PAR2) in A549, whose activation leads to $G_q$-, $G_i$- and $G_{12/13}$-mediated signaling. Pinacidil is an opener for endogenous ATP-sensitive potassium ($K_{ATP}$) ion channel in A549, whose activation leads to Rho- and JAK-mediated signaling. PMA is an activator for protein kinase C (PKC), whose activation leads to PKC pathway and degranulation. Histamine is a natural agonist for endogenous histamine receptors (dominantly H1R, and possibly H3R), whose activation leads to dual signaling, possibly through Gq and $G_i$ mediated signaling in A549. Forskolin is an activator for adenylyl cyclases, whose activation leads to cAMP-PKA pathway in A549. Figure 8 shows the DMR signals of A549 cells in response to stimulation with the second panel of markers as determined by the Epic® cellular assays. Each DMR signal represents an averaged response of 4 replicates.

### F. Example 5: DMR Index of Ligands

283. A DMR index was generated for four ligand molecules. Quiescent A431 and proliferating A549 cells were separately exposed to the ligand molecules for about 1hr to generate their primary profiles, followed by stimulation with each marker at a fixed concentration (as mentioned in Figure 7 and 8) for another 1hr or so to generate secondary profiles of the molecule against the panels of markers in both types of cells. Next, one or more specific DMR parameters were chosen as a readout for plotting the DMR index of the molecule. For the pinacidil DMR response in A549, the amplitude of the N-DMR (i.e., the amplitude 30min after stimulation) was chosen. For the poly(I:C) DMR response in A549, the amplitude of the late DMR (i.e., the amplitude 50min after stimulation) was chosen. For the PMA response in A549, the late DMR amplitude (i.e., the amplitude 50min after stimulation) was chosen. For the SLIGKV-amide DMR response in A549, the P-DMR amplitude (i.e., the amplitude 20min after stimulation) was chosen. For the forskolin response in A549 cells, the P-DMR amplitude (i.e., the amplitude 50min after stimulation) was chosen. For the histamine response in A549, both the early DMR amplitude (i.e., amplitude 10min after stimulation) and the late response (i.e., the amplitude 30min after stimulation) were chosen. For the epinephrine response in A431, the P-DMR amplitude (i.e., the amplitude 50min after stimulation) was chosen. For the nicotinic acid DMR in A431, the P-DMR amplitude (i.e., the amplitude 3min after stimulation) was chosen. For the EGF DMR in A431, both the P-DMR (i.e., the amplitude 4min after stimulation) and N-DMR amplitudes (i.e., the decaying amplitude between 4min and 40min after simulation) were chosen. For the histamine response in A431, the P-DMR amplitude (i.e., the amplitude 3min after stimulation) was chosen. The percentage of modulation of the marker against each marker was calculated by normalizing the secondary profile of the molecule against the marker in the cell to the primary profile of the same marker acting on the same cell.

284. As shown in Figures 9, 10, 11, and 12, the DMR index of a ligand molecule can include two types of responses: the agonist mode response (i.e., the ligand-triggered primary profile) in both cell lines, and the antagonist mode DMR index (i.e., the secondary profiles of the ligand against the two panels of markers, each for one cell line).

285. Figure 9 shows the DMR index for a known ligand, PKA/PKG inhibitor HA1077. HA1007 triggered a similar N-DMR signal in both A549 and A431 cells. HA1077 almost completely attenuated the pinacidil response in A549, partially attenuated the epinephrine response in A431, and partially attenuated the histamine response in A431. However, HA1077 significantly potentiated the PMA response and the histamine response in A549.

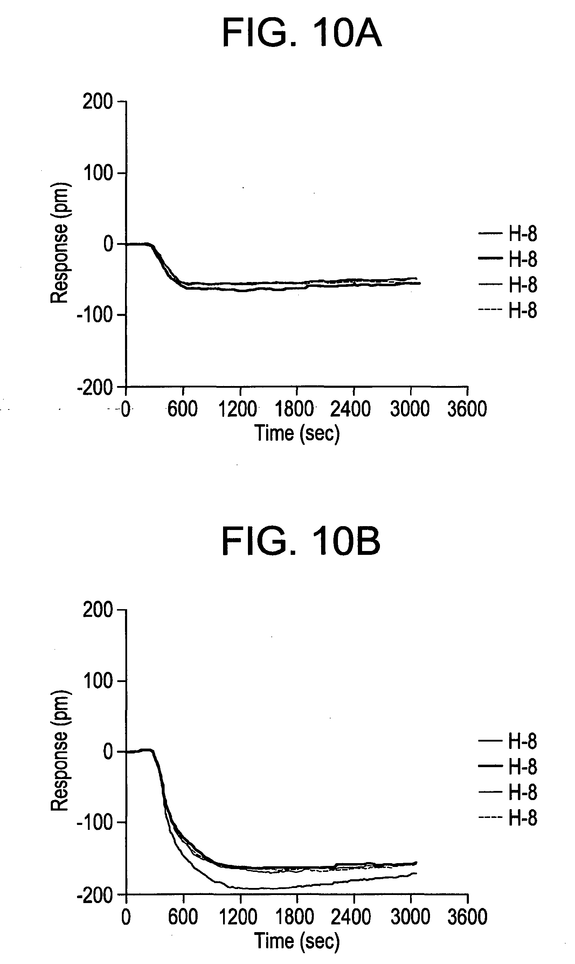

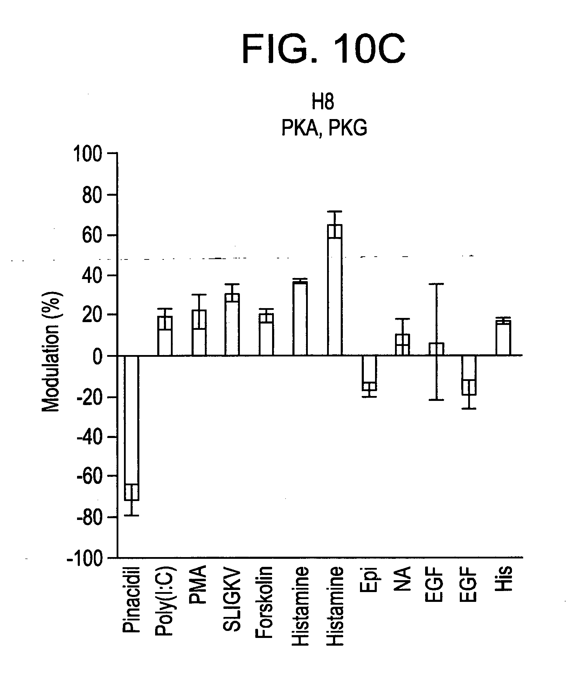

286. Figure 10 shows the DMR index of another known ligand, PKA/PKG inhibitor H-8. H-8 resulted in a similar DMR index as compared to HA-1077. However, H-8 was less effective at attenuating the histamine response and the epinephrine response in A431 cells. These results suggest that both inhibitors modulate the same target PKA.

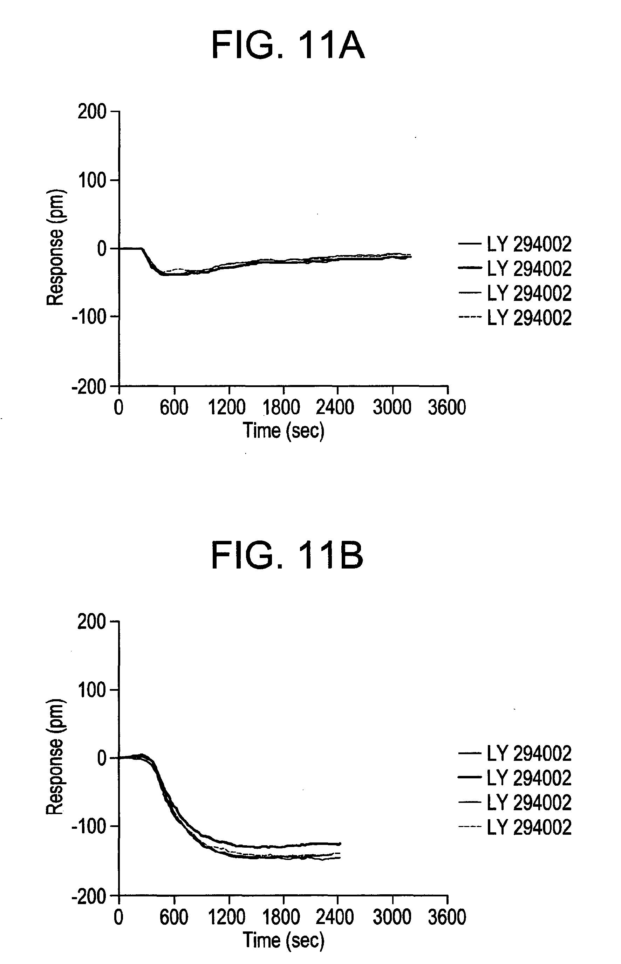

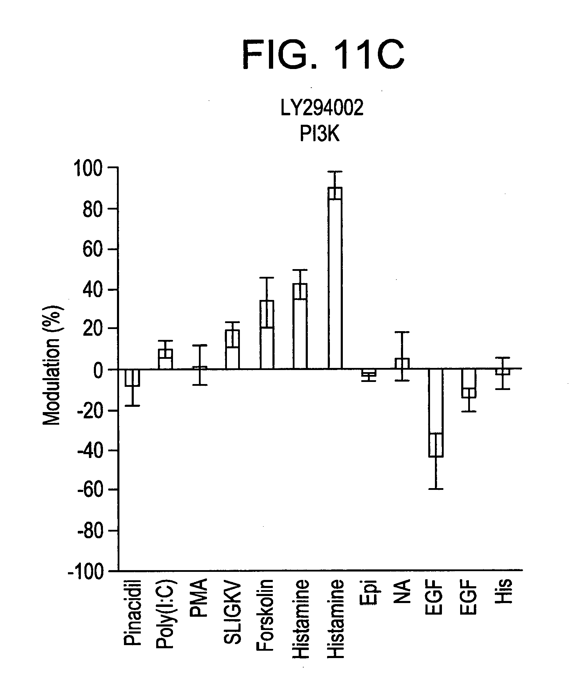

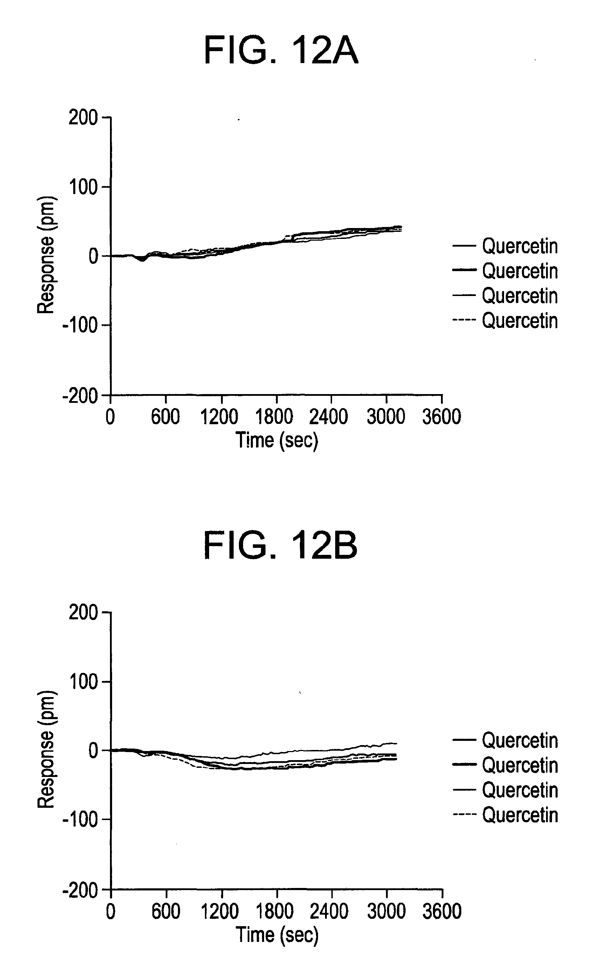

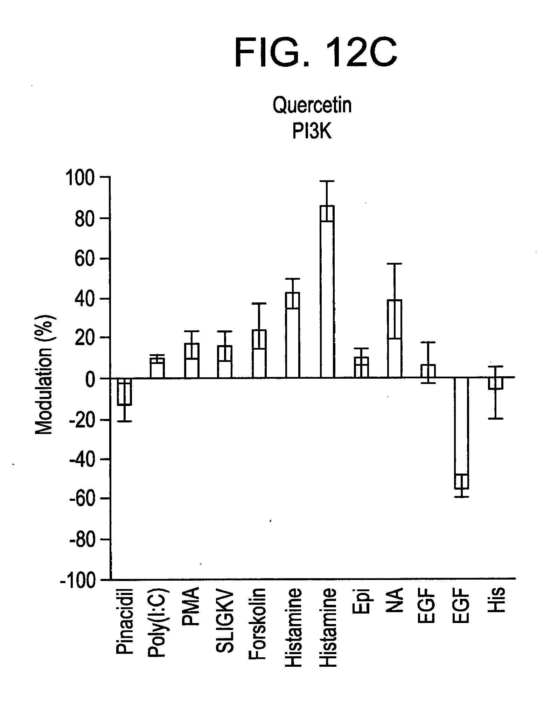

287. Figure 11 shows the DMR index of a known ligand, PI3K inhibitor LY294002. LY294002 selectively and partially attenuated the EGF response in A431, but potentiated the histamine response in A431. As shown in Figure 12, another PI3K inhibitor quercetin produced a similar DMR index. These results suggest that both inhibitors modulate the same target PI3K.

288. As shown in Figures 9, 10, 11, and 12, the DMR index of HA1077 or H-8 is dramatically different from the DMR index of LY294002 or quercetin, suggesting that HA1077 and H-8 target different cellular target (s) than LY290042 or quercein

### G. Example 6: Preparation of A431 and A549 Cells for Assay on EPIC® system

#### 1. Purpose:

a) This protocol provides a guidance of how to thaw, passage, seed and prepare two different adherent cell lines (A431 and A549) for cell profile pharmacology analysis of a molecule with the Epic® systems.

#### 2. Principles of Method And Recommendations:

a) Principles:

(1) When cells are exposed to certain molecules, they can react in a way that can be detected and monitored through the Epic® system. Whether it is due to endocytosis, apoptosis, cellular trafficking or cellular rearrangement these movements can be seen on the sensing volume of the RWG biosensor and tracked.

(2) Currently there are two cell lines being used to study these effects. A549 is an adherent cell line, and a hypotriploid human cell line with the modal chromosome number of 12, occurring in 24% of cells and is epithelial in morphology. A431 cells are adherent cells derived from a human epidermoid carcinoma and also exhibit epithelial morphology.

b) Recommendations:

(1) Both A549 and A431 cells should be used between passages 2-15. Higher passages may cause variable biosensor responses.

#### 3. Equipment And Set Up

a) A431 cell line (#CRL- 1555, ATCC, Manassas, VA)
b) A549 cell line (#CCL-185, ATCC, Manassas, VA)

c) DMEM (#10566- 016, Invitrogen, Carlsbad, CA)

d) Fetal Bovine Serum, Certified, Heat- inactivated (#10082- 139, Invitrogen, Carlsbad, CA)

e) Penicillin- Streptomycin liquid (100X) (#15140- 122, Invitrogen, Carlsbad, CA)

f) Geneticin (100x) (#10131- 027, Invitrogen, Carlsbad, CA)

g) Trypsin- EDTA (0.25% Trypsin with EDTA 4Na) 1X (#25200- 056, Invitrogen, Carlsbad, CA)

h) 1M HEPES buffer solution (#15630- 080, Invitrogen, Carlsbad, CA)

i) Hank's Balanced Salt Solution (1xHBSS), 1X with calcium and magnesium, but no phenol red (#14025- 092, Invitrogen, Carlsbad, CA)

j) Z series, Z-pak (#8320312, Beckman-Coulter, Fullerton, CA)

k) Dilu- Vial (#3- 341- 13, Fisher Scientific, Pittsburgh, PA)

l) Corning 25 cm$^2$ cell culture flask with vented cap (T-25) (#430639, Corning Inc., Coming, NY)

m) Coming 75 cm$^2$ cell culture flask with vented cap (T-75) (#430641, Coming Inc., Corning, NY)

n) Coulter Counter Z1 (Model Z1 D, Beckman-Coulter, Fullerton, CA)

o) Coming Epic® 384well biosensor microplates cell culture compatible (#5040, Coming Incorporated, Coming, NY)

p) Coming 384 well polypropylene molecule storage plate (#3656, Coming Incorporated, Coming, NY)

## 4. Solutions And Reagents

a) Complete DMEM (cDMEM)

(1) DMEM
(2) 10% Fetal Bovine Serum
(3) 100ug/ml Penicillin - Streptomycin (1X)

b) Cell assay buffer solution

(1) HBSS (1xHBSS containing 10mM Hepes, pH 7.1)

## 5. Procedure

a) First Cell Propagation (Splitting/Passaging the cells from T-25 flask)

(1) Place complete DMEM media in 37°C water bath to allow it to warm to 37°C
(2) Place Trypsin/EDTA 0.25% at room temperature
(3) Aspirate off spent media
(4) Wash cells in the flask by adding 2ml (T-25) 0.25% Trypsin/EDTA to the flask and gently rocking flask back and forth
(5) Aspirate off Trypsin/EDTA
(6) Add 2ml (T-25) of 0.25% Trypsin/EDTA to flasks
(7) Incubate in 37°C, CO$_2$ (5%) incubator for 3-5 minutes (A549) or 8 -12 minutes (A431) or until cells begin to detach from the surface, do not rock the flask back and forth, it may lead to cell clumping
(8) Add 3ml complete media to T-25 flask and disperse cells by pipetting up and down several times in the flask.
(9) Ready to split into fresh flasks.

(a) 1:10 dilution (A431)- add 1.5ml newly dispersed cells into 15ml of cDMEM in fresh T-75 flask - ~5 days between splitting cells
(b) 1:15 dilution (A549) - add 1ml newly dispersed cells into 14ml of cDMEM in fresh T-75 flask - ~4 days between splitting cells

(10) Place T-75 flask into CO$_2$ (5%) incubator set at 37°C with 95% humidity until cells reach >90% confluency by visual inspection under a microscope-ready to propagate (Split) again

b) Cell Propagation (Splitting/Passaging the cells from T-75 flask)

(1) Place complete media in 37°C water bath to allow it to warm to 37°C
(2) Place Trypsin/EDTA 0.25% at room temperature
(3) Aspirate off spent media
(4) Wash cells in the flask by adding 3ml (T-75) 0.25% Trypsin/EDTA to the flask and gently rocking flask back and forth
(5) Aspirate off Trypsin/EDTA
(6) Add 3ml (T-75) of 0.25% Trypsin/EDTA to flasks
(7) Incubate in 37°C, CO$_2$ (5%) incubator for 3-5 minutes (A549) or 8 -12 minutes (A431) or until cells begin to detach from the surface, do not rock the flask back and forth, it may lead to cell clumping
(8) After almost all the cells have detached from the flask surface, add 12ml (T-75) of complete media to cells. Disperse cells by pipetting up and down several times in the flask.
(9) Ready to split into fresh flasks.

(a) 1:10 dilution (A431)- add 1.5ml newly dispersed cells into 14ml of cDMEM in fresh T-75 flask - ~5 days between splitting cells

(b) 1:15 dilution (A549) - add 1ml newly dispersed cells into 14ml of cDMEM in fresh T-75 flask - ~4 days between splitting cells

(10) Place T-75 flask into $CO_2$ (5%) incubator set at 37°C with 95% humidity until cells reach >90% confluency by visual inspection under a microscope-ready to propagate (Split) again

c) Cell Counting

(1) Turn on the Beckman-Coulter particle counter
(2) Fill a counting vessel with 10 ml of diluent
(3) To the 10ml add 50$\mu$l of cells
(4) Push start button to begin counting
(5) Counter will display results when finished
(6) Do duplicate counts - more if the variability of counts are more than ~5%
(7) To determine the concentration of cells/ml in original stock:

[ (# of cells) (10ml) ] / [ (0.05ml in sample) (0.5ml Coulter sampling volume) ] or (# of cells) (400)

(8) Total cells = Concentration of cells /ml * total volume of cells

d) Cell Seeding

(1) Collect cells from T-75 flask after harvesting
(2) Counting the cell numbers
(3) Spin cells for 5 minutes at ~2000rpm at room temperature
(4) Aspirate off media
(5) Resuspend the cells to the final seeding concentration, 400, 000 cells/ml for A431 and 320,000 cells/ml for A549, using the cDMEM medium in a 50 ml centrifuge tube
(6) Use automatic pipettor to add 50$\mu$l of cells into each well of a 384well biosensor plate
(7) Make sure that there are no air bubbles trapped in the bottoms of the wells
(8) Each bubble needs to be removed by pipetting out the bubble and adding back into well
(9) Incubate the plate in the laminar flow hood for at least 15-30 minutes.
(10) Incubate cells at 37°C and 95% humidity in 5% $CO_2$ incubator till desired confluency is reached, usually overnight

e) Starving A431 cells

(1) The day before the assay is run, the cells need to be starved for at least 16 hours
(2) Place Serum Free Media (DMEM with 1X Pen/Strep) at 37°C
(3) Remove all of the serum media by flicking the plate into an appropriate vessel or using a plate washer
(4) Add 50$\mu$l of Serum Free Media to each well
(5) Repeat step (3) and (4) once
(6) Incubate cells at 37°C with 95% humidity in 5% $CO_2$ incubator until the next day

**6. Preparations for An Experiment On Epic® Beta System**

a) Using the Biotek ELx405UCW plate washer, wash cells

(1) Remove the cell assay plates having cells from the $CO_2$ incubator
(2) Remove lid
(3) Wash the cells with the plate washer twice using 50$\mu$l HBSS
(4) Add 50$\mu$l HBSS/well
(5) Aspirate enough buffer to leave behind 40$\mu$l

b) Replace lid
c) Put the plates with covers in the hotel of the Epic® instrument for at least 1 hour

**7. Set Up The Parameters And Perform Experiemnts On Epic® Beta System**

a) Choose appropriate assay parameters including data acquisition rate (typically 15sec/read) and time for baseline (~2min) and assays (~60 or 90 min)
b) Choose appropriate robotic parameters including the type of compound resource plates, removing the lid, compound addition and mixing, and placing the lid back
c) Change tips (CyBio Cat # OL2001- 25- 250)
d) Load Source Plate (molecule plate) and Test Plate (cell assay plate)
e) Type in "Plate ID" - the plate name
f) Run with "Assay Protocol" using Standard Protocol - each scan is the average of 2 sweeps across the bottom of the assay plate

(a) Pre-Scan for 120 seconds for baseline
(b) Add 10$\mu$l of the molecules at 5X concentration

(c) Scan for an additional 2400 seconds (~1 hour)

g) Unload Source Plate and Test plate

### VI. References

289. Fang, .Y., Ferrie, A.M., and Li, G. (2005) "Probing cytoskeleton modulation by optical biosensors". FEBS Lett., 579, 4175-4180.

290. Fang, Y., Ferrie, A.M., Fontaine, N.H., and Yuen, P.K. (2005) "Characteristics of dynamic mass redistribution of EGF receptor signaling in living cells measured with label free optical biosensors". Anal. Chem., 77, 5720-5725.

291. Fang, Y., Li, G., and Peng, J. (2005) Optical biosensor provides insights for bradykinin B2 receptor signaling in A431 cells. FEBS Lett. 579, 6365-6374.

292. Fang, Y., Ferrie, A.M., Fontaine, N.H., Mauro, J. and Balakrishnan, J. (2006) "Resonant waveguide grating biosensor for living cell sensing". Biophys. J., 91, 1925-1940.

293. Fang, Y. (2006) "Label-free cell-based assays with optical biosensors in drug discovery". Assays and Drug development Technologies. 4(5), 583-595.

294. Fang, Y., Li, G. and Ferrie, A.M. (2007) "Non-invasive optical biosensor for assaying endogenous G protein-coupled receptors in adherent cells". J. Pharmacol. Toxicol. Methods, 55, 314-322.

295. Fang, Y. and Ferrie, A.M. (2007) "Optical biosensor differentiates signaling of endogenous PAR1 and PAR2 in A431 cells" BMC Cell Biol., 8, 24(1-12).

296. Fang, Y. (2007) "Non-invasive optical biosensor for probing cell signaling" Sensors, 7, 2316-2329.

297. Fang, Y., and Ferrie, A.M. (2008) "label-free optical biosensor for ligand-directed functional selectivity acting on β2 adrenoceptor in living cells". FEBS Lett. 582, 558-564.

298. Lee, P.H., Gao, A., van Staden, C., Ly, J., Salon, J., Xu, A., Fang, Y., and Verkleeren, R. (2008) Evaluation of dynamic mass redistribution technology for pharmacological studies of recombinant and endogenously expressed G protein-coupled receptors. Assay and Drug Development Technologies, 6, 83-93.

299. Fang, Y., Frutos, A.G., Verklereen, R. (2008) Label-free cell assays for GPCR screening. Comb. Chem.& HTS 11, 357-369.

### Claims

1. A method for characterizing a molecule via a cell-based biosensor assay, comprising:

   a. generating a label-free biosensor response of a molecule in each cell in a cell panel comprising at least two types of cells, wherein the biosensor detects a dynamic mass redistribution (DMR), thereby producing a first biosensor data of the molecule in each cell;

   b. generating a label-free biosensor response of each ligand in a ligand panel in each cell in the cell panel in the presence of the molecule, thereby producing second biosensor data of the molecule for each ligand/cell pair, wherein a ligand is a molecule able to bind to and form a complex with a biomolecule to serve a biological purpose;

   c. extracting a specific set of biosensor parameters from the first and second biosensor data, wherein the biosensor parameters in the set are chosen independently;

   d. normalizing each biosensor parameter against the biosensor parameter from the first biosensor data to generate a modulation comparison;

   e. plotting the modulation comparison of at least one biosensor parameter as a function of the ligand or the cell to generate a molecule modulation index that expresses the ability of the molecule to modulate the biosensor response of the ligand panel acting on the cell panel;

   f. optionally combining the first biosensor data and the molecule modulation index to generate a molecule biosensor index; and

   g. comparing the molecule modulation index or the molecule biosensor index to a library of modulator biosensor indexes.

2. The method of claim 1, further comprising identifying one or more ligand modulation profiles in the modulator biosensor index similar to one or more of the molecule modulation profiles in the molecule biosensor index.

3. The method of claim 1, wherein the molecule shares a similar mode of action with the modulator when the molecule biosensor index is similar to the modulator biosensor index.

4. The method of claim 1, wherein the library of modulator biosensor indexes comprises a molecule modulation index.

5. The method of claim 1, wherein different biosensor parameters are chosen for different ligands or cells.

6. The method of claim 1, wherein the cells in the cell panel are primary cells.

### Patentansprüche

1. Verfahren zur Charakterisierung eines Moleküls über einen zellbasierten Biosensor-Assay, das Folgendes umfasst:

a. Erzeugen einer markierungsfreien Biosensor-Reaktion von einem Molekül in jeder Zelle in einer Zellreihe, die mindestens zwei Arten von Zellen umfasst, worin der Biosensor eine dynamische Massenumverteilung (DMR) detektiert, wodurch erste Biosensor-Daten des Moleküls in jeder Zelle erzeugt werden;

b. Erzeugen einer markierungsfreien Biosensor-Reaktion von jedem Liganden in einer Ligandenreihe in jeder Zelle in der Zellreihe in Gegenwart des Moleküls, wodurch zweite Biosensor-Daten des Moleküls für jeden Liganden/jedes Zellpaar erzeugt werden, worin ein Ligand ein Molekül ist, das zu einem Biomolekül binden und mit diesem einen Komplex bilden kann, um einen biologischen Zweck zu erfüllen;

c. Extrahieren einer spezifischen Reihe von Biosensor-Parametern aus den ersten und zweiten Biosensor-Daten, worin die Biosensor-Parameter in der Reihe unabhängig ausgewählt sind;

d. Normalisieren jedes Biosensor-Parameters gegen den Biosensor-Parameter aus den ersten Biosensor-Daten, um einen Modulationsvergleich zu erzeugen;

e. Auftragen des Modulationsvergleichs von mindestens einem Biosensor-Parameter als Funktion des Liganden oder der Zelle, um einen Molekül-Modulationsindex zu erzeugen, der die Fähigkeit des Moleküls zur Modulation der Biosensor-Reaktion der Ligandenreihe, die auf die Zellreihe wirkt, ausdrückt;

f. optionales Kombinieren der ersten Biosensor-Daten und des Molekül-Modulationsindex, um einen Molekül-Biosensor-Index zu erzeugen; und

g. Vergleichen des Molekül-Modulationsindex oder des Molekül-Biosensor-Index mit einer Bibliothek von Modulator-Biosensor-Indizes.

2. Verfahren nach Anspruch 1, das weiter das Identifizieren von einem oder mehreren Liganden-Modulations-Profilen in dem Modulator-Biosensor-Index umfasst, die einem oder mehreren der Molekül-Modulations-Profile in dem Molekül-Biosensor-Index ähneln.

3. Verfahren nach Anspruch 1, worin das Molekül eine ähnliche Wirkungsweise wie der Modulator aufweist, wenn der Molekül-Biosensor-Index dem Modulator-Biosensor-Index ähnelt.

4. Verfahren nach Anspruch 1, worin die Bibliothek von Modulator-Biosensor-Indizes einen Molekül-Modulationsindex umfasst.

5. Verfahren nach Anspruch 1, worin unterschiedliche Biosensor-Parameter für unterschiedliche Liganden oder Zellen ausgewählt werden.

6. Verfahren nach Anspruch 1, worin die Zellen in der Zellreihe Primärzellen sind.

**Revendications**

1. Procédé pour caractériser une molécule par l'intermédiaire d'un essai de biocapteur à base de cellules comprenant les étapes consistant à :

a. générer une réponse de biocapteur exempte de marqueur d'une molécule dans chaque cellule dans un panel de cellules comprenant au moins deux types de cellules, dans lequel le biocapteur détecte une redistribution de masse dynamique (RMD), produisant ainsi des premières données de biocapteur de la molécule dans chaque cellule ;

b. générer une réponse de biocapteur exempte de marqueur de chaque ligand dans un panel de ligands dans chaque cellule du panel de cellules en présence de la molécule, produisant ainsi des deuxièmes données de biocapteur de la molécule pour chaque paire ligand / cellule, dans lequel un ligand est une molécule capable de se lier à une biomolécule et de former un complexe avec elle pour servir un objectif biologique ;

c. extraire un ensemble spécifique de paramètres de biocapteur des premières et deuxièmes données de biocapteur, dans lequel les paramètres de biocapteur dans l'ensemble sont choisis de façon indépendante ;

d. normaliser chaque paramètre de biocapteur par rapport au paramètre de biocapteur des premières données de biocapteur pour générer une comparaison de la modulation ;

e. représenter graphiquement la comparaison de modulation d'au moins un paramètre de biocapteur comme fonction du ligand ou de la cellule pour générer un indice de modulation de molécule qui exprime la capacité de la molécule à moduler la réponse de biocapteur du panel de ligands agissant sur le panel de cellules ;

f. éventuellement combiner les premières données de biocapteur et l'indice de modulation de molécule pour générer un indice de biocapteur de molécule ; et

g. comparer l'indice de modulation de molécule ou l'indice de biocapteur de molécule à une banque d'indices de biocapteurs de modulateur.

2. Procédé selon la revendication 1, comprenant en outre l'étape consistant à identifier un ou plusieurs profils de modulation de ligands dans l'indice de biocapteur de modulateur similaire(s) à un ou plusieurs des profils de modulation de molécule dans l'indice de biocapteur de molécule.

3. Procédé selon la revendication 1, dans lequel la molécule partage un mode d'action similaire avec le modulateur quand l'indice de biocapteur de molécule est similaire à l'indice de biocapteur de modulateur.

4. Procédé selon la revendication 1, dans lequel la banque d'indices de biocapteurs de modulateur comprend un indice de modulation de molécule.

5. Procédé selon la revendication 1, dans lequel les différents paramètres de biocapteur sont choisis pour différents ligands ou cellules.

6. Procédé selon la revendication 1, dans lequel les cellules dans le panel de cellules sont des cellules primaires.

# FIG. 1

# FIG. 2

Cell          Pathway          Target

Multiple types of cells      Multiple pathways      Multiple Targets

Integrative pharmacology of drug compounds

EP 2 361 388 B1

# FIG. 3A

—— ADP

# FIG. 3B

—— ATP

# FIG. 3C

—— UTP

# FIG. 3D

—— UDP

## FIG. 3E

—— Adenosine

## FIG. 3F

—— Spermine

## FIG. 3G

—— Histamine

## FIG. 3H

—— (-) epinephrine

## FIG. 3I

—— Elaidic acid

## FIG. 3J

—— Lipoxin A4

## FIG. 3K

—— Bradykinin

## FIG. 3L

—— Adrenomedulin

# FIG. 3M

SFLLR-amide

# FIG. 3N

Agiotensin

# FIG. 3O

Neuropetide Y

# FIG. 3P

SLIGKV-amide

# FIG. 4

## FIG. 5A

## FIG. 5B

## FIG. 5C

# FIG. 6A

EP 2 361 388 B1

# FIG. 6B

# FIG. 6C

FIG. 6D

# FIG. 6E

# FIG. 6F

## FIG. 7A

## FIG. 7B

## FIG. 7C

## FIG. 7D

# FIG. 8A

# FIG. 8B

# FIG. 8C

# FIG. 8D

# FIG. 8E

# FIG. 8F

# FIG. 9A

# FIG. 9B

# FIG. 9C

HA1077
PKA, PKG

# FIG. 10A

# FIG. 10B

# FIG. 10C

H8
PKA, PKG

# FIG. 11A

# FIG. 11B

# FIG. 11C

LY294002
PI3K

# FIG. 12A

# FIG. 12B

# FIG. 12C

Quercetin
PI3K

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61117450 A
- US 20060160109 A
- US 20060223051 A

### Non-patent literature cited in the description

- **FANG, Y. ; FERRIE, A.M.** label-free optical biosensor for ligand-directed functional selectivity acting on β2 adrenoceptor in living cells. *FEBS Lett.,* 2008, vol. 582, 558-564
- **FANG, Y. et al.** Characteristics of dynamic mass redistribution of EGF receptor signaling in living cells measured with label free optical biosensors. *Anal. Chem.,* 2005, vol. 77, 5720-5725
- **FANG, Y. et al.** Resonant waveguide grating biosensor for living cell sensing. *Biophys. J.,* 2006, vol. 91, 1925-1940
- **FANG, .Y. ; FERRIE, A.M. ; LI, G.** Probing cytoskeleton modulation by optical biosensors. *FEBS Lett.,* 2005, vol. 579, 4175-4180
- **FANG, Y. ; FERRIE, A.M. ; FONTAINE, N.H. ; YUEN, P.K.** Characteristics of dynamic mass redistribution of EGF receptor signaling in living cells measured with label free optical biosensors. *Anal. Chem.,* 2005, vol. 77, 5720-5725
- **FANG, Y. ; LI, G. ; PENG, J.** Optical biosensor provides insights for bradykinin B2 receptor signaling in A431 cells. *FEBS Lett.,* 2005, vol. 579, 6365-6374
- **FANG, Y. ; FERRIE, A.M. ; FONTAINE, N.H. ; MAURO, J. ; BALAKRISHNAN, J.** Resonant waveguide grating biosensor for living cell sensing. *Biophys. J.,* 2006, vol. 91, 1925-1940
- **FANG, Y.** Label-free cell-based assays with optical biosensors in drug discovery. *Assays and Drug development Technologies,* 2006, vol. 4 (5), 583-595
- **FANG, Y. ; LI, G. ; FERRIE, A.M.** Non-invasive optical biosensor for assaying endogenous G protein-coupled receptors in adherent cells. *J. Pharmacol. Toxicol. Methods,* 2007, vol. 55, 314-322
- **FANG, Y. ; FERRIE, A.M.** Optical biosensor differentiates signaling of endogenous PAR1 and PAR2 in A431 cells. *BMC Cell Biol.,* 2007, vol. 8 (1-12), 24
- **FANG, Y.** Non-invasive optical biosensor for probing cell signaling. *Sensors,* 2007, vol. 7, 2316-2329
- **LEE, P.H. ; GAO, A. ; VAN STADEN, C. ; LY, J. ; SALON, J. ; XU, A. ; FANG, Y. ; VERKLEEREN, R.** Evaluation of dynamic mass redistribution technology for pharmacological studies of recombinant and endogenously expressed G protein-coupled receptors. *Assay and Drug Development Technologies,* 2008, vol. 6, 83-93
- **FANG, Y. ; FRUTOS, A.G. ; VERKLEREEN, R.** Label-free cell assays for GPCR screening. *Comb. Chem.& HTS,* 2008, vol. 11, 357-369